# EUROPEAN PATENT APPLICATION

(11) **EP 4 825 875 A1**
(43) Date of publication of application: **21.10.2026**
(21) Application number: 25171017.4
(22) Date of filing: 16.04.2025
(51) Int. Cl.: C07K 16/28, C07K 16/30

(54) **BISPECIFIC BINDING MOLECULE**

(71) Applicant: BioNTech SE, 55131 Mainz (DE)
(72) Inventor: Sahin, Ugur, 55131 Mainz (DE); Falck, Georg, 55131 Mainz (DE); Stadler, Christiane, 55131 Mainz (DE)
(74) Representative: Maiwald GmbH

(57) **Abstract**

The present invention relates to a bispecific binding molecule that binds to CD3 and PSMA. The present invention further relates to such a bispecific binding molecule for use in treating and/or preventing cancer.

## Description

### FIELD OF THE INVENTION

The present invention relates to a bispecific binding molecule that binds to CD3 and PSMA. The present invention further relates to such a bispecific binding molecule for use in treating and/or preventing cancer.

### BACKGROUND TO THE INVENTION

Prostate-specific membrane antigen (PSMA) is a membrane protein commonly expressed in prostate tissue, comprising an internal portion, a transmembrane portion, and an external portion. PSMA has increased expression in cancer tissue, including in prostate cancer but also in other types of cancer. PSMA has therefore been proposed as a diagnostic and therapeutic target for such cancers.

Antibodies, also known as immunoglobulins, are large, multi-domain, multi-subunit proteins that are used in immunotherapy to bind to (and inhibit) therapeutic targets. Variable heavy domains of camelid-derived heavy chain only immunoglobulins (comprising VHHs, also known as nanobodies or single-domain antibodies) are small antibody-based fragments similar to a single variable heavy (VH) immunoglobulin domain. A key difference between single domain antibodies and conventional antibodies is therefore the lack of constant heavy 1 (CH1), variable light (VL) and constant light (CL) domains. VHHs *per se* refer to the variable domain and typically do not include any constant domains (i.e. CH2 and CH3 domains).

CD3 (cluster of differentiation 3) is a protein complex expressed on T cells. The CD3 complex is composed of a CD3γ chain, a CD3δ chain, and two CD3ε chains. These chains associate with the T-cell receptor (TCR) α and β chains and two CD3ζ chains. Antigen binding by the TCR is translated into an intracellular activation signal by the CD3 subunits via a phosphorylation cascade. The phosphorylation causes the activation of transcription factors such as NFAT and NFkB, which leads to increased expression of cytokines and effector proteins such as granzymes and perforin and ultimately enables the T cell to perform its effector functions, i.e. killing of the target cell in case of a cytotoxic T cell.

Due to their ability to bind and activate T cells, antibodies binding to CD3 are useful in T-cell engager molecules. Bispecific T-cell engager molecules are antigen binding proteins comprising at least two antigen binding sites, wherein one binds to T cells, usually via CD3, and the other binds to a tumor cell via a tumor-specific molecule, thereby engaging the T cell and recruiting it to the tumor. Desired properties of T-cell engagers are the induction of an efficient cell killing activity, a manageable side effect profile, a long shelf life, good solubility, stability, ease of formulation and production scalability. There remains a need in the art for effective T-cell engagers for many indications.

Although various approaches have been investigated in the art, there is still a need for novel, improved immunotherapeutic candidates (such as bispecific T-cell engager molecules) targeting both PSMA and CD3.

### SUMMARY OF THE INVENTION

The present invention provides a bispecific binding molecule comprising a CD3-binding moiety and a first prostate-specific membrane antigen (PSMA)-binding moiety.

In some embodiments, the bispecific binding molecule comprises or is a bispecific antigen-binding molecule.

In some embodiments, the bispecific binding molecule comprises or is a bispecific antibody.

In some embodiments, the CD3-binding moiety comprises a CD3 heavy chain variable (CD3-VH) domain.

In some embodiments, the CD3-binding moiety comprises a CD3 light chain variable (CD3-VL) domain.

In some embodiments, the CD3-binding moiety comprises a CD3 heavy chain variable (CD3-VH) domain and a CD3 light chain variable (CD3-VL) domain.

In some embodiments, the CD3-binding moiety comprises a Fab.

In some embodiments, the Fab comprises:
(i) a heavy chain comprising a CD3 heavy chain variable (CD3-VH) domain and a CD3 heavy chain constant (CD3-CH1) domain; and
(ii) a light chain comprising a CD3 light chain variable (CD3-VL) domain and a CD3 light chain constant (CD3-CL) domain.

In some embodiments, the first PSMA-binding moiety comprises a PSMA-binding VHH domain.

In some embodiments, the bispecific binding molecule further comprises a second PSMA-binding moiety.

In some embodiments, each PSMA-binding moiety comprises a PSMA-binding VHH domain.

In some embodiments, the first PSMA-binding moiety and the second PSMA-binding moiety are the same.

In some embodiments, the first PSMA-binding moiety is linked to the CD3-binding moiety.

In some embodiments, the C-terminus of the first PSMA-binding moiety is linked to the N-terminus of the CD3-VH domain

In some embodiments, the C-terminus of the first PSMA-binding moiety is linked to the N-terminus of the CD3-VL domain.

In some embodiments, the bispecific binding molecule further comprises an Fc region comprising a first subunit and a second subunit.

In some embodiments, the CD3-binding moiety is linked to the Fc region.

In some embodiments, the second PSMA-binding moiety is linked to the Fc region.

In some embodiments, the C-terminus of the CD3-CH1 domain is linked to the first subunit of the Fc region.

In some embodiments, the C-terminus of the CD3-CL domain is linked to the first subunit of the Fc region.

In some embodiments, the C-terminus of the second PSMA-binding moiety is linked to the second subunit of the Fc region.

In some embodiments, the C-terminus of the CD3-CH1 domain is linked to a hinge region of the first subunit of the Fc region

In some embodiments, the C-terminus of the CD3-CL domain is linked to a hinge region of the first subunit of the Fc region.

In some embodiments, the C-terminus of the second PSMA-binding moiety is linked to a hinge region of the second subunit of the Fc region.

In some embodiments, the first subunit of the Fc region and the second subunit of the Fc region each comprise a CH2 domain.

In some embodiments, the first subunit of the Fc region and the second subunit of the Fc region each comprise a CH3 domain.

In some embodiments, the first subunit of the Fc region and the second subunit of the Fc region each comprise a CH2 domain and a CH3 domain.

In some embodiments, the CH3 domain of one subunit of the Fc region comprises one or more mutations selected from the list consisting of: Y349C, T366S, L368A, and Y407V.

In some embodiments, the CH3 domain of one subunit of the Fc region comprises one or more mutations selected from the list consisting of: S354C and T366W.

In some embodiments, the CH3 domain of the first subunit of the Fc region comprises one or more mutations selected from the list consisting of: Y349C, T366S, L368A, and Y407V.

In some embodiments, the CH3 domain of the second subunit of the Fc region comprises one or more mutations selected from the list consisting of: S354C and T366W.

In some embodiments, the CH3 domain of the second subunit of the Fc region comprises one or more mutations selected from the list consisting of: Y349C, T366S, L368A, and Y407V.

In some embodiments, the CH3 domain of the first subunit of the Fc region comprises one or more mutations selected from the list consisting of: S354C and T366W.

In some embodiments of the bispecific binding molecule according to the invention:
(i) the CD3-binding moiety comprises a Fab, wherein the Fab comprises:
   (a) a heavy chain comprising the CD3-VH domain and a heavy chain constant (CD3-CH1) domain; and
   (b) a light chain comprising the CD3-VL domain and a light chain constant (CD3-CL) domain;
(ii) the bispecific binding molecule further comprises a second PSMA-binding moiety, wherein the first PSMA-binding moiety and the second PSMA-binding moiety are the same, wherein the first PSMA-binding moiety and the second PSMA-binding moiety each comprise a PSMA-binding VHH domain;
(iii) the bispecific binding molecule further comprises an Fc region comprising a first subunit and a second subunit; wherein the first subunit of the Fc region and the second subunit of the Fc region each comprise a CH2 domain and a CH3 domain; optionally wherein the CH3 domain of one subunit of the Fc region comprises one or more mutations selected from the list consisting of: Y349C, T366S, L368A, and Y407V; and the CH3 domain of the other subunit of the Fc region comprises one or more mutations selected from the list consisting of: S354C and T366W;
(iv) the C-terminus of the PSMA-binding VHH domain of the first PSMA-binding moiety is linked to the N-terminus of the CD3-VH domain;
(v) the C-terminus of the CD3-CH1 domain is linked to the N-terminus of the CH2 domain of the first subunit of the Fc region, preferably to the N-terminus of a hinge region of the CH2 domain of the first subunit of the Fc region; and
(vi) the C-terminus of the PSMA-binding VHH domain of the second PSMA-binding moiety is linked to the N-terminus of the CH2 domain of the second subunit of the Fc region, preferably to the N-terminus of a hinge region of the CH2 domain of the second subunit of the Fc region.

In some embodiments, the CD3-binding moiety comprises heavy chain complementarity determining regions (HCDRs) 1-3, wherein (according to Kabat numbering):
HCDR1 comprises the amino acid sequence according to SEQ ID NO: 1,
HCDR2 comprises the amino acid sequence according to SEQ ID NO: 2, and
HCDR3 comprises the amino acid sequence according to SEQ ID NO: 3,

In some embodiments, the CD3-binding moiety comprises light chain complementarity determining regions (LCDRs) 1-3, wherein (according to Kabat numbering):
LCDR1 comprises the amino acid sequence according to SEQ ID NO: 4,
LCDR2 comprises the amino acid sequence according to SEQ ID NO: 5, and
LCDR3 comprises the amino acid sequence according to SEQ ID NO: 6.

In some embodiments, the CD3-binding moiety comprises heavy chain complementarity determining regions (HCDRs) 1-3, and light chain complementarity determining regions (LCDRs) 1-3, wherein (according to Kabat numbering):
HCDR1 comprises the amino acid sequence according to SEQ ID NO: 1,
HCDR2 comprises the amino acid sequence according to SEQ ID NO: 2,
HCDR3 comprises the amino acid sequence according to SEQ ID NO: 3,
LCDR1 comprises the amino acid sequence according to SEQ ID NO: 4,
LCDR2 comprises the amino acid sequence according to SEQ ID NO: 5, and
LCDR3 comprises the amino acid sequence according to SEQ ID NO: 6.

In some embodiments, the CD3-binding moiety comprises heavy chain complementarity determining regions (HCDRs) 1-3, wherein (according to IMGT numbering):
HCDR1 comprises the amino acid sequence according to SEQ ID NO: 7,
HCDR2 comprises the amino acid sequence according to SEQ ID NO: 8,
HCDR3 comprises the amino acid sequence according to SEQ ID NO: 9,

In some embodiments, the CD3-binding moiety comprises light chain complementarity determining regions (LCDRs) 1-3, wherein (according to IMGT numbering):
LCDR1 comprises the amino acid sequence according to SEQ ID NO: 10,
LCDR2 comprises the amino acid sequence according to SEQ ID NO: 11, and
LCDR3 comprises the amino acid sequence according to SEQ ID NO: 12.

In some embodiments, the CD3-binding moiety comprises heavy chain complementarity determining regions (HCDRs) 1-3, and light chain complementarity determining regions (LCDRs) 1-3, wherein (according to IMGT numbering):
HCDR1 comprises the amino acid sequence according to SEQ ID NO: 7,
HCDR2 comprises the amino acid sequence according to SEQ ID NO: 8,
HCDR3 comprises the amino acid sequence according to SEQ ID NO: 9,
LCDR1 comprises the amino acid sequence according to SEQ ID NO: 10,
LCDR2 comprises the amino acid sequence according to SEQ ID NO: 11, and
LCDR3 comprises the amino acid sequence according to SEQ ID NO: 12;
optionally wherein one or more of the CDRs comprises one, two or three amino acid mutations relative to the recited sequences.

In some embodiments, the HCDR1, HCDR2 and HCDR3 sequences consist of:
(i) SEQ ID NO: 1, SEQ ID NO: 2, and SEQ ID NO: 3, respectively; or
(ii) SEQ ID NO: 7, SEQ ID NO: 8, and SEQ ID NO: 9, respectively.

In some embodiments, the LCDR1, LCDR2 and LCDR3 sequences consist of:
(i) SEQ ID NO: 4, SEQ ID NO: 5, and SEQ ID NO: 6, respectively; or
(ii) SEQ ID NO: 10, SEQ ID NO: 11, and SEQ ID NO: 12, respectively.

In some embodiments, the CD3-binding moiety comprises a CD3-VH domain comprising a sequence having at least 70%, at least 75%, at least 80%, at least 85%, at least 90%, at least 95%, at least 97%, at least 99%, or 100% identity to any one of SEQ ID NO: 13, SEQ ID NO: 14, or SEQ ID NO: 15.

In some embodiments, the CD3-VH domain comprises or consists of a sequence as set forth in any one of SEQ ID NO: 13, SEQ ID NO: 14, or SEQ ID NO: 15.

In some embodiments, the CD3-binding moiety comprises a CD3-VL domain comprising a sequence having at least 70%, at least 75%, at least 80%, at least 85%, at least 90%, at least 95%, at least 97%, at least 99%, or 100% identity to any one of SEQ ID NO: 17, SEQ ID NO: 18, or SEQ ID NO: 19.

In some embodiments, the CD3-VL domain comprises a sequence as set forth in any one of SEQ ID NO: 17, SEQ ID NO: 18, or SEQ ID NO: 19.

In some embodiments, the CD3-binding moiety comprises a heavy chain comprising a sequence having at least 70%, at least 75%, at least 80%, at least 85%, at least 90%, at least 95%, at least 97%, at least 99%, or 100% identity to any one of SEQ ID NO: 21, SEQ ID NO: 22, or SEQ ID NO: 23.

In some embodiments, the CD3-binding moiety comprises a heavy chain comprising a sequence as set forth in any one of SEQ ID NO: 21, SEQ ID NO: 22, or SEQ ID NO: 23.

In some embodiments, the CD3-binding moiety comprises a light chain comprising a sequence having at least 70%, at least 75%, at least 80%, at least 85%, at least 90%, at least 95%, at least 97%, at least 99%, or 100% identity to any one of SEQ ID NO: 25, SEQ ID NO: 26, or SEQ ID NO: 27.

In some embodiments, the CD3-binding moiety comprises a light chain comprising a sequence as set forth in any one of SEQ ID NO: 25, SEQ ID NO: 26, or SEQ ID NO: 27.

In some embodiments, the CD3 is human CD3. In some embodiments, the CD3 is CD3ε. In some embodiments, the CD3 is human CD3ε.

In some embodiments, the, or each, PSMA-binding moiety independently comprises heavy chain complementarity determining regions (HCDRs) 1-3, wherein (according to Kabat numbering):
HCDR1 comprises the amino acid sequence according to SEQ ID NO: 29,
HCDR2 comprises the amino acid sequence according to SEQ ID NO: 30, and
HCDR3 comprises the amino acid sequence according to SEQ ID NO: 31;
optionally wherein one or more of the CDRs comprises one, two or three amino acid mutations relative to the recited sequences.

In some embodiments, the, or each, PSMA-binding moiety independently comprises heavy chain complementarity determining regions (HCDRs) 1-3, wherein (according to Kabat numbering):
HCDR1 comprises the amino acid sequence according to SEQ ID NO: 36,
HCDR2 comprises the amino acid sequence according to SEQ ID NO: 37, and
HCDR3 comprises the amino acid sequence according to SEQ ID NO: 38;
optionally wherein one or more of the CDRs comprises one, two or three amino acid mutations relative to the recited sequences.

In some embodiments, the, or each, PSMA-binding moiety independently comprises a heavy chain complementarity determining regions (HCDRs) 1-3, wherein (according to IMGT numbering):
HCDR1 comprises the amino acid sequence according to SEQ ID NO: 32,
HCDR2 comprises the amino acid sequence according to SEQ ID NO: 33, and
HCDR3 comprises the amino acid sequence according to SEQ ID NO: 34;
optionally wherein one or more of the CDRs comprises one, two or three amino acid mutations relative to the recited sequences.

In some embodiments, the, or each, PSMA-binding moiety independently comprises a heavy chain complementarity determining regions (HCDRs) 1-3, wherein (according to IMGT numbering):
HCDR1 comprises the amino acid sequence according to SEQ ID NO: 39,
HCDR2 comprises the amino acid sequence according to SEQ ID NO: 40, and
HCDR3 comprises the amino acid sequence according to SEQ ID NO: 41;
optionally wherein one or more of the CDRs comprises one, two or three amino acid mutations relative to the recited sequences.

In some embodiments, the, or each, PSMA-binding moiety independently comprises a sequence having at least 70%, at least 75%, at least 80%, at least 85%, at least 90%, at least 95%, at least 97%, at least 99%, or 100% identity to SEQ ID NO: 35.

In some embodiments, the, or each, PSMA-binding moiety independently comprises or consists of a sequence as set forth in SEQ ID NO: 35.

In some embodiments, the, or each, PSMA-binding moiety independently comprises a sequence having at least 70%, at least 75%, at least 80%, at least 85%, at least 90%, at least 95%, at least 97%, at least 99%, or 100% identity to SEQ ID NO: 42.

In some embodiments, the, or each, PSMA-binding moiety independently comprises or consists of a sequence as set forth in SEQ ID NO: 42.

In some embodiments, the bispecific binding molecule comprises:
(i) a first polypeptide comprising the amino acid sequence according to SEQ ID NO: 65 or a variant having at least 80% sequence identity thereto;
(ii) a second polypeptide comprising the amino acid sequence according to SEQ ID NO: 71 or a variant having at least 80% sequence identity thereto; and/or
(iii) a third polypeptide comprising the amino acid sequence according to SEQ ID NO: 73 or a variant having at least 80% sequence identity thereto.

In some embodiments, the bispecific binding molecule comprises:
(i) a first polypeptide comprising the amino acid sequence according to SEQ ID NO: 66 or a variant having at least 80% sequence identity thereto;
(ii) a second polypeptide comprising the amino acid sequence according to SEQ ID NO: 71 or a variant having at least 80% sequence identity thereto; and/or
(iii) a third polypeptide comprising the amino acid sequence according to SEQ ID NO: 74 or a variant having at least 80% sequence identity thereto.

In some embodiments, the bispecific binding molecule comprises:
(i) a first polypeptide comprising the amino acid sequence according to SEQ ID NO: 67 or a variant having at least 80% sequence identity thereto;
(ii) a second polypeptide comprising the amino acid sequence according to SEQ ID NO: 71 or a variant having at least 80% sequence identity thereto; and/or
(iii) a third polypeptide comprising the amino acid sequence according to SEQ ID NO: 75 or a variant having at least 80% sequence identity thereto.

In some embodiments, the bispecific binding molecule comprises:
(i) a first polypeptide comprising the amino acid sequence according to SEQ ID NO: 68 or a variant having at least 80% sequence identity thereto;
(ii) a second polypeptide comprising the amino acid sequence according to SEQ ID NO: 72 or a variant having at least 80% sequence identity thereto; and/or
(iii) a third polypeptide comprising the amino acid sequence according to SEQ ID NO: 73 or a variant having at least 80% sequence identity thereto.

In some embodiments, the bispecific binding molecule comprises:
(i) a first polypeptide comprising the amino acid sequence according to SEQ ID NO: 69 or a variant having at least 80% sequence identity thereto;
(ii) a second polypeptide comprising the amino acid sequence according to SEQ ID NO: 72 or a variant having at least 80% sequence identity thereto; and/or
(iii) a third polypeptide comprising the amino acid sequence according to SEQ ID NO: 74 or a variant having at least 80% sequence identity thereto.

In some embodiments, the bispecific binding molecule comprises:
(i) a first polypeptide comprising the amino acid sequence according to SEQ ID NO: 70 or a variant having at least 80% sequence identity thereto;
(ii) a second polypeptide comprising the amino acid sequence according to SEQ ID NO: 72 or a variant having at least 80% sequence identity thereto; and/or
(iii) a third polypeptide comprising the amino acid sequence according to SEQ ID NO: 75 or a variant having at least 80% sequence identity thereto.

The present invention also provides one or more polynucleotide(s) comprising a nucleic acid sequence encoding the bispecific binding molecule according to the invention.

In some embodiments, the nucleic acid sequence is selected from the list consisting of: a DNA sequence, a cDNA sequence, an RNA sequence, and an mRNA sequence.

In some embodiments, the nucleic acid sequence is an RNA sequence.

In some embodiments, the RNA sequence is an mRNA sequence.

In some embodiments, the RNA is modRNA (N1-methyl-pseudouridine).

In some embodiments, the RNA sequence comprises a 5'-cap.

In some embodiments, the 5'-cap is CC413.

In some embodiments, the 5'-cap is m₂^{7,3'-O}Gppp(ₘ₁^{2'-O})ApG, which comprises the following structure:

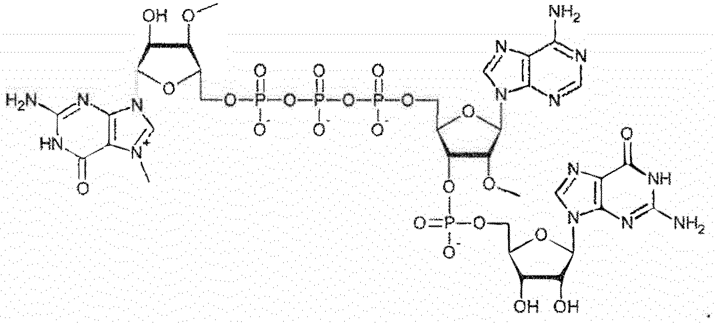

In some embodiments, the RNA sequence comprises a poly adenine sequence (poly(A) tail).

In some embodiments, the poly adenine sequence comprises the nucleic acid sequence according to SEQ ID NO: 43 or a variant having at least 80% sequence identity thereto.

In some embodiments, the RNA sequence comprises a 5'UTR.

In some embodiments, the 5'UTR comprises the nucleic acid sequence according to SEQ ID NO: 44 or a variant having at least 80% sequence identity thereto.

In some embodiments, the RNA sequence comprises a 3'UTR.

In some embodiments, the 3'UTR comprises the nucleic acid sequence according to SEQ ID NO: 45 or a variant having at least 80% sequence identity thereto.

In some embodiments, the first polynucleotide comprising a nucleic acid sequence encoding the following polypeptide chain from N to C terminus:
PSMA-binding moiety, preferably the PSMA-binding VHH domain, - (optional linker) - CD3-VH domain - CD3-CH1 domain - CH2 domain - CH3 domain

In some embodiments, the second polynucleotide comprising a nucleic acid sequence encoding the following polypeptide chain from N to C terminus:
PSMA-binding moiety, preferably the PSMA-binding VHH domain, - (optional linker) - CH2 domain - CH3 domain

In some embodiments, the third polynucleotide comprising a nucleic acid sequence encoding the following polypeptide chain from N to C terminus:
CD3-VL domain - CD3-CL domain

In some embodiments, a plurality of polynucleotides comprises:
(i) a first polynucleotide comprising a nucleic acid sequence encoding the following polypeptide chain from N to C terminus:
   PSMA-binding moiety, preferably the PSMA-binding VHH domain, - (optional linker) - CD3-VH domain - CD3-CH1 domain - CH2 domain - CH3 domain;
(ii) a second polynucleotide comprising a nucleic acid sequence encoding the following polypeptide chain from N to C terminus:
   PSMA-binding moiety, preferably the PSMA-binding VHH domain, - (optional linker) - CH2 domain - CH3 domain; and
(iii) a third polynucleotide comprising a nucleic acid sequence encoding the following polypeptide chain from N to C terminus:
   CD3-VL domain - CD3-CL domain.

In some embodiments, the first polynucleotide comprises a nucleic acid sequence comprising in 5'-3' orientation:
5'-cap - 5'UTR - PSMA-binding moiety, preferably the PSMA-binding VHH domain, - (optional linker) - CD3-VH domain -CD3-CH1 domain - CH2 domain - CH3 domain - 3'UTR - Poly(A).

In some embodiments, the second polynucleotide comprises a nucleic acid sequence comprising in 5'-3' orientation:
5'-cap - 5'UTR - PSMA-binding moiety, preferably the PSMA-binding VHH domain, - (optional linker) - CH2 domain - CH3 domain - 3'UTR - Poly(A).

In some embodiments, the third polynucleotide comprises a nucleic acid sequence comprising in 5'-3' orientation:
5'-cap - 5'UTR - CD3-VL domain - CD3-CL domain - 3'UTR - Poly(A).

In some embodiments:
(i) the first polynucleotide comprises a nucleic acid sequence comprising in 5'-3' orientation:
   5'-cap - 5'UTR - PSMA-binding moiety, preferably the PSMA-binding VHH domain, - (optional linker) - CD3-VH domain -CD3-CH1 domain - CH2 domain - CH3 domain - 3'UTR - Poly(A);
(ii) the second polynucleotide comprises a nucleic acid sequence comprising in 5'-3' orientation:
   5'-cap - 5'UTR - PSMA-binding moiety, preferably the PSMA-binding VHH domain, - (optional linker) - CH2 domain - CH3 domain - 3'UTR - Poly(A); and
(ii) the third polynucleotide comprises a nucleic acid sequence comprising in 5'-3' orientation:
   5'cap - 5'UTR - CD3-VL domain - CD3-CL domain - 3'UTR - Poly(A).

In some embodiments, the first polynucleotide comprises a nucleic acid sequence according to SEQ ID NO: 46 or a variant having at least 80% sequence identity to SEQ ID NO: 46.

In some embodiments, the first polynucleotide comprises a nucleic acid sequence according to SEQ ID NO: 47 or a variant having at least 80% sequence identity to SEQ ID NO: 47.

In some embodiments, the first polynucleotide comprises a nucleic acid sequence according to SEQ ID NO: 48 or a variant having at least 80% sequence identity to SEQ ID NO: 48.

In some embodiments, the first polynucleotide comprises a nucleic acid sequence according to SEQ ID NO: 49 or a variant having at least 80% sequence identity to SEQ ID NO: 49.

In some embodiments, the first polynucleotide comprises a nucleic acid sequence according to SEQ ID NO: 50 or a variant having at least 80% sequence identity to SEQ ID NO: 50.

In some embodiments, the first polynucleotide comprises a nucleic acid sequence according to SEQ ID NO: 51 or a variant having at least 80% sequence identity to SEQ ID NO: 51.

In some embodiments, the second polynucleotide comprises a nucleic acid sequence according to SEQ ID NO: 52 or a variant having at least 80% sequence identity to SEQ ID NO: 52.

In some embodiments, the second polynucleotide comprises a nucleic acid sequence according to SEQ ID NO: 53 or a variant having at least 80% sequence identity to SEQ ID NO: 53

In some embodiments, the third polynucleotide comprises a nucleic acid sequence according to SEQ ID NO: 54 or a variant having at least 80% sequence identity to SEQ ID NO: 54.

In some embodiments, the third polynucleotide comprises a nucleic acid sequence according to SEQ ID NO: 55 or a variant having at least 80% sequence identity to SEQ ID NO: 55.

In some embodiments, the third polynucleotide comprises a nucleic acid sequence according to SEQ ID NO: 56 or a variant having at least 80% sequence identity to SEQ ID NO: 56.

In some embodiments:
(i) the first polynucleotide comprises a nucleic acid sequence according to SEQ ID NO: 46 or a variant having at least 80% sequence identity to SEQ ID NO: 46;
(ii) the second polynucleotide comprises a nucleic acid sequence according to SEQ ID NO: 52 or a variant having at least 80% sequence identity to SEQ ID NO: 52; and
(iii) the third polynucleotide comprises a nucleic acid sequence according to SEQ ID NO: 54 or a variant having at least 80% sequence identity to SEQ ID NO: 54.

In some embodiments:
(i) the first polynucleotide comprises a nucleic acid sequence according to SEQ ID NO: 47 or a variant having at least 80% sequence identity to SEQ ID NO: 47;
(ii) the second polynucleotide comprises a nucleic acid sequence according to SEQ ID NO: 52 or a variant having at least 80% sequence identity to SEQ ID NO: 52; and
(iii) the third polynucleotide comprises a nucleic acid sequence according to SEQ ID NO: 55 or a variant having at least 80% sequence identity to SEQ ID NO: 55.

In some embodiments:
(i) the first polynucleotide comprises a nucleic acid sequence according to SEQ ID NO: 48 or a variant having at least 80% sequence identity to SEQ ID NO: 48;
(ii) the second polynucleotide comprises a nucleic acid sequence according to SEQ ID NO: 52 or a variant having at least 80% sequence identity to SEQ ID NO: 52; and
(iii) the third polynucleotide comprises a nucleic acid sequence according to SEQ ID NO: 56 or a variant having at least 80% sequence identity to SEQ ID NO: 56.

In some embodiments:
(i) the first polynucleotide comprises a nucleic acid sequence according to SEQ ID NO: 49 or a variant having at least 80% sequence identity to SEQ ID NO: 49;
(ii) the second polynucleotide comprises a nucleic acid sequence according to SEQ ID NO: 53 or a variant having at least 80% sequence identity to SEQ ID NO: 53; and
(iii) the third polynucleotide comprises a nucleic acid sequence according to SEQ ID NO: 54 or a variant having at least 80% sequence identity to SEQ ID NO: 54.

In some embodiments:
(i) the first polynucleotide comprises a nucleic acid sequence according to SEQ ID NO: 50 or a variant having at least 80% sequence identity to SEQ ID NO: 50;
(ii) the second polynucleotide comprises a nucleic acid sequence according to SEQ ID NO: 53 or a variant having at least 80% sequence identity to SEQ ID NO: 53; and
(iii) the third polynucleotide comprises a nucleic acid sequence according to SEQ ID NO: 55 or a variant having at least 80% sequence identity to SEQ ID NO: 55.

In some embodiments:
(i) the first polynucleotide comprises a nucleic acid sequence according to SEQ ID NO: 51 or a variant having at least 80% sequence identity to SEQ ID NO: 51;
(ii) the second polynucleotide comprises a nucleic acid sequence according to SEQ ID NO: 53 or a variant having at least 80% sequence identity to SEQ ID NO: 53; and
(iii) the third polynucleotide comprises a nucleic acid sequence according to SEQ ID NO: 56 or a variant having at least 80% sequence identity to SEQ ID NO: 56.

The present invention provides a vector which comprises one or more polynucleotide(s) according to the invention.

The present invention provides a composition comprising
(i) the bispecific binding molecule according to the invention;
(ii) the one or more polynucleotide(s) according to the invention; or
(iii) the vector according to the invention.

The present invention provides a pharmaceutical composition comprising:
(i)
   (a) the bispecific binding molecule according to the invention; or
   (b) the one or more polynucleotide(s) according to the invention; or
   (c) the vector according to the invention; or
   (d) the composition according to the invention;
   and
(ii) a pharmaceutically acceptable carrier, diluent or excipient.

In some embodiments, the pharmaceutical composition comprises:
(i) the bispecific binding molecule according to the invention; and
(ii) a pharmaceutically acceptable carrier, diluent or excipient.

In some embodiments, the pharmaceutical composition comprises:
(i) the one or more polynucleotide(s) according to the invention; and
(ii) a pharmaceutically acceptable carrier, diluent or excipient.

In some embodiments, the pharmaceutical composition comprises:
(i) the vector according to the invention;
   and
(ii) a pharmaceutically acceptable carrier, diluent or excipient.

In some embodiments, the pharmaceutical composition comprises:
(i) the composition according to the invention;
   and
(ii) a pharmaceutically acceptable carrier, diluent or excipient.

In some embodiments, the pharmaceutical composition further comprises a vesicle, a nanoparticle, a lipid nanoparticle (LNP), a liposome or a polymeric mixture.

In some embodiments, the bispecific binding molecule, the one or more polynucleotide(s), the vector, or the composition is encapsulated within the vesicle, nanoparticle, LNP, liposome or polymeric mixture.

In some embodiments, the pharmaceutical composition further comprises an LNP.

In some embodiments, the pharmaceutical composition is formulated for parenteral administration.

In some embodiments, the pharmaceutical composition is formulated for intravenous administration.

In some embodiments, the pharmaceutical composition is formulated for intraarterial administration.

The present invention provides a method for treating and/or preventing a disease which comprises the step of administering the bispecific binding molecule according to the invention; or the one or more polynucleotide(s) according to the invention; or the vector according to the invention; or the composition according to the invention, or the pharmaceutical composition according to the invention, to a subject.

The present invention provides use of the bispecific binding molecule according to the invention; or the one or more polynucleotide(s) according to the invention; or the vector according to the invention; or the composition according to the invention, or the pharmaceutical composition according to the invention, in the manufacture of a medicament for treating and/or preventing a disease.

The present invention provides the bispecific binding molecule according to the invention; or the one or more polynucleotide(s) according to the invention; or the vector according to the invention; or the composition according to the invention, or the pharmaceutical composition according to the invention, for use in the treatment or prevention of a disease.

In some embodiments, the disease is cancer.

In some embodiments, the cancer is prostate cancer.

### BRIEF DESCRIPTION OF THE FIGURES

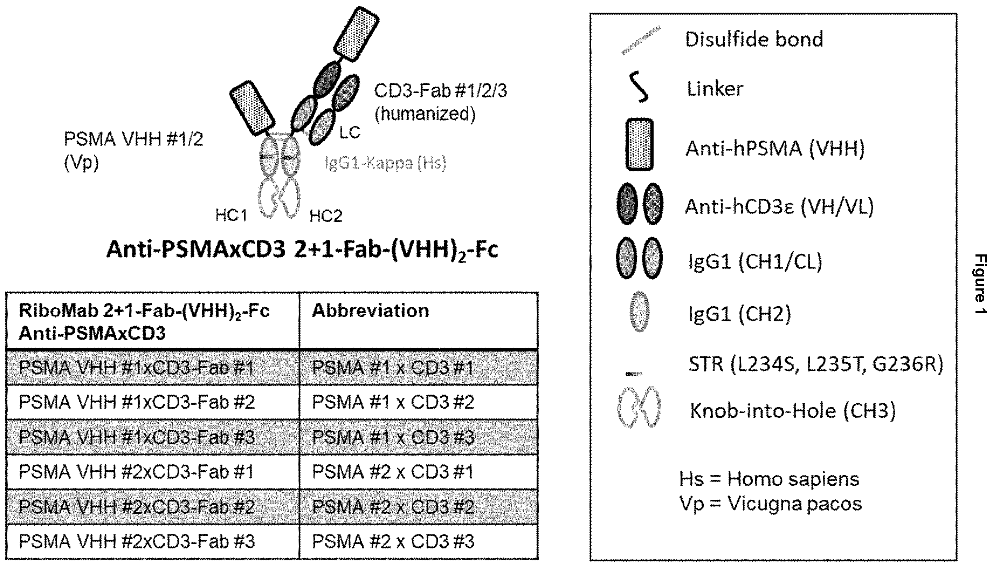

Figure 1 - Structure of the anti-PSMAxCD3 2+1-Fab-(VHH)₂-Fc RiboMab. The 2+1-Fab-(VHH)2-Fc RiboMab consists of three different polypeptide chains. One heavy chain comprises a human PSMA binding alpaca VHH and a human IgG1 Fc part with advanced hole mutations and the Fc-silencing STR mutations. The other heavy chain is generated from the same PSMA binding VHH, a C-terminally via short glycine serine linker fused human CD3ε binding humanized Fab. The Fab is C-terminally linked to an Fc-part with corresponding advanced knob mutations.

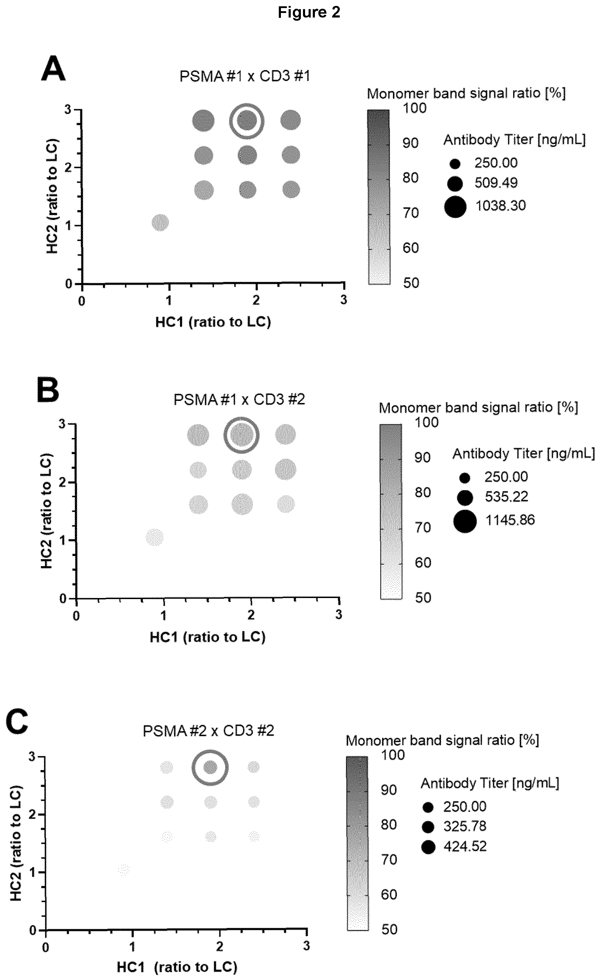

Figure 2 - Optimal ratios for the application of the individual RiboMab IVT-RNA chains. The optimal ratio of the individual RNA chains was determined via the level of antibody expression titer via sandwich ELISA, proportional to the size of the individual data point and the ratio of fully assembled antibody (monomer) in relation to aggregated or not fully assembled chains via Western Blot analysis, illustrated by the opacity of the data point.

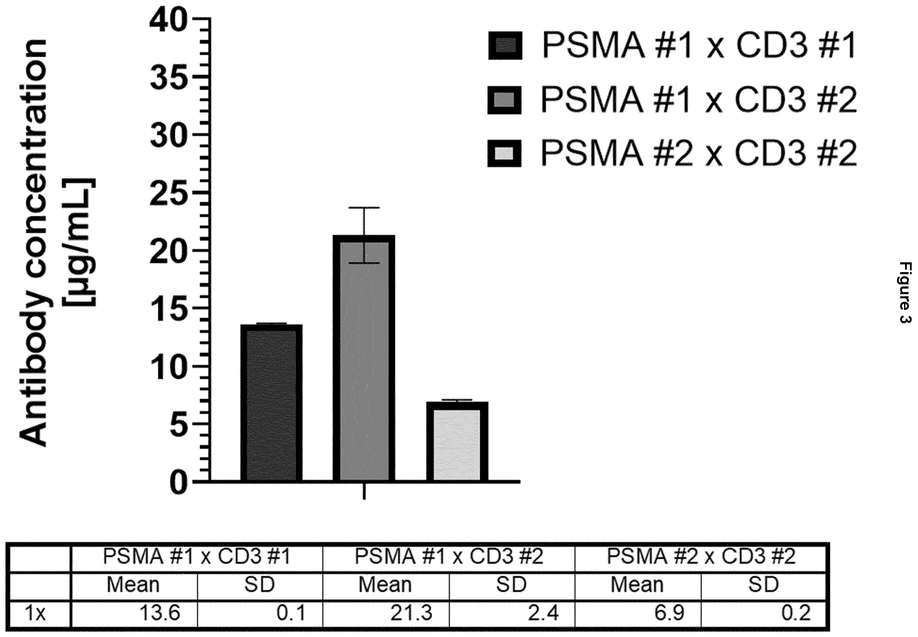

Figure 3 - Production titers of anti-PSMAxCD3 RiboMabs after Ribofection of HEK cells. HEK Expi293F cells were transiently transfected with three different anti-PSMAxCD3 RiboMab coding RNA mixes via ribofection. Resulting antibody titers were determined via Gyros ELISA. The results are a mean of two technical replicates. All supernatant samples were unconcentrated (1x).

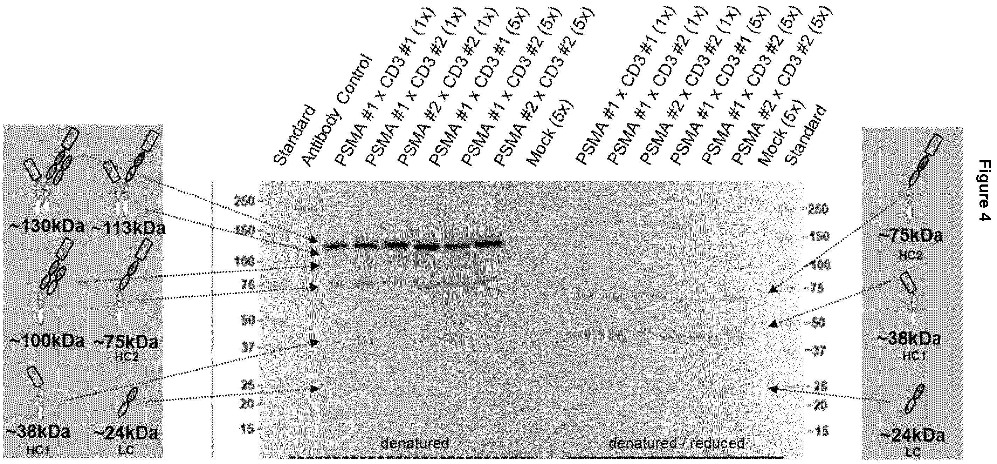

Figure 4 - Western blot analysis of in vitro produced anti-PSMAxCD3 RiboMabs. The Western blot shows several protein species present in the supernatant four days after transfection, indicated with arrows. All three individual chains are present, and the fully assembled antibody is the dominant species in all samples, both unconcentrated (1x) and five times concentrated (5x)

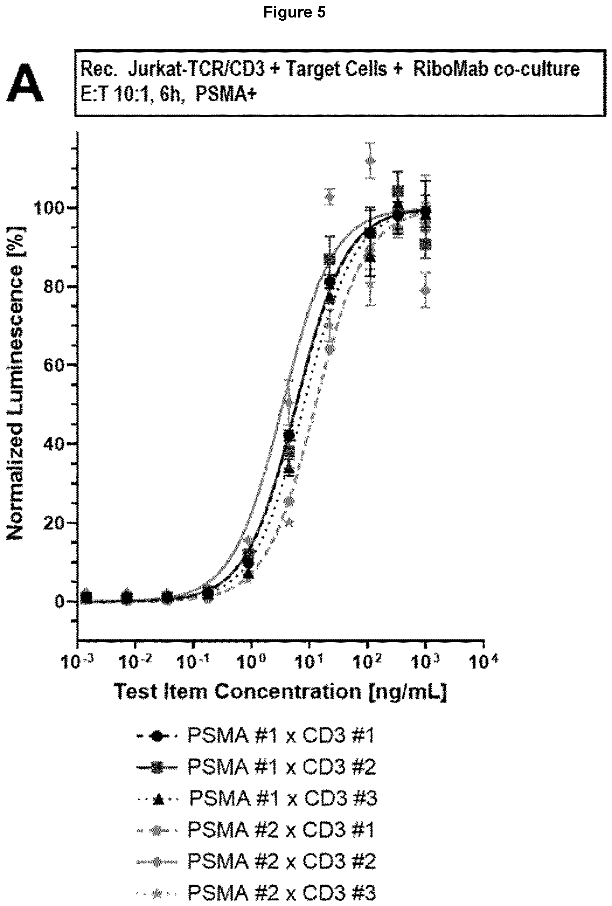

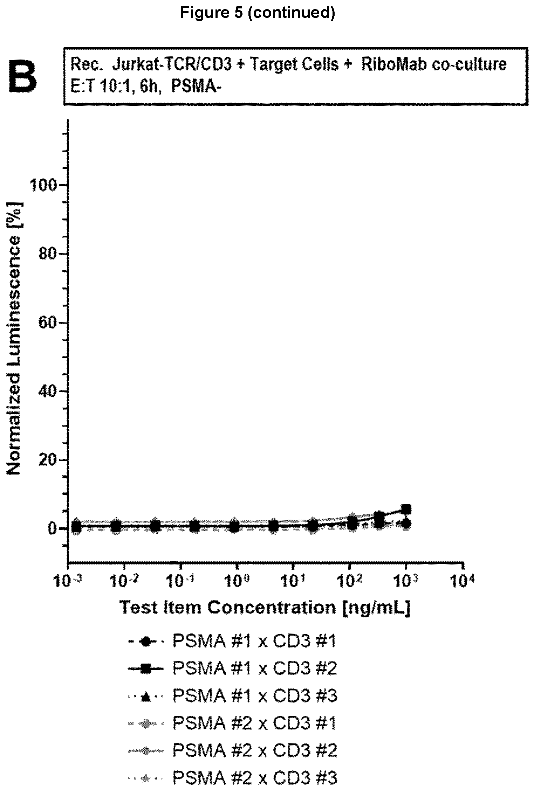

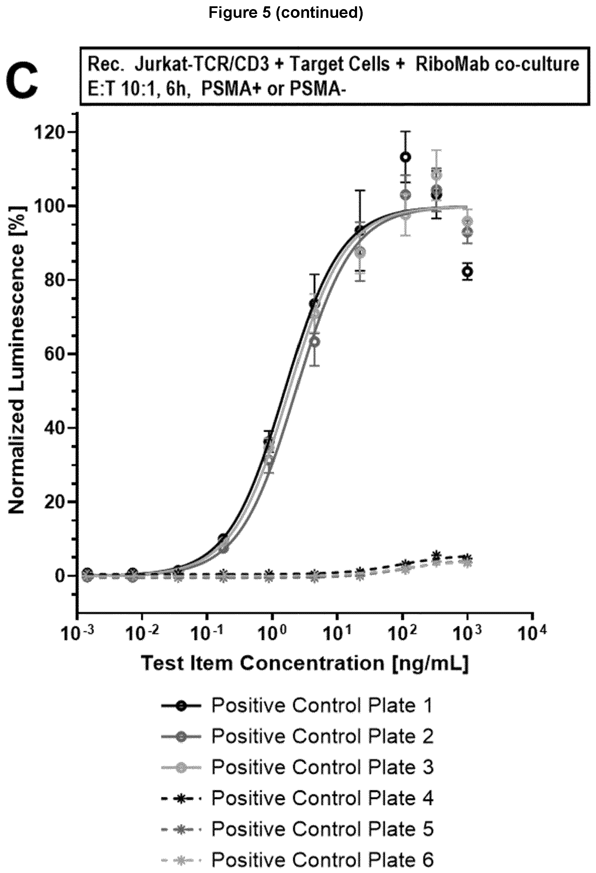

Figure 5 - Recombinant Jurkat-TCR/CD3 activation assay shows binding and specific activation via CD3 for all RiboMabs. The graphs show the results of the T-cell activation bioreporter assay. The abscissa represents the RiboMab concentration in the supernatant as determined by ELISA and the ordinate value is the luminescence normalized to the upper and lower baseline value determined by dose-response curve fit for each individual sample. The error bars are calculated from the standard deviation of three individual test well replicates. The assay was conducted with (A) PSMA-positive LNCaP cells or (B) PSMA-negative HEK293/T17 cells. (C) The positive controls on the different 96-well plates yield similar EC₅₀ values and the negative controls show no increased luminescence.

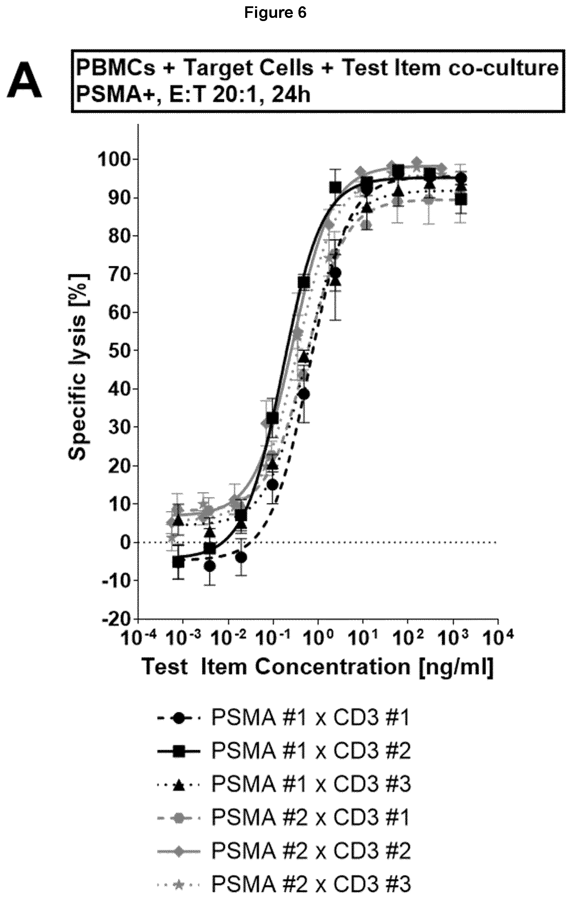

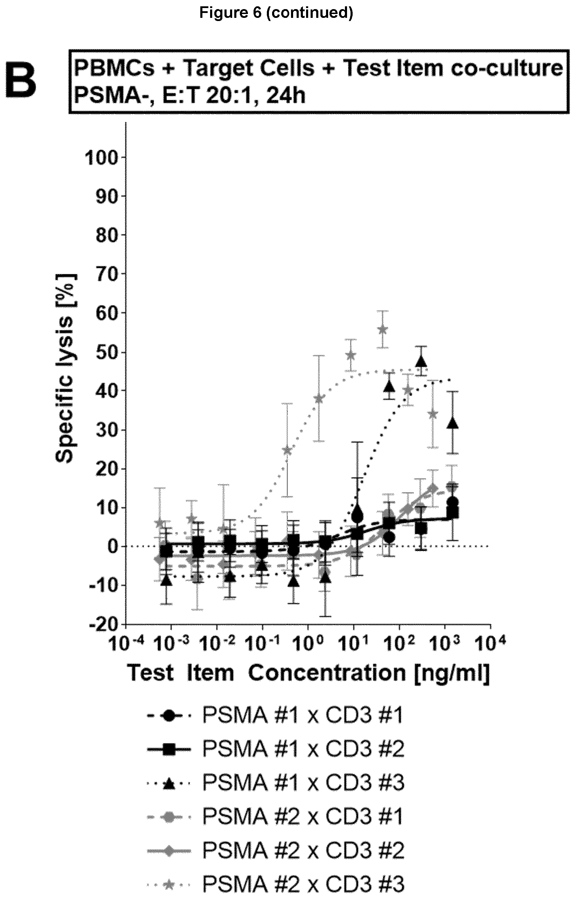

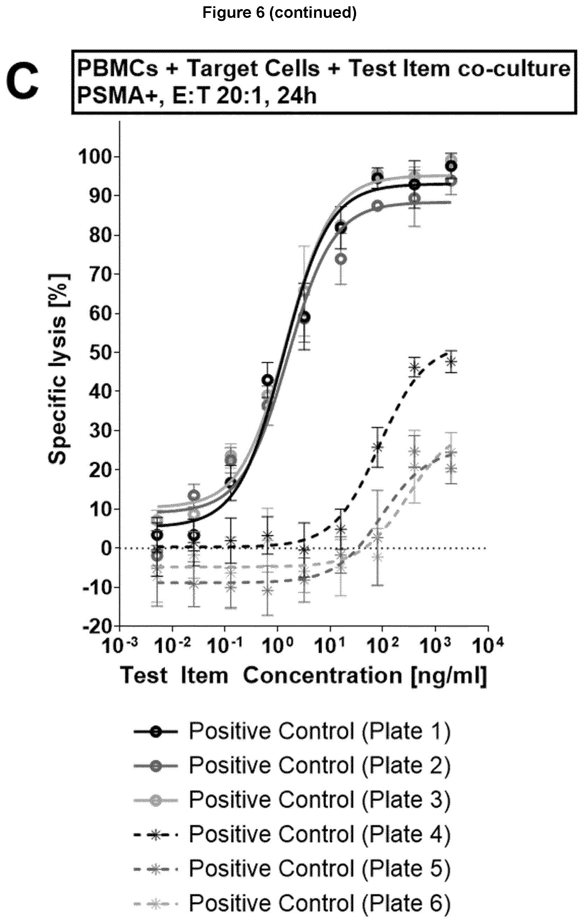

Figure 6 - T-cell dependent cellular cytotoxicity assay verifies potent, specific and concentration dependent lysis mediated by RiboMabs when co-cultured with PBMCs and target presenting cells. The graphs show the increase in specific lysis as it relates to increasing RiboMab concentrations in cell culture supernatant, as determined by sandwich ELISA. The error bars are calculated from the standard deviation of three individual test well replicates. The assay was conducted with (A) PSMA-positive LNCaP cells or (B) PSMA-negative HEK293/T17 cells. (C) The positive controls on the different 96-well plates yield similar EC₅₀ values and the negative controls show no lysis.

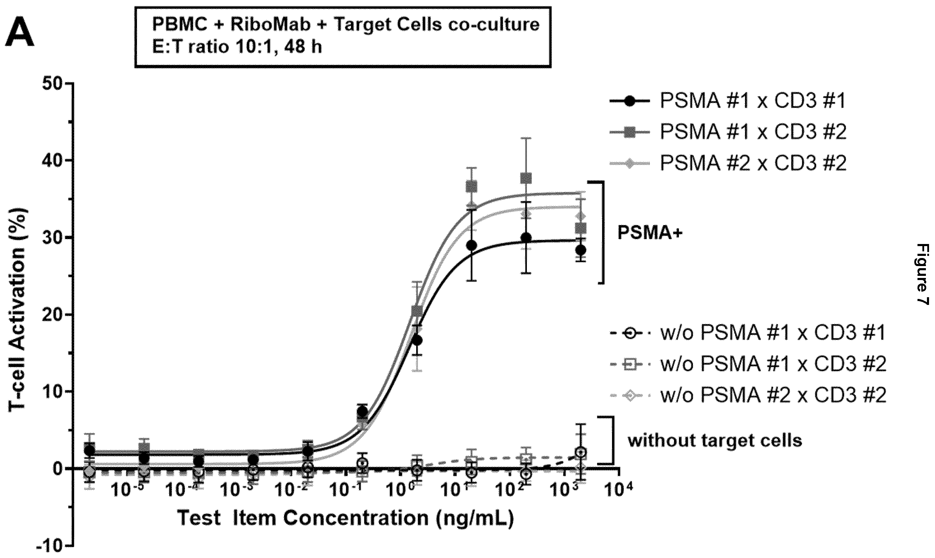

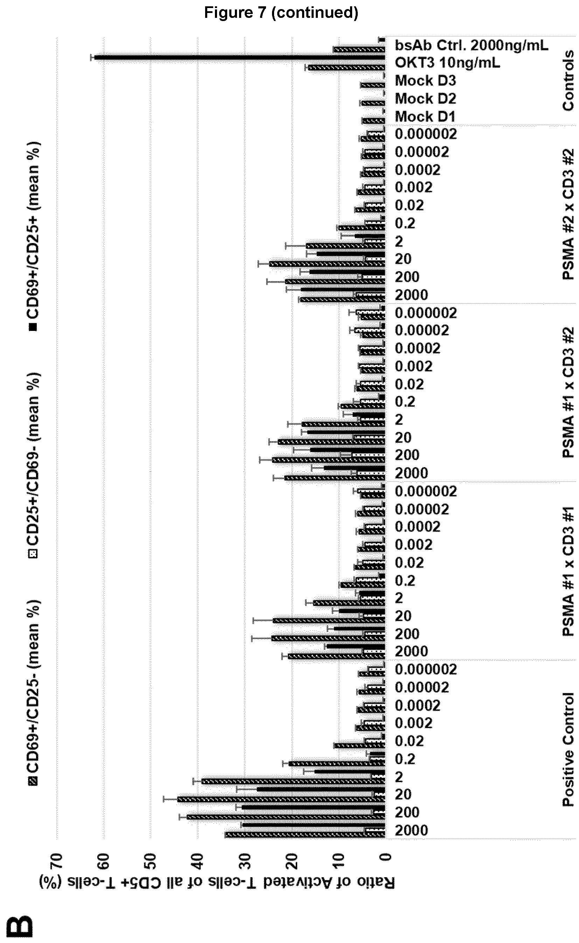

Figure 7 - Bispecific PSMAxCD3 RiboMabs induce T-cell activation via CD3 binding, specifically in the presence of PSMA expressing cells. Cell staining to monitor CD5, CD25 and CD69 expression on PBMCs yielded the (A) ratio of activated T-cells and (B) the individual T-cell activation status concerning early activation marker CD69 and later activation marker CD25.

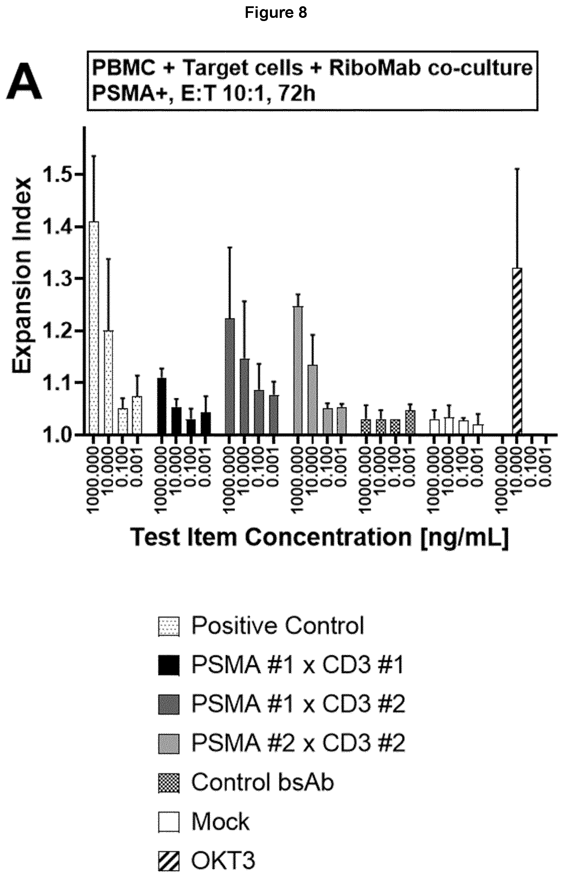

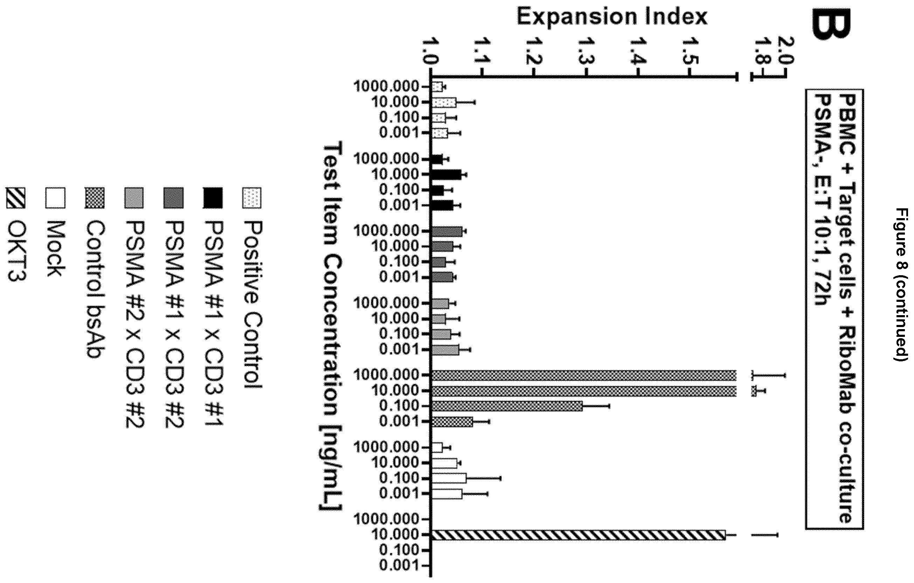

Figure 8 - RiboMabs convey target specific and concentration dependent induction of T-cell proliferation. The proliferation results after 72 h of incubation of a mix of target cells, PBMCs (E:T 10:1) and RiboMabs in different concentrations are depicted here as expansion indices (ratio of the number of cells after incubation time and cells at the start). The error bars are representations of the standard deviation based on three replicates. The assay was conducted with (A) PSMA-positive cells and (B) PSMA-negative cells.

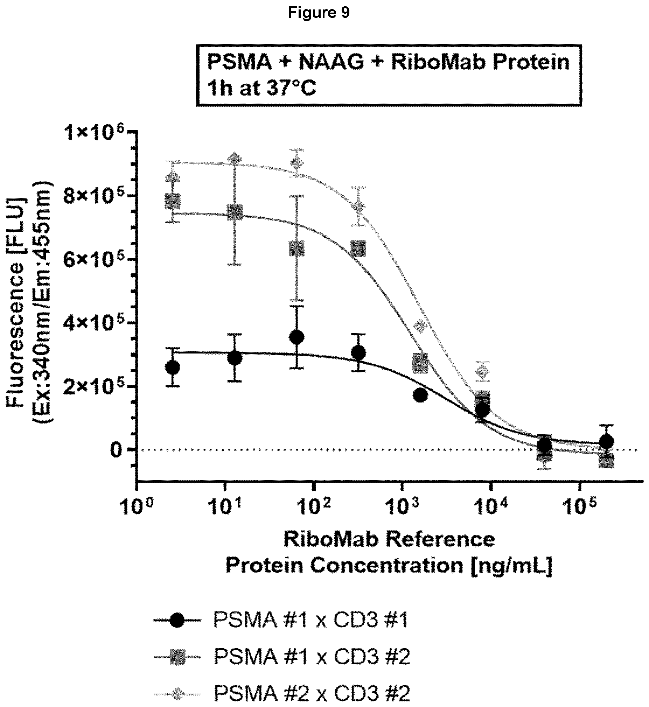

Figure 9 - PSMA glutamate carboxypeptidase inhibition assay. Inhibition of the glutamate carboxypeptidase function of PSMA of the reference proteins of several RiboMab candidates were examined using purified, active human PSMA and NAAG as substrate.

### DETAILED DESCRIPTION OF THE INVENTION

### PSMA

An illustrative amino acid sequence of PSMA is the human PSMA sequence designated as UniProt: Q04609.

In some embodiments, PSMA as described herein is defined according to the UniProt entry: Q04609 (UniProtKB online database Release 2023_05).

In some embodiments, PSMA is human PSMA.

In some embodiments, PSMA comprises an amino acid sequence according to SEQ ID NO: 57 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 57. In some embodiments, the PSMA antigen consists of an amino acid sequence according to SEQ ID NO: 57.

SEQ ID NO: 57
MWNLLHETDSAVATARRPRWLCAGALVLAGGFFLLGFLFGWFIKSSNEATNITPKHNMKAF
LDELKAENIKKFLYNFTQIPHLAGTEQNFQLAKQIQSQWKEFGLDSVELAHYDVLLSYPNKTH
PNYISIINEDGNEIFNTSLFEPPPPGYENVSDIVPPFSAFSPQGMPEGDLVYVNYARTEDFFK
LERDMKINCSGKIVIARYGKVFRGNKVKNAQLAGAKGVILYSDPADYFAPGVKSYPDGWNLP
GGGVQRGNILNLNGAGDPLTPGYPANEYAYRRGIAEAVGLPSIPVHPIGYYDAQKLLEKMG
GSAPPDSSWRGSLKVPYNVGPGFTGNFSTQKVKMHIHSTNEVTRIYNVIGTLRGAVEPDRY
VILGGHRDSWVFGGIDPQSGAAVVHEIVRSFGTLKKEGWRPRRTILFASWDAEEFGLLGST
EWAEENSRLLQERGVAYINADSSIEGNYTLRVDCTPLMYSLVHNLTKELKSPDEGFEGKSLY
ESWTKKSPSPEFSGMPRISKLGSGNDFEVFFQRLGIASGRARYTKNWETNKFSGYPLYHSV
YETYELVEKFYDPMFKYHLTVAQVRGGMVFELANSIVLPFDCRDYAVVLRKYADKIYSISMK
HPQEMKTYSVSFDSLFSAVKNFTEIASKFSERLQDFDKSNPIVLRMMNDQLMFLERAFIDPL
GLPDRPFYRHVIYAPSSHNKYAGESFPGIYDALFDIESKVDPSKAWGEVKRQIYVAAFTVQA
AAETLSEVA

### BINDING MOLECULE

The present invention provides a bispecific binding molecule comprising a CD3-binding moiety and a first prostate-specific membrane antigen (PSMA)-binding moiety.

In some embodiment, the binding molecule comprises an antigen-binding molecule comprising a CD3-binding moiety and a first prostate-specific membrane antigen (PSMA)-binding moiety.

In some embodiments, the bispecific binding molecule comprises one or more amino acid sequence(s) as shown in Table 1.

In some embodiments, the bispecific binding molecule comprises a bispecific antibody.

A "binding moiety" as used herein (e.g. a CD3-binding moiety or a PSMA binding moiety) may comprise an antigen-binding domain, which is well-known term in the art and typically means a protein or polypeptide comprising at least one complementarity determining region(s) (CDR(s)).

It will be understood that an binding domain is a domain of a molecule that interacts with a target (e.g., a target antigen) such that a stable association is formed. An example of a molecule comprising an binding domain is an antibody (or antibody-like molecule) or fragment thereof that binds to its cognate antigen. Such binding interaction will be known in the art. The binding interaction may be non-covalent, reversible covalent or irreversible covalent.

In some embodiments, a binding moiety as used herein (e.g. a CD3-binding moiety and/or a PSMA binding moiety) comprises one or more complementarity determining regions (CDRs).

It will be understood that the bispecific binding molecule according to the invention is capable of binding to (i) one or more PSMA antigen(s) associated with (e.g. presented on the surface of) tumour cells and/or (ii) CD3 associated with (e.g. presented on the surface of) T cells. Thus, the bispecific binding molecule according to the invention is capable of recruiting T cells to the site of the tumour cells.

In some embodiments, the CD3-binding moiety of the bispecific binding molecule is capable of binding to T cells. In some embodiments, the CD3-binding moiety of the bispecific binding molecule is capable of recruiting T cells.

In some embodiments, the T cells are cytotoxic T cells. In some embodiments, the T cells are capable of killing target cells. In some embodiments, the T cells are capable of killing target tumour or cancer cells.

Accordingly, it will also be understood that the bispecific binding molecule according to the invention may be defined as a T cell engager molecule.

In some embodiments, the bispecific binding molecule further comprises a second PSMA-binding moiety.

It will be understood that the bispecific binding molecule according to the invention may therefore comprise a CD3 binding moiety, a first PSMA-binding moiety, and a second PSMA-binding moiety.

In some embodiments, the bispecific binding molecule comprises:
(i) a first PSMA-binding moiety;
(ii) a second PSMA-binding moiety; and
(iii) a CD3-binding moiety.

It will be understood that a bispecific binding molecule comprising (i), (ii) and (iii) as described above may be defined as a (PSMA-binding moiety)₂-CD3 binding moiety format.

In some embodiments, the first PSMA-binding moiety comprises a PSMA-binding single domain variable heavy immunoglobulin (VHH) domain.

In some embodiments, the second PSMA-binding moiety comprises a PSMA-binding VHH domain.

In some embodiments, the first PSMA-binding moiety comprises a PSMA-binding VHH domain and the second PSMA-binding moiety comprises a PSMA-binding VHH domain. In some embodiments, each PSMA-binding moiety comprises a PSMA-binding VHH domain.

In some embodiments, the first PSMA-binding moiety and the second PSMA-binding moiety are the same.

In some embodiments, the CD3-binding moiety comprises a CD3 heavy chain variable (CD3-VH) domain.

In some embodiments, the CD3-binding moiety comprises a CD3 light chain variable (CD3-VL) domain.

In some embodiments, the CD3-binding moiety comprises a CD3 heavy chain variable (CD3-VH) domain and CD3 light chain variable (CD3-VL) domain.

The terms "heavy chain variable domain" or "VH" may refer to the portion of the heavy chain of a binding molecule, antigen-binding molecule or antibody, that typically contains three CDRs, where each CDR is positioned between stretches of amino acids known as framework regions that form a scaffold to support the CDRs.

The terms "light chain variable domain" or "VL" may refer to the fragment of the light chain of a -binding molecule, antigen-binding molecule or antibody, that typically contains three CDRs, where each CDR is positioned between stretches of amino acids known as framework regions that form a scaffold to support the CDRs.

The terms "complementarity determining region" or "CDR" refer to a highly variable loop in the variable region which interact with a cognate antigen and is largely responsible for determining the ability of a binding molecule (e.g., an antibody) to bind to an antigen and for determining binding affinity. The CDRs within a variable region are typically numbered from the amino to the carboxy terminus, with CDR1 being closest to the amino terminus of the variable region and CDR3 being closest to the carboxy terminus of the variable region.

It will also be understood that each CDR is positioned between stretches of amino acids known as framework regions that form a scaffold to support the CDRs.

In some embodiments, the bispecific binding molecule comprises:
(i) a first PSMA-binding moiety;
(ii) a second PSMA-binding moiety; and
(iii) a CD3-binding moiety comprising:
   (i) a heavy chain comprising a CD3 heavy chain variable (CD3-VH) domain; and
   (ii) a light chain comprising a CD3 light chain variable (CD3-VL) domain.

In some embodiments, the bispecific binding molecule comprises:
(i) a first PSMA-binding moiety comprising a PSMA-binding VHH domain;
(ii) a second PSMA-binding moiety comprising a PSMA-binding VHH domain; and
(iii) a CD3-binding moiety comprising:
   (i) a heavy chain comprising a CD3 heavy chain variable (CD3-VH) domain; and
   (ii) a light chain comprising a CD3 light chain variable (CD3-VL) domain.

In some embodiments, the CD3-binding moiety comprises a fragment binding region (Fab).

It will be understood that a Fab typically comprises: (i) a heavy chain comprising one heavy constant (CH1) domain and one heavy variable (VH) domain; and (ii) a light chain comprising one light constant domain (CL) and one light variable (VL) domain.

In some embodiments, the CD3-binding moiety comprises a Fab comprising:
(i) a heavy chain comprising a CD3 heavy chain variable (CD3-VH) domain and a CD3 heavy chain constant (CD3-CH1) domain; and
(ii) a light chain comprising a CD3 light chain variable (CD3-VL) domain and a CD3 light chain constant (CD3-CL) domain.

In some embodiments, the bispecific binding molecule comprises:
(i) a first PSMA-binding moiety comprising a PSMA-binding VHH domain;
(ii) a second PSMA-binding moiety comprising a PSMA-binding VHH domain; and
(iii) a CD3-binding moiety comprising a Fab, wherein the Fab comprises:
   (i) a heavy chain comprising a CD3 heavy chain variable (CD3-VH) domain and a CD3 heavy chain constant (CD3-CH1) domain; and
   (ii) a light chain comprising a CD3 light chain variable (CD3-VL) domain and a CD3 light chain constant (CD3-CL) domain.

It will be understood that a bispecific binding molecule comprising (i), (ii) and (iii) as described above may be defined as a (VHH)₂-Fab format.

In some embodiments, the bispecific binding molecule further comprises an Fc region comprising a first subunit and a second subunit.

In some embodiments, the bispecific binding molecule comprises:
(i) a first PSMA-binding moiety comprising a PSMA-binding VHH domain;
(ii) a second PSMA-binding moiety comprising a PSMA-binding VHH domain;
(iii) a CD3-binding moiety comprising a Fab, wherein the Fab comprises:
   (i) a heavy chain comprising a CD3 heavy chain variable (CD3-VH) domain and a CD3 heavy chain constant (CD3-CH1) domain; and
   (ii) a light chain comprising a CD3 light chain variable (CD3-VL) domain and a CD3 light chain constant (CD3-CL) domain; and
(iv) an Fc region comprising a first subunit and a second subunit.

In some embodiments, the bispecific binding molecule comprises:
(i) a first PSMA-binding moiety comprising a PSMA-binding VHH domain;
(ii) a second PSMA-binding moiety comprising a PSMA-binding VHH domain;
(iii) a CD3-binding moiety comprising a Fab, wherein the Fab comprises:
   (i) a heavy chain comprising a CD3 heavy chain variable (CD3-VH) domain and a CD3 heavy chain constant (CD3-CH1) domain; and
   (ii) a light chain comprising a CD3 light chain variable (CD3-VL) domain and a CD3 light chain constant (CD3-CL) domain; and
(iv) an Fc region comprising
   (a) a first subunit comprising a CH2 domain and a CH3 domain; and
   (b) a second subunit comprising a CH2 domain and a CH3 domain.

It will be understood that the first PSMA-binding moiety, the second PSMA-binding moiety, the CD3-binding moiety and/or the Fc region may be arranged in any format.

In some embodiments, the first PSMA-binding moiety is linked to the CD3-binding moiety.

In some embodiments, the C-terminus of the first PSMA-binding moiety is linked to the N-terminus of the CD3-binding moiety.

It will be understood that any suitable linker molecule can be used to link the first PSMA-binding moiety to the CD3-binding moiety.

In some embodiments, the linker that links the first PSMA-binding moiety to the CD3-binding moiety is a peptide linker. In some embodiments, the linker comprises one or more serine amino acid(s). In some embodiments, the linker comprises one or more glycine amino acid(s). In some embodiments, the linker comprises one or more serine amino acid(s) and one or more glycine amino acid(s).

In some embodiments, the linker that links the first PSMA-binding moiety to the CD3-binding moiety has a length of 10 to 30 amino acids. In some embodiments, the linker has a length of 10 to 30 amino acids and comprises one or more serine amino acid(s) and one or more glycine amino acid(s).

In some embodiments, the linker that links the first PSMA-binding moiety to the CD3-binding moiety is a (G4S)₂ linker.

In some embodiments, the linker that links the first PSMA-binding moiety to the CD3-binding moiety comprises an amino acid sequence according to SEQ ID NO: 58 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 58. In some embodiments, the linker consists of an amino acid sequence according to SEQ ID NO: 58.

In some embodiments, the C-terminus of the first PSMA-binding moiety is linked to the N-terminus of the CD3-VH domain

In some embodiments, the C-terminus of the first PSMA-binding moiety is linked to the N-terminus of the CD3-VL domain.

In some embodiments, the C-terminus of the PSMA-binding VHH domain of the first PSMA-binding moiety is linked to the N-terminus of the CD3-VH domain

In some embodiments, the C-terminus of the PSMA-binding VHH domain of the first PSMA-binding moiety is linked to the N-terminus of the CD3-VL domain.

In some embodiments, the CD3-binding moiety is linked to the Fc region.

It will be understood that any suitable linker molecule can be used to link the CD3-binding moiety is linked to the Fc region.

In some embodiments, the linker that links the CD3-binding moiety is linked to the Fc region is a peptide linker. In some embodiments, the linker comprises one or more serine amino acid(s). In some embodiments, the linker comprises one or more glycine amino acid(s). In some embodiments, the linker comprises one or more serine amino acid(s) and one or more glycine amino acid(s).

In some embodiments, the linker that links the CD3-binding moiety is linked to the Fc region has a length of 10 to 30 amino acids. In some embodiments, the linker has a length of 10 to 30 amino acids and comprises one or more serine amino acid(s) and one or more glycine amino acid(s).

In some embodiments, the linker that links the CD3-binding moiety to the Fc region is a (G4S)₂ linker.

In some embodiments, the linker that links the CD3-binding moiety to the Fc region comprises an amino acid sequence according to SEQ ID NO: 58 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 58. In some embodiments, the linker consists of an amino acid sequence according to SEQ ID NO: 58.

In some embodiments, the C-terminus of the CD3-binding moiety is linked to the N-terminus of the first subunit of the Fc region.

In some embodiments, the C-terminus of the CD3-binding moiety is linked to a hinge region of the first subunit of the Fc region.

In some embodiments, the C-terminus of the CD3-binding moiety is linked to the first subunit of the Fc region via the hinge region, which is part of both the CH1 and CH2 domain.

In some embodiments, the C-terminus of the CD3-binding moiety is linked to the CH2 domain of the first subunit of the Fc region, preferably to the N-terminus of the CH2 domain of the first subunit of the Fc region, more preferably to the N-terminus of a hinge region of the CH2 domain of the first subunit of the Fc region.

In some embodiments, the C-terminus of the CD3-binding moiety is linked to a hinge region which is linked to the CH2 domain of the first subunit of the Fc region. In some embodiments, the C-terminus of the CD3-binding moiety comprises part of a hinge region, wherein the hinge region also comprises part of the N-terminus of the CH2 domain of the first subunit of the Fc region.

In some embodiments, the C-terminus of the CD3-binding moiety is linked to the N-terminus of a CH2 domain of the second subunit of the Fc region.

In some embodiments, the C-terminus of the CD3-binding moiety is linked to a hinge region which is linked to the N-terminus of a CH2 domain of the second subunit of the Fc region. In some embodiments, the C-terminus of the CD3-binding moiety comprises part of a hinge region, wherein the hinge region also comprises part of the N-terminus of a CH2 domain of the second subunit of the Fc region.

In some embodiments, the C-terminus of the CD3-CH1 domain is linked to the first subunit of the Fc region.

In some embodiments, the C-terminus of the CD3-CH1 domain is linked to a hinge region of the first subunit of the Fc region

In some embodiments, the C-terminus of the CD3-CH1 domain is linked to the first subunit of the Fc region via a hinge region, which is part of both the CH1 and CH2 domain.

In some embodiments, the C-terminus of the CD3-CH1 domain is linked to a hinge region which is linked to the first subunit of the Fc region. In some embodiments, the C-terminus of the CD3-CH1 domain comprises part of a hinge region, wherein the hinge region also comprises part of the first subunit of the Fc region.

In some embodiments, the C-terminus of the CD3-CH1 domain is linked to the CH2 domain of the first subunit of the Fc region.

In some embodiments, the C-terminus of the CD3-CH1 domain is linked to a hinge region which is linked to the CH2 domain of the first subunit of the Fc region. In some embodiments, the C-terminus of the CD3-CH1 domain comprises part of a hinge region, wherein the hinge region also comprises part of the CH2 domain of the first subunit of the Fc region.

In some embodiments, the C-terminus of the CD3-CH1 domain is linked to the N-terminus of a CH2 domain of the second subunit of the Fc region, preferably to the N-terminus of a hinge region of the CH2 domain of the second subunit of the Fc region.

In some embodiments, the C-terminus of the CD3-CH1 domain is linked to a hinge region which is linked to the N-terminus of a CH2 domain of the second subunit of the Fc region. In some embodiments, the C-terminus of the CD3-CH1 domain comprises part of a hinge region, wherein the hinge region also comprises part of the N-terminus of a CH2 domain of the second subunit of the Fc region.

In some embodiments, the C-terminus of the CD3-CL domain is linked to the first subunit of the Fc region.

In some embodiments, the C-terminus of the CD3-CL domain is linked to a hinge region of the first subunit of the Fc region

In some embodiments, the C-terminus of the CD3-CL domain is linked to the first subunit of the Fc region via the hinge region, which is part of both the CH1 and CH2 domain.

In some embodiments, the C-terminus of the CD3-CL domain is linked to a hinge region which is linked to the first subunit of the Fc region. In some embodiments, the C-terminus of the CD3-CL domain comprises part of a hinge region, wherein the hinge region also comprises part of the first subunit of the Fc region.

In some embodiments, the C-terminus of the CD3-CL domain is linked to the CH2 domain of the first subunit of the Fc region.

In some embodiments, the C-terminus of the CD3-CL domain is linked to a hinge region which is linked to the CH2 domain of the first subunit of the Fc region. In some embodiments, the C-terminus of the CD3-CL domain comprises part of a hinge region, wherein the hinge region also comprises part of the CH2 domain of the first subunit of the Fc region.

In some embodiments, the C-terminus of the CD3-CL domain is linked to the N-terminus of a CH2 domain of the second subunit of the Fc region.

In some embodiments, the C-terminus of the CD3-CL domain is linked to a hinge region which is linked to the N-terminus of a CH2 domain of the second subunit of the Fc region. In some embodiments, the C-terminus of the CD3-CL domain comprises part of a hinge region, wherein the hinge region also comprises part of the N-terminus of a CH2 domain of the second subunit of the Fc region.

In some embodiments, the second PSMA-binding moiety is linked to the Fc region.

In some embodiments, the C-terminus of the second PSMA-binding moiety is linked to the N-terminus of the second subunit of the Fc region.

It will be understood that any suitable linker molecule can be used to link the second PSMA-binding moiety to the second subunit of the Fc region.

In some embodiments, the linker that links the second PSMA-binding moiety to the second subunit of the Fc region is a peptide linker. In some embodiments, the linker comprises one or more serine amino acid(s). In some embodiments, the linker comprises one or more glycine amino acid(s). In some embodiments, the linker comprises one or more serine amino acid(s) and one or more glycine amino acid(s).

In some embodiments, the linker that links the second PSMA-binding moiety to the second subunit of the Fc region has a length of 10 to 30 amino acids. In some embodiments, the linker has a length of 10 to 30 amino acids and comprises one or more serine amino acid(s) and one or more glycine amino acid(s).

In some embodiments, the linker that links the second PSMA-binding moiety to the second subunit of the Fc region is a (G4S)₂ linker.

In some embodiments, the linker that links the second PSMA-binding moiety to the second subunit of the Fc region comprises an amino acid sequence according to SEQ ID NO: 58 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 58. In some embodiments, the linker consists of an amino acid sequence according to SEQ ID NO: 58.

In some embodiments, the C-terminus of the second PSMA-binding moiety is linked to a hinge region which is linked to the second subunit of the Fc region. In some embodiments, the second PSMA-binding moiety is linked to a hinge region, wherein the hinge region also comprises part of the second subunit of the Fc region.

In some embodiments, the C-terminus of the second PSMA-binding moiety is linked to the CH2 domain of the second subunit of the Fc region.

In some embodiments, the C-terminus of the second PSMA-binding moiety is linked to a hinge region which is linked to the CH2 domain of the second subunit of the Fc region. In some embodiments, the C-terminus of the second PSMA-binding moiety is linked to a hinge region, wherein the hinge region also comprises part of the CH2 domain of the second subunit of the Fc region.

In some embodiments, the C-terminus of the second PSMA-binding moiety is linked to the N-terminus of a CH2 domain of the second subunit of the Fc region, preferably to the N-terminus of a hinge region of the CH2 domain of the second subunit of the Fc region.

In some embodiments, the C-terminus of the second PSMA-binding moiety is linked to a hinge region which is linked to the N-terminus of a CH2 domain of the second subunit of the Fc region. In some embodiments, the C-terminus of the second PSMA-binding moiety is linked to a hinge region, wherein the hinge region also comprises part of the N-terminus of a CH2 domain of the second subunit of the Fc region.

In some embodiments, the C-terminus of the PSMA-binding VHH domain of the second PSMA-binding moiety is linked to the second subunit of the Fc region.

In some embodiments, the C-terminus of the PSMA-binding VHH domain of the second PSMA-binding moiety is linked to a hinge region which is linked to the second subunit of the Fc region. In some embodiments, the C-terminus of the PSMA-binding VHH domain of the second PSMA-binding moiety is linked to a hinge region, wherein the hinge region also comprises part of the second subunit of the Fc region.

In some embodiments, the C-terminus of the PSMA-binding VHH domain of the second PSMA-binding moiety is linked to the CH2 domain of the second subunit of the Fc region.

In some embodiments, the C-terminus of the PSMA-binding VHH domain of the second PSMA-binding moiety is linked to a hinge region which is linked to the CH2 domain of the second subunit of the Fc region. In some embodiments, the C-terminus of the PSMA-binding VHH domain of the second PSMA-binding moiety is linked to a hinge region, wherein the hinge region also comprises part of the CH2 domain of the second subunit of the Fc region.

In some embodiments, the C-terminus of the PSMA-binding VHH domain of the second PSMA-binding moiety is linked to the N-terminus of a CH2 domain of the second subunit of the Fc region.

In some embodiments, the C-terminus of the PSMA-binding VHH domain of the second PSMA-binding moiety is linked to a hinge region which is linked to the N-terminus of a CH2 domain of the second subunit of the Fc region. In some embodiments, the C-terminus of the PSMA-binding VHH domain of the second PSMA-binding moiety is linked to a hinge region, wherein the hinge region also comprises part of the N-terminus of a CH2 domain of the second subunit of the Fc region.

In some embodiments, the bispecific binding molecule may comprise a structure as depicted in Figure 1.

In some embodiments, the bispecific binding molecule comprises:
(i) the CD3-binding moiety comprises a Fab, wherein the Fab comprises:
   (a) a heavy chain comprising the CD3-VH domain and a heavy chain constant (CD3-CH1) domain; and
   (b) a light chain comprising the CD3-VL domain and a light chain constant (CD3-CL) domain;
(ii) the bispecific binding molecule further comprises a second PSMA-binding moiety, wherein the first PSMA-binding moiety and the second PSMA-binding moiety are the same, wherein the first PSMA-binding moiety and the second PSMA-binding moiety each comprise a PSMA-binding VHH domain;
(iii) the bispecific binding molecule further comprises an Fc region comprising a first subunit and a second subunit; wherein the first subunit of the Fc region and the second subunit of the Fc region each comprise a CH2 domain and a CH3 domain, and wherein the CH2 domain comprises a hinge region;
(iv) the C-terminus of the PSMA-binding VHH domain of the first PSMA-binding moiety is linked to the N-terminus of the CD3-VH domain;
(v) the C-terminus of the CD3-CH1 domain is linked to the N-terminus of the hinge region of the CH2 domain of the first subunit of the Fc region; and
(vi) the C-terminus of the PSMA-binding VHH domain of the second PSMA-binding moiety is linked to the N-terminus of the hinge region of the CH2 domain of the second subunit of the Fc region.

In some embodiments, the CH3 domain of one subunit of the Fc region comprises one or more mutations selected from the list consisting of: Y349C, T366S, L368A, and Y407V; and the CH3 domain of the other subunit of the Fc region comprises one or more mutations selected from the list consisting of: S354C and T366W.

In some embodiments, the bispecific binding molecule comprises:
(i) a CD3-binding moiety comprising a Fab, wherein the Fab comprises:
   (a) a heavy chain comprising the CD3-VH domain and a heavy chain constant (CD3-CH1) domain; and
   (b) a light chain comprising the CD3-VL domain and a light chain constant (CD3-CL) domain;
(ii) a first PSMA-binding moiety and a second PSMA-binding moiety, wherein the first PSMA-binding moiety and the second PSMA-binding moiety are the same, wherein the first PSMA-binding moiety and the second PSMA-binding moiety each independently comprise a PSMA-binding VHH domain;
(iii) an Fc region comprising a first subunit and a second subunit; wherein the first subunit of the Fc region and the second subunit of the Fc region each independently comprise a CH2 domain and a CH3 domain;
(iv) the C-terminus of the PSMA-binding VHH domain of the first PSMA-binding moiety is linked to the N-terminus of the CD3-VH domain;
(v) the C-terminus of the CD3-CH1 domain is linked to, or comprises part of, a hinge region, wherein the hinge region is linked to, or also comprises part of, the N-terminus of the CH2 domain of the first subunit of the Fc region; and
(vi) the C-terminus of the PSMA-binding VHH domain of the second PSMA-binding moiety is linked to a hinge region, wherein the hinge region is linked to, or also comprises part of, the N-terminus of the CH2 domain of the second subunit of the Fc region.

In some embodiments, the bispecific binding molecule comprises:
(i) a CD3-binding moiety comprising a Fab, wherein the Fab comprises:
   (a) a heavy chain comprising the CD3-VH domain and a heavy chain constant (CD3-CH1) domain; and
   (b) a light chain comprising the CD3-VL domain and a light chain constant (CD3-CL) domain;
(ii) a first PSMA-binding moiety and a second PSMA-binding moiety, wherein the first PSMA-binding moiety and the second PSMA-binding moiety are the same, wherein the first PSMA-binding moiety and the second PSMA-binding moiety each independently comprise a PSMA-binding VHH domain;
(iii) an Fc region comprising a first subunit and a second subunit; wherein the first subunit of the Fc region and the second subunit of the Fc region each independently comprise a CH2 domain and a CH3 domain;
(iv) the C-terminus of the PSMA-binding VHH domain of the first PSMA-binding moiety is linked to the N-terminus of the CD3-VH domain;
(v) the C-terminus of the CD3-CH1 domain comprises part of a hinge region, wherein the hinge region comprises part of the N-terminus of the CH2 domain of the first subunit of the Fc region and part of the C-terminus of the CD3-CH1 domain; and
(vi) the C-terminus of the PSMA-binding VHH domain of the second PSMA-binding moiety is linked to a hinge region, wherein the hinge region comprises part of the N-terminus of the CH2 domain of the second subunit of the Fc region and part of the C-terminus of the PSMA-binding VHH domain.

In some embodiments, the CH3 domain of one subunit of the Fc region comprises one or more mutations selected from the list consisting of: Y349C, T366S, L368A, and Y407V; and the CH3 domain of the other subunit of the Fc region comprises one or more mutations selected from the list consisting of: S354C and T366W.

In some embodiments, the CH2 domain of one subunit of the Fc region comprises one or more STR mutations. In some embodiments, the CH2 domain of both subunits of the Fc region comprises one or more STR mutations.

In some embodiments, the CH2 domain of one subunit of the Fc region comprises L234S, L235T, and G236R mutations. In some embodiments, the CH2 domain of both subunits of the Fc region comprises L234S, L235T, and G236R mutations.

In some embodiments, the CH2 domain of one subunit of the Fc region comprises one or more LALA mutations. In some embodiments, the CH2 domain of both subunits of the Fc region comprises one or more LALA mutations.

In some embodiments, the CH2 domain of one subunit of the Fc region comprises L234A and L235A mutations. In some embodiments, the CH2 domain of both subunits of the Fc region comprises L234A and L235A mutations.

Suitable assays and techniques for measuring/quantifying binding activity of the bispecific binding molecule according to the invention may include, but are not limited to, ELISA, surface plasmon resonance (SPR), bio-layer interferometry (BLI), quartz crystal microbalance (QCM), bioluminescence assays and flow cytometry. Other suitable techniques will be known in the art.

In particular, it will be understood that the ability of a binding molecule to bind to its cognate target may be assessed by flow cytometry, where the effectiveness of a binding molecule to bind to a cognate target expressed by a population of cells can be measured in a flow cytometry assay.

Moreover, it will be understood that EC₅₀ is a measure of the concentration of a binding molecule that induces a specific response that is 50% between the maximum response and the baseline response. As such, EC₅₀ can be used to assess the ability of a binding molecule to bind to a target.

### PSMA-binding moiety

As used herein, a "PSMA-binding moiety" comprises a binding domain that is capable of binding to PSMA. In some embodiments, the, or each, PSMA-binding moiety independently comprises an antigen-binding domain, e.g. a PSMA-binding VHH domain.

It will be understood that the term "PSMA-binding moiety" also encompasses fragments thereof.

In some embodiments, the, or each, PSMA-binding moiety independently binds to SEQ ID NO: 57 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 57.

In some embodiments, the first PSMA-binding moiety and the second PSMA-binding moiety are the same.

In some embodiments, the, or each, PSMA-binding moiety independently comprises one, two or three CDRs.

In some embodiments, the, or each, PSMA-binding moiety independently comprises three CDRs.

It will be understood that CDRs of the, or each, PSMA-binding moiety may be termed HCDRs. It will also be understood that each of the three CDRs may be termed HCDR1, HCDR2 and HCDR3 respectively.

In some embodiments, the, or each, PSMA-binding moiety independently further comprises framework regions. In some embodiments, the framework regions are a scaffold (e.g. amino acid sequences) that support the CDRs such that the, or each, PSMA-binding moiety specifically binds to PSMA via the CDRs.

The CDRs may be determined according to the Kabat definition and/or the IMGT definition, both of which are well-known in the art (e.g. see Edelman et al., Proc Natl Acad Sci U S A. 1969 May;63(1):78-85; Kabat et al., Sequences of Proteins of Immunological Interest, Fifth Edition. 1991 NIH Publication No. 91-3242; and Marie-Paule Lefranc, Véronique Giudicelli, Chantal Ginestoux, Julia Bodmer, Werner Müller, Ronald Bontrop, Marc Lemaitre, Ansar Malik, Valérie Barbié, Denys Chaume, IMGT, the international ImMunoGeneTics database, Nucleic Acids Research, Volume 27, Issue 1, 1 January 1999, Pages 209-212).

It will be understood that a variant of a PSMA-binding moiety as described herein may have an equivalent function to a full length PSMA-binding moiety amino acid sequence as described herein and may suitably maintain the capacity to bind to PSMA.

In some embodiments, the first PSMA-binding moiety and the second PSMA-binding moiety specifically binds to PSMA.

In some embodiments, it will be understood herein that "specifically binds to" refers to the antibody-like binding of the, or each, PSMA-binding moiety, e.g. via the HCDRs 1-3, to its target: PSMA. In some embodiments, "specifically binds to" means that the, or each, PSMA-binding moiety binds to an epitope on PSMA.

In some embodiments, the, or each, PSMA-binding moiety independently comprises heavy chain complementarity determining regions (HCDRs) 1-3, wherein (according to Kabat numbering):
HCDR1 comprises the amino acid sequence according to SEQ ID NO: 29,
HCDR2 comprises the amino acid sequence according to SEQ ID NO: 30, and
HCDR3 comprises the amino acid sequence according to SEQ ID NO: 31.

In some embodiments, one or more of the CDRs comprises one, two or three amino acid mutations relative to the recited sequences.

In some embodiments, the HCDR1 sequence comprises one, two or three amino acid mutations relative to the recited sequences. In some embodiments, the HCDR2 sequence comprises one, two or three amino acid mutations relative to the recited sequences. In some embodiments, the HCDR3 sequence comprises one, two or three amino acid mutations relative to the recited sequences.

In some embodiments, the, or each, PSMA-binding moiety independently comprises heavy chain complementarity determining regions (HCDRs) 1-3, wherein (according to Kabat numbering):
HCDR1 comprises the amino acid sequence according to SEQ ID NO: 36,
HCDR2 comprises the amino acid sequence according to SEQ ID NO: 37, and
HCDR3 comprises the amino acid sequence according to SEQ ID NO: 38.

In some embodiments, one or more of the CDRs comprises one, two or three amino acid mutations relative to the recited sequences.

In some embodiments, the HCDR1 sequence comprises one, two or three amino acid mutations relative to the recited sequences. In some embodiments, the HCDR2 sequence comprises one, two or three amino acid mutations relative to the recited sequences. In some embodiments, the HCDR3 sequence comprises one, two or three amino acid mutations relative to the recited sequences.

In some embodiments, the, or each, PSMA-binding moiety independently comprises heavy chain complementarity determining regions (HCDRs) 1-3, wherein (according to IMGT numbering):
HCDR1 comprises the amino acid sequence according to SEQ ID NO: 32,
HCDR2 comprises the amino acid sequence according to SEQ ID NO: 33, and
HCDR3 comprises the amino acid sequence according to SEQ ID NO: 34.

In some embodiments, one or more of the CDRs comprises one, two or three amino acid mutations relative to the recited sequences.

In some embodiments, the HCDR1 sequence comprises one, two or three amino acid mutations relative to the recited sequences. In some embodiments, the HCDR2 sequence comprises one, two or three amino acid mutations relative to the recited sequences. In some embodiments, the HCDR3 sequence comprises one, two or three amino acid mutations relative to the recited sequences.

In some embodiments, the, or each, PSMA-binding moiety independently comprises heavy chain complementarity determining regions (HCDRs) 1-3, wherein (according to IMGT numbering):
HCDR1 comprises the amino acid sequence according to SEQ ID NO: 39,
HCDR2 comprises the amino acid sequence according to SEQ ID NO: 40, and
HCDR3 comprises the amino acid sequence according to SEQ ID NO: 41.

In some embodiments, one or more of the CDRs comprises one, two or three amino acid mutations relative to the recited sequences.

In some embodiments, the HCDR1 sequence comprises one, two or three amino acid mutations relative to the recited sequences. In some embodiments, the HCDR2 sequence comprises one, two or three amino acid mutations relative to the recited sequences. In some embodiments, the HCDR3 sequence comprises one, two or three amino acid mutations relative to the recited sequences.

In some embodiments, the, or each, PSMA-binding moiety of the invention independently comprises a HCDR1 sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the HCDR1 sequence described herein.

In some embodiments, the, or each, PSMA-binding moiety of the invention independently comprises a HCDR2 sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the HCDR2 sequence described herein.

In some embodiments, the, or each, PSMA-binding moiety of the invention independently comprises a HCDR3 sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the HCDR3 sequence described herein.

It will be understood that a mutation in any of the CDRs described herein may encompass a deletion of an amino acid, an insertion of an amino acid, or a substitution of an amino acid. It will also be understood that such a mutation may not prevent the, or each, PSMA-binding moiety according to the invention from binding to PSMA. In other words, a PSMA-binding moiety according to the invention comprising a mutation in one or more CDRs described herein may suitably maintain the capacity (e.g. affinity) to bind to PSMA. In some embodiments, the mutation suitably maintains the same capacity (e.g. affinity) to bind to PSMA as the parent binding molecule. The term "parent binding molecule" in this context refers to the, or each, PSMA-binding moiety without the mutation(s) in question.

In some embodiments, the, or each, PSMA-binding moiety independently comprises or consists of a sequence having at least 70%, at least 75%, at least 80%, at least 85%, at least 90%, at least 95%, at least 97%, at least 99%, or 100% identity to SEQ ID NO: 35.

In some embodiments, the, or each, PSMA-binding moiety independently comprises a sequence as set forth in SEQ ID NO: 35.

In some embodiments, the, or each, PSMA-binding moiety independently consists of a sequence as set forth in SEQ ID NO: 35.

In some embodiments, the, or each, PSMA-binding moiety independently comprises or consists of a sequence having at least 70%, at least 75%, at least 80%, at least 85%, at least 90%, at least 95%, at least 97%, at least 99%, or 100% identity to SEQ ID NO: 42.

In some embodiments, the, or each, PSMA-binding moiety independently comprises a sequence as set forth in SEQ ID NO: 42.

In some embodiments, the, or each, PSMA-binding moiety independently consists of a sequence as set forth in SEQ ID NO: 42.

It will be understood that a PSMA-binding moiety having % identity to the SEQ ID NO of any of the PSMA-binding moieties defined herein may have an equivalent function to the PSMA-binding moiety having the sequence set forth in the SEQ ID NO defined herein and may suitably maintain the capacity to bind to PSMA, e.g. the same capacity as the PSMA-binding moiety having the sequence set forth in the SEQ ID NO defined herein.

In some embodiments, the, or each, PSMA-binding moiety independently comprises a PSMA-binding fragment thereof.

In some embodiments, the, or each, PSMA-binding moiety independently comprises a PSMA-binding VHH domain.

In some embodiments, the, or each, PSMA-binding moiety independently consists of a PSMA-binding VHH domain.

In some embodiments, the, or each, PSMA-binding moiety independently is a PSMA-binding VHH domain.

In some embodiments, the PSMA-binding VHH domain of the first PSMA-binding moiety and the PSMA-binding VHH domain of the second PSMA-binding moiety are the same.

In some embodiments, the, or each, PSMA-binding VHH domain independently comprises a PSMA-binding fragment thereof.

As used herein, the terms "single domain variable heavy immunoglobulin domain" and "VHH domain" refer to a fragment of an antibody, which comprises a single monomeric variable antibody domain. The terms "single domain variable heavy immunoglobulin domain" and "VHH domain" may be considered as a type of nanobody.

In some embodiments, the, or each, PSMA-binding moiety is independently selected from the list consisting of: a non-human binding moiety, a chimeric binding moiety, a humanised binding moiety, and a fully human binding moiety.

In some embodiments, the, or each, PSMA-binding VHH domain is independently selected from the list consisting of: a non-human VHH domain, a chimeric VHH domain, a humanised VHH domain, and a fully human VHH domain.

In some embodiments, the, or each, PSMA-binding moiety is independently humanised. In some embodiments, the, or each, PSMA-binding VHH domain is independently humanised.

In some embodiments, the, or each, PSMA-binding moiety is independently chimeric. In some embodiments, the, or each, PSMA-binding VHH domain is independently chimeric.

Techniques for obtaining antibodies and fragments thereof are well-known in the art. By way of example, it is well-known that antibodies may be obtained by standard techniques comprising immunizing an animal with a target antigen and isolating antibodies from serum.

It will be understood that a fragment of an antibody comprises an amino acid sequence that is shorter than the full-length sequence of an antibody, but retains the biological activity (e.g., binding specificity and/or affinity) of the full-length antibody.

The term "humanised antibody" typically refers to a genetically engineered antibody generated in a non-human animal that has been engineered to reduce immunogenicity when used in a human whilst retaining antigen specificity. The humanised antibody typically contains human antibody constant domains and non-human variable domains that are modified to contain a high level of sequence homology to human variable domains.

Suitable assays and techniques for measuring/quantifying binding activity of PSMA-binding moiety according to the invention may include, but are not limited to, ELISA, surface plasmon resonance (SPR), bio-layer interferometry (BLI), quartz crystal microbalance (QCM), bioluminescence assays and flow cytometry. Other suitable techniques will be known in the art.

In particular, it will be understood that the ability of a binding molecule to bind to its cognate target may be assessed by flow cytometry, where the effectiveness of a binding molecule to bind to a cognate target expressed by a population of cells can be measured in a flow cytometry assay.

Moreover, it will be understood that EC₅₀ is a measure of the concentration of a binding molecule that induces a specific response that is 50% between the maximum response and the baseline response. As such, EC₅₀ can be used to assess the ability of a binding molecule to bind to a target.

### CD3-binding moiety

As used herein, a "CD3-binding moiety" comprises a binding domain, e.g. an antigen-binding domain, that is capable of binding to CD3. In some embodiments, the CD3-binding moiety comprises a CD3-binding Fab domain.

It will be understood that the term "CD3-binding moiety" also encompasses fragments thereof.

In some embodiments, CD3 is human CD3. In some embodiments, CD3 is CD3ε.

In some embodiments, the CD3-binding moiety comprises one, two, three, four, five or six CDRs.

In some embodiments, the CD3-binding moiety comprises six CDRs.

In some embodiments, the CD3-binding moiety comprises a heavy chain variable (CD3-VH) domain.

In some embodiments, the CD3-binding moiety comprises a light chain variable (CD3-VL) domain.

In some embodiments, the CD3-binding moiety comprises a CD3-VH domain and CD3-VL domain.

It will be understood that the CD3-VH domain and/or CD3-VL domain are capable of binding to CD3. It will also be understood that the CD3-VH domain and CD3-VL domain may form the antigen binding domain of the CD3-binding moiety as described herein.

In some embodiments, the CD3-VH domain comprises one or more complementarity determining regions (CDRs). In some embodiments, the CD3-VH domain comprises one, two or three CDRs. In some embodiments, the CD3-VH domain comprises three CDRs. It will be understood that CDRs of the CD3-VH domain may be termed HCDRs. It will also be understood that each of the three CDRs of the CD3-VH domain may be termed HCDR1, HCDR2 and HCDR3 respectively.

In some embodiments, the CD3-VL domain comprises one or more CDRs. In some embodiments, the CD3-VL domain comprises one, two or three CDRs. In some embodiments, the CD3-VL domain comprises three CDRs. It will be understood that CDRs of the CD3-VL domain may be termed LCDRs. It will also be understood that each of the three CDRs of the CD3-VL domain may be termed LCDR1, LCDR2 and LCDR3 respectively.

In some embodiments, the CD3-binding moiety further comprises framework regions. In some embodiments, the framework regions are a scaffold (e.g. amino acid sequences) that support the CDRs such that the CD3-binding moiety specifically binds to CD3 via the CDRs.

The CDRs may be determined according to the Kabat definition and/or the IMGT definition, both of which are well-known in the art.

It will be understood that a variant of a CD3-binding moiety as described herein may have an equivalent function to a full length CD3-binding moiety amino acid sequence as described herein and may suitably maintain the capacity to bind to CD3.

It will be understood that a variant of a CD3-VH domain as described herein may have an equivalent function to a full length CD3-VH domain amino acid sequence as described herein and may suitably maintain the capacity to bind to CD3.

It will be understood that a variant of a CD3-VL domain as described herein may have an equivalent function to a full length CD3-VL domain amino acid sequence as described herein and may suitably maintain the capacity to bind to CD3.

In some embodiments, the CD3-binding moiety specifically binds to CD3.

In some embodiments, it will be understood herein that "specifically binds to" refers to the antibody-like binding of the CD3-binding moiety to its target: CD3. In some embodiments, "specifically binds to" means that the CD3-binding moiety binds to an epitope on CD3.

In some embodiments, the CD3-binding moiety is capable of recruiting immune cells.

All CDRs specified herein, i.e. each CDR of the CD3-binding variable domains of the antigen binding proteins described herein, i.e. HCDR1, HCDR2, HCDR3, LCDR1, LCDR2 and LCDR3 defined below, can be variant CDRs. The amino acid sequence of a variant CDR can be varied by one or more, preferably one, two or three amino acid deletions, insertions and/or substitutions from the sequence of the corresponding non-variant CDR. In some embodiments, the variant CDR comprises not more than 3, preferably not more than two, more preferably not more than one conservative substitution. In some embodiments, the variant CDR has an amino acid sequence that is highly homologous to the sequence of the corresponding non-variant CDR. The variant CDRs, when in the context of the antigen binding proteins of the invention have at least 80%, 85%, 90%, 95%, 97%, 98%, 99% or 100% of the binding affinity to CD3 compared to the non-variant CDR.

In some embodiments, the CD3-binding moiety comprises a CD3-VH domain; wherein the CD3-VH domain comprises a HCDR3 sequence, wherein the HCDR3 sequence comprises or consists of SEQ ID NO: 3 (Kabat).

In some embodiments, the CD3-binding moiety comprises a CD3-VH domain; wherein the CD3-VH domain comprises a HCDR3 sequence, wherein the HCDR3 sequence comprises or consists of SEQ ID NO: 9 (IMGT).

In some embodiments, the CD3-binding moiety comprises a CD3-VH domain; wherein the CD3-VH domain comprises a HCDR1 sequence, wherein the HCDR1 sequence comprises or consists of SEQ ID NO: 1 (Kabat).

In some embodiments, the CD3-binding moiety comprises a CD3-VH domain; wherein the CD3-VH domain comprises a HCDR1 sequence, wherein the HCDR1 sequence comprises or consists of SEQ ID NO: 7 (IMGT).

In some embodiments, the CD3-binding moiety comprises a CD3-VH domain; wherein the CD3-VH domain comprises a HCDR2 sequence, wherein the HCDR2 sequence comprises or consists of SEQ ID NO: 2 (Kabat).

In some embodiments, the CD3-binding moiety comprises a CD3-VH domain; wherein the CD3-VH domain comprises a HCDR2 sequence, wherein the HCDR2 sequence comprises or consists of SEQ ID NO: 8 (IMGT).

In some embodiments, the CD3-binding moiety comprises a CD3-VL domain; wherein the CD3-VL domain comprises a LCDR3 sequence, wherein the LCDR3 sequence comprises or consists of SEQ ID NO: 6 (Kabat).

In some embodiments, the CD3-binding moiety comprises a CD3-VL domain; wherein the CD3-VL domain comprises a LCDR3 sequence, wherein the LCDR3 sequence comprises or consists of SEQ ID NO: 12 (IMGT).

In some embodiments, the CD3-binding moiety comprises a CD3-VL domain; wherein the CD3-VL domain comprises a LCDR1 sequence, wherein the LCDR1 sequence comprises or consists of SEQ ID NO: 4 (Kabat).

In some embodiments, the CD3-binding moiety comprises a CD3-VL domain; wherein the CD3-VL domain comprises a LCDR1 sequence, wherein the LCDR1 sequence comprises or consists of SEQ ID NO: 10 (IMGT).

In some embodiments, the CD3-binding moiety comprises a CD3-VL domain; wherein the CD3-VL domain comprises a LCDR2 sequence, wherein the LCDR2 sequence comprises or consists of SEQ ID NO: 5 (Kabat).

In some embodiments, the CD3-binding moiety comprises a CD3-VL domain; wherein the CD3-VL domain comprises a LCDR2 sequence, wherein the LCDR2 sequence comprises or consists of SEQ ID NO: 11 (IMGT).

In some embodiments, one or more of the CDRs comprises one, two or three amino acid mutations relative to the recited sequences.

In some embodiments, the HCDR1 sequence comprises one, two or three amino acid mutations relative to the recited sequences. In some embodiments, the HCDR2 sequence comprises one, two or three amino acid mutations relative to the recited sequences. In some embodiments, the HCDR3 sequence comprises one, two or three amino acid mutations relative to the recited sequences.

In some embodiments, the LCDR1 sequence comprises one, two or three amino acid mutations relative to the recited sequences. In some embodiments, the LCDR2 sequence comprises one, two or three amino acid mutations relative to the recited sequences. In some embodiments, the LCDR3 sequence comprises one, two or three amino acid mutations relative to the recited sequences.

In some embodiments, the CD3-binding moiety comprises a CD3-VH domain; wherein the CD3-VH domain comprises HCDRs 1-3, wherein (according to Kabat numbering):
HCDR1 comprises the amino acid sequence according to SEQ ID NO: 1,
HCDR2 comprises the amino acid sequence according to SEQ ID NO: 2, and
HCDR3 comprises the amino acid sequence according to SEQ ID NO: 3;
optionally wherein one or more of the CDRs comprises one, two or three amino acid mutations relative to the recited sequences.

In some embodiments, the CD3-binding moiety comprises a CD3-VL domain; wherein the CD3-VL domain comprises LCDRs 1-3, wherein (according to Kabat numbering):
LCDR1 comprises the amino acid sequence according to SEQ ID NO: 4,
LCDR2 comprises the amino acid sequence according to SEQ ID NO: 5, and
LCDR3 comprises the amino acid sequence according to SEQ ID NO: 6;
optionally wherein one or more of the CDRs comprises one, two or three amino acid mutations relative to the recited sequences.

In some embodiments, the CD3-binding moiety comprises a CD3-VH domain and a CD3-VL domain; wherein the CD3-VH domain comprises HCDRs 1-3, and the CD3-VL domain comprises LCDRs 1-3, wherein (according to Kabat numbering):
HCDR1 comprises the amino acid sequence according to SEQ ID NO: 1,
HCDR2 comprises the amino acid sequence according to SEQ ID NO: 2, and
HCDR3 comprises the amino acid sequence according to SEQ ID NO: 3,
LCDR1 comprises the amino acid sequence according to SEQ ID NO: 4,
LCDR2 comprises the amino acid sequence according to SEQ ID NO: 5, and
LCDR3 comprises the amino acid sequence according to SEQ ID NO: 6;
optionally wherein one or more of the CDRs comprises one, two or three amino acid mutations relative to the recited sequences.

In some embodiments, the CD3-binding moiety comprises a CD3-VH domain; wherein the CD3-VH domain comprises HCDRs 1-3, wherein (according to IMGT numbering):
HCDR1 comprises the amino acid sequence according to SEQ ID NO: 7,
HCDR2 comprises the amino acid sequence according to SEQ ID NO: 8,
HCDR3 comprises the amino acid sequence according to SEQ ID NO: 9;
optionally wherein one or more of the CDRs comprises one, two or three amino acid mutations relative to the recited sequences.

In some embodiments, the CD3-binding moiety comprises a CD3-VL domain; wherein the CD3-VL domain comprises LCDRs 1-3, wherein (according to IMGT numbering):
LCDR1 comprises the amino acid sequence according to SEQ ID NO: 10,
LCDR2 comprises the amino acid sequence according to SEQ ID NO: 11, and
LCDR3 comprises the amino acid sequence according to SEQ ID NO: 12;
optionally wherein one or more of the CDRs comprises one, two or three amino acid mutations relative to the recited sequences.

In some embodiments, the CD3-binding moiety comprises a CD3-VH domain and a CD3-VL domain; wherein the CD3-VH domain comprises HCDRs 1-3, and the CD3-VL domain comprises LCDRs 1-3, wherein (according to IMGT numbering):
HCDR1 comprises the amino acid sequence according to SEQ ID NO: 7,
HCDR2 comprises the amino acid sequence according to SEQ ID NO: 8,
HCDR3 comprises the amino acid sequence according to SEQ ID NO: 9,
LCDR1 comprises the amino acid sequence according to SEQ ID NO: 10,
LCDR2 comprises the amino acid sequence according to SEQ ID NO: 11, and
LCDR3 comprises the amino acid sequence according to SEQ ID NO: 12;
optionally wherein one or more of the CDRs comprises one, two or three amino acid mutations relative to the recited sequences.

In some embodiments, the HCDR1, HCDR2 and HCDR3 sequences comprise SEQ ID NO: 1, SEQ ID NO: 2, and SEQ ID NO: 3, respectively.

In some embodiments, the HCDR1, HCDR2 and HCDR3 sequences consist of SEQ ID NO: 1, SEQ ID NO: 2, and SEQ ID NO: 3, respectively.

In some embodiments, the HCDR1, HCDR2 and HCDR3 sequences comprise SEQ ID NO: 7, SEQ ID NO: 8, and SEQ ID NO: 9, respectively.

In some embodiments, the HCDR1, HCDR2 and HCDR3 sequences consist of SEQ ID NO: 7, SEQ ID NO: 8, and SEQ ID NO: 9, respectively.

In some embodiments, the LCDR1, LCDR2 and LCDR3 sequences comprise SEQ ID NO: 4, SEQ ID NO: 5, and SEQ ID NO: 6, respectively.

In some embodiments, the LCDR1, LCDR2 and LCDR3 sequences consist of SEQ ID NO: 4, SEQ ID NO: 5, and SEQ ID NO: 6, respectively.

In some embodiments, the LCDR1, LCDR2 and LCDR3 sequences comprise SEQ ID NO: 10, SEQ ID NO: 11, and SEQ ID NO: 12, respectively.

In some embodiments, the LCDR1, LCDR2 and LCDR3 sequences consist of SEQ ID NO: 10, SEQ ID NO: 11, and SEQ ID NO: 12 respectively.

In the VH domain and VL domain the CDR sequences are interspersed with FR sequences. Like the CDR sequences, the FR sequences differ depending on whether they are defined according to Kabat or IMGT. Preferably, CDR sequences defined according to Kabat are combined with FR sequences defined according to Kabat and CDR sequences defined according to IMGT are combined with FR sequences according to IMGT.

In some embodiments, the CD3-VH domain comprises a sequence having at least 70%, at least 75%, at least 80%, at least 85%, at least 90%, at least 95%, at least 97%, at least 99%, or 100% identity to any one of SEQ ID NO: 13, SEQ ID NO: 14, or SEQ ID NO: 15.

In some embodiments, the CD3-VH domain comprises a sequence having at least 70%, at least 75%, at least 80%, at least 85%, at least 90%, at least 95%, at least 97%, at least 99%, or 100% identity to SEQ ID NO: 13.

In some embodiments, the CD3-VH domain comprises a sequence having at least 70%, at least 75%, at least 80%, at least 85%, at least 90%, at least 95%, at least 97%, at least 99%, or 100% identity to SEQ ID NO: 14.

In some embodiments, the CD3-VH domain comprises a sequence having at least 70%, at least 75%, at least 80%, at least 85%, at least 90%, at least 95%, at least 97%, at least 99%, or 100% identity to SEQ ID NO: 15.

In some embodiments, the CD3-VH domain comprises of a sequence as set forth in any one of SEQ ID NO: 13, SEQ ID NO: 14, or SEQ ID NO: 15.

In some embodiments, the CD3-VH domain comprises of a sequence as set forth in SEQ ID NO: 13. In some embodiments, the CD3-VH domain comprises of a sequence as set forth in SEQ ID NO: 14. In some embodiments, the CD3-VH domain comprises of a sequence as set forth in SEQ ID NO: 15.

In some embodiments, the CD3-VH domain consists of a sequence as set forth in any one of SEQ ID NO: 13, SEQ ID NO: 14, or SEQ ID NO: 15.

In some embodiments, the CD3-VH domain consists of a sequence as set forth in SEQ ID NO: 13. In some embodiments, the CD3-VH domain consists of a sequence as set forth in SEQ ID NO: 14. In some embodiments, the CD3-VH domain consists of a sequence as set forth in SEQ ID NO: 15.

It will be understood that the variants of the CD3-VH domains described herein comprise the HCDR1, HCDR2 and HCDR3 sequences described herein (i.e. SEQ ID NO: 1, SEQ ID NO: 2, and SEQ ID NO: 3, respectively defined according to Kabat; or SEQ ID NO: 7, SEQ ID NO: 8, and SEQ ID NO: 9, respectively defined according to IMGT), optionally wherein one or more of the CDRs comprises one, two or three amino acid mutations relative to the recited sequences.

In some embodiments, the CD3-VL domain comprises a sequence having at least 70%, at least 75%, at least 80%, at least 85%, at least 90%, at least 95%, at least 97%, at least 99%, or 100% identity to any one of SEQ ID NO: 17, SEQ ID NO: 18, or SEQ ID NO: 19.

In some embodiments, the CD3-VL domain comprises a sequence having at least 70%, at least 75%, at least 80%, at least 85%, at least 90%, at least 95%, at least 97%, at least 99%, or 100% identity to SEQ ID NO: 17.

In some embodiments, the CD3-VL domain comprises a sequence having at least 70%, at least 75%, at least 80%, at least 85%, at least 90%, at least 95%, at least 97%, at least 99%, or 100% identity to SEQ ID NO: 18.

In some embodiments, the CD3-VL domain comprises a sequence having at least 70%, at least 75%, at least 80%, at least 85%, at least 90%, at least 95%, at least 97%, at least 99%, or 100% identity to SEQ ID NO: 19.

In some embodiments, the CD3-VL domain comprises a sequence as set forth in any one of SEQ ID NO: 17, SEQ ID NO: 18, or SEQ ID NO: 19.

In some embodiments, the CD3-VL domain comprises a sequence as set forth in SEQ ID NO: 17. In some embodiments, the CD3-VL domain comprises a sequence as set forth in SEQ ID NO: 18. In some embodiments, the CD3-VL domain comprises a sequence as set forth in SEQ ID NO: 19.

In some embodiments, the CD3-VL domain consists of a sequence as set forth in any one of SEQ ID NO: 17, SEQ ID NO: 18, or SEQ ID NO: 19.

In some embodiments, the CD3-VL domain consists of a sequence as set forth in SEQ ID NO: 17. In some embodiments, the CD3-VL domain consists of a sequence as set forth in SEQ ID NO: 18. In some embodiments, the CD3-VL domain consists of a sequence as set forth in SEQ ID NO: 19.

It will be understood that the variants of the CD3-VL domains described herein comprise the LCDR1, LCDR2 and LCDR3 sequences described herein (i.e. SEQ ID NO: 4, SEQ ID NO: 5, and SEQ ID NO: 6, respectively defined according to Kabat; or SEQ ID NO: 10, SEQ ID NO: 11, and SEQ ID NO: 12, respectively defined according to IMGT), optionally wherein one or more of the CDRs comprises one, two or three amino acid mutations relative to the recited sequences.

In some embodiments, the CD3-binding moiety comprises:
(i) a VH domain comprising a sequence having at least 70%, at least 75%, at least 80%, at least 85%, at least 90%, at least 95%, at least 97%, at least 99%, or 100% identity to SEQ ID NO: 13; and
(ii) a VL domain comprising a sequence having at least 70%, at least 75%, at least 80%, at least 85%, at least 90%, at least 95%, at least 97%, at least 99%, or 100% identity to SEQ ID NO: 17.

In some embodiments, the CD3-binding moiety comprises:
(i) a VH domain consisting of a sequence having at least 70%, at least 75%, at least 80%, at least 85%, at least 90%, at least 95%, at least 97%, at least 99%, or 100% identity to SEQ ID NO: 13; and
(ii) a VL domain consisting of a sequence having at least 70%, at least 75%, at least 80%, at least 85%, at least 90%, at least 95%, at least 97%, at least 99%, or 100% identity to SEQ ID NO: 17.

In some embodiments, the CD3-binding moiety comprises:
(i) a VH domain comprising a sequence as set forth in SEQ ID NO: 13; and
(ii) a VL domain comprising a sequence as set forth in SEQ ID NO: 17.

In some embodiments, the CD3-binding moiety comprises:
(i) a VH domain consisting of a sequence as set forth in SEQ ID NO: 13; and
(ii) a VL domain consisting of a sequence as set forth in SEQ ID NO: 17.

In some embodiments, the CD3-binding moiety comprises:
(i) a VH domain comprising a sequence having at least 70%, at least 75%, at least 80%, at least 85%, at least 90%, at least 95%, at least 97%, at least 99%, or 100% identity to SEQ ID NO: 14; and
(ii) a VL domain comprising a sequence having at least 70%, at least 75%, at least 80%, at least 85%, at least 90%, at least 95%, at least 97%, at least 99%, or 100% identity to SEQ ID NO: 18.

In some embodiments, the CD3-binding moiety comprises:
(i) a VH domain consisting of a sequence having at least 70%, at least 75%, at least 80%, at least 85%, at least 90%, at least 95%, at least 97%, at least 99%, or 100% identity to SEQ ID NO: 14; and
(ii) a VL domain consisting of a sequence having at least 70%, at least 75%, at least 80%, at least 85%, at least 90%, at least 95%, at least 97%, at least 99%, or 100% identity to SEQ ID NO: 18.

In some embodiments, the CD3-binding moiety comprises:
(i) a VH domain comprising a sequence as set forth in SEQ ID NO: 14; and
(ii) a VL domain comprising a sequence as set forth in SEQ ID NO: 18.

In some embodiments, the CD3-binding moiety comprises:
(i) a VH domain consisting of a sequence as set forth in SEQ ID NO: 14; and
(ii) a VL domain consisting of a sequence as set forth in SEQ ID NO: 18.

In some embodiments, the CD3-binding moiety comprises:
(i) a VH domain comprising a sequence having at least 70%, at least 75%, at least 80%, at least 85%, at least 90%, at least 95%, at least 97%, at least 99%, or 100% identity to SEQ ID NO: 15; and
(ii) a VL domain comprising a sequence having at least 70%, at least 75%, at least 80%, at least 85%, at least 90%, at least 95%, at least 97%, at least 99%, or 100% identity to SEQ ID NO: 19.

In some embodiments, the CD3-binding moiety comprises:
(i) a VH domain consisting of a sequence having at least 70%, at least 75%, at least 80%, at least 85%, at least 90%, at least 95%, at least 97%, at least 99%, or 100% identity to SEQ ID NO: 15; and
(ii) a VL domain consisting of a sequence having at least 70%, at least 75%, at least 80%, at least 85%, at least 90%, at least 95%, at least 97%, at least 99%, or 100% identity to SEQ ID NO: 19.

In some embodiments, the CD3-binding moiety comprises:
(i) a VH domain comprising a sequence as set forth in SEQ ID NO: 15; and
(ii) a VL domain comprising a sequence as set forth in SEQ ID NO: 19.

In some embodiments, the CD3-binding moiety comprises:
(i) a VH domain consisting of a sequence as set forth in SEQ ID NO: 15; and
(ii) a VL domain consisting of a sequence as set forth in SEQ ID NO: 19.

In some embodiments, the CD3-binding moiety comprises a fragment binding region (Fab). In some embodiments, the CD3-binding moiety consists of a Fab. In some embodiments, the CD3-binding moiety is a Fab.

It will be understood that a Fab typically comprises: (i) a heavy chain comprising one heavy constant (CH) domain and one heavy variable (VH) domain; and (ii) a light chain comprising one light constant domain (CL) and one light variable (VL) domain.

It will be understood that the Fab may comprise any of the CD3 binding CDRs, VH domains and/or VL domains as described herein.

It will be understood that the term "Fab" also encompasses fragments thereof.

In some embodiments, the heavy chain constant domain is selected from the list consisting of: a constant heavy 1 (CH1) domain, a constant heavy 2 (CH2) domain, and a constant heavy 3 (CH3) domain.

In some embodiments, the light chain constant domain is selected from the list consisting of: a light chain kappa constant domain and a light chain lambda constant domain.

In some embodiments, the CD3-binding moiety comprises a Fab comprising:
(i) a heavy chain comprising a CD3-VH domain as described herein and a CD3 heavy chain constant (CD3-CH1) domain; and
(ii) a light chain comprising a CD3-VL domain as described herein and a CD3 light chain constant (CD3-CL) domain.

In some embodiments, the heavy chain comprises a sequence having at least 70%, at least 75%, at least 80%, at least 85%, at least 90%, at least 95%, at least 97%, at least 99%, or 100% identity to any one of SEQ ID NO: 21, SEQ ID NO: 22, or SEQ ID NO: 23.

In some embodiments, the heavy chain comprises a sequence having at least 70%, at least 75%, at least 80%, at least 85%, at least 90%, at least 95%, at least 97%, at least 99%, or 100% identity to SEQ ID NO: 21.

In some embodiments, the heavy chain comprises a sequence having at least 70%, at least 75%, at least 80%, at least 85%, at least 90%, at least 95%, at least 97%, at least 99%, or 100% identity to SEQ ID NO: 22.

In some embodiments, the heavy chain comprises a sequence having at least 70%, at least 75%, at least 80%, at least 85%, at least 90%, at least 95%, at least 97%, at least 99%, or 100% identity to SEQ ID NO: 23.

In some embodiments, the heavy chain comprises a sequence as set forth in any one of SEQ ID NO: 21, SEQ ID NO: 22, or SEQ ID NO: 23.

In some embodiments, the heavy chain comprises a sequence as set forth in SEQ ID NO: 21. In some embodiments, the heavy chain comprises a sequence as set forth in SEQ ID NO: 22. In some embodiments, the heavy chain comprises a sequence as set forth in SEQ ID NO: 23.

In some embodiments, the heavy chain consists of a sequence as set forth in any one of SEQ ID NO: 21, SEQ ID NO: 22, or SEQ ID NO: 23.

In some embodiments, the heavy chain consists of a sequence as set forth in SEQ ID NO: 21. In some embodiments, the heavy chain consists of a sequence as set forth in SEQ ID NO: 22. In some embodiments, the heavy chain consists of a sequence as set forth in SEQ ID NO: 23.

In some embodiments, the light chain comprises a sequence having at least 70%, at least 75%, at least 80%, at least 85%, at least 90%, at least 95%, at least 97%, at least 99%, or 100% identity to any one of SEQ ID NO: 25, SEQ ID NO: 26, or SEQ ID NO: 27.

In some embodiments, the light chain comprises a sequence having at least 70%, at least 75%, at least 80%, at least 85%, at least 90%, at least 95%, at least 97%, at least 99%, or 100% identity to SEQ ID NO: 25.

In some embodiments, the light chain comprises a sequence having at least 70%, at least 75%, at least 80%, at least 85%, at least 90%, at least 95%, at least 97%, at least 99%, or 100% identity to SEQ ID NO: 26.

In some embodiments, the light chain comprises a sequence having at least 70%, at least 75%, at least 80%, at least 85%, at least 90%, at least 95%, at least 97%, at least 99%, or 100% identity to SEQ ID NO: 27.

In some embodiments, the light chain comprises a sequence as set forth in any one of SEQ ID NO: 25, SEQ ID NO: 26, or SEQ ID NO: 27.

In some embodiments, the light chain comprises a sequence as set forth in SEQ ID NO: 25. In some embodiments, the light chain comprises a sequence as set forth in SEQ ID NO: 26. In some embodiments, the light chain comprises a sequence as set forth in SEQ ID NO: 27.

In some embodiments, the light chain consists of a sequence as set forth in any one of SEQ ID NO: 25, SEQ ID NO: 26, or SEQ ID NO: 27.

In some embodiments, the light chain consists of a sequence as set forth in SEQ ID NO: 25. In some embodiments, the light chain consists of a sequence as set forth in SEQ ID NO: 26. In some embodiments, the light chain consists of a sequence as set forth in SEQ ID NO: 27.

In some embodiments, the CD3-binding moiety comprises:
(i) a heavy chain comprising a sequence having at least 70%, at least 75%, at least 80%, at least 85%, at least 90%, at least 95%, at least 97%, at least 99%, or 100% identity to SEQ ID NO: 21; and
(ii) a light chain comprising a sequence having at least 70%, at least 75%, at least 80%, at least 85%, at least 90%, at least 95%, at least 97%, at least 99%, or 100% identity to SEQ ID NO: 25.

In some embodiments, the CD3-binding moiety comprises:
(i) a heavy chain consisting of a sequence having at least 70%, at least 75%, at least 80%, at least 85%, at least 90%, at least 95%, at least 97%, at least 99%, or 100% identity to SEQ ID NO: 21; and
(ii) a light chain consisting of a sequence having at least 70%, at least 75%, at least 80%, at least 85%, at least 90%, at least 95%, at least 97%, at least 99%, or 100% identity to SEQ ID NO: 25.

In some embodiments, the CD3-binding moiety comprises:
(i) a heavy chain comprising a sequence as set forth in SEQ ID NO: 21; and
(ii) a light chain comprising a sequence as set forth in SEQ ID NO: 25.

In some embodiments, the CD3-binding moiety comprises:
(i) a heavy chain consisting of a sequence as set forth in SEQ ID NO: 21; and
(ii) a light chain consisting of a sequence as set forth in SEQ ID NO: 25.

In some embodiments, the CD3-binding moiety comprises:
(i) a heavy chain comprising a sequence having at least 70%, at least 75%, at least 80%, at least 85%, at least 90%, at least 95%, at least 97%, at least 99%, or 100% identity to SEQ ID NO: 22; and
(ii) a light chain comprising a sequence having at least 70%, at least 75%, at least 80%, at least 85%, at least 90%, at least 95%, at least 97%, at least 99%, or 100% identity to SEQ ID NO: 26.

In some embodiments, the CD3-binding moiety comprises:
(i) a heavy chain consisting of a sequence having at least 70%, at least 75%, at least 80%, at least 85%, at least 90%, at least 95%, at least 97%, at least 99%, or 100% identity to SEQ ID NO: 22; and
(ii) a light chain consisting of a sequence having at least 70%, at least 75%, at least 80%, at least 85%, at least 90%, at least 95%, at least 97%, at least 99%, or 100% identity to SEQ ID NO: 26.

In some embodiments, the CD3-binding moiety comprises:
(i) a heavy chain comprising a sequence as set forth in SEQ ID NO: 22; and
(ii) a light chain comprising a sequence as set forth in SEQ ID NO: 26.

In some embodiments, the CD3-binding moiety comprises:
(i) a heavy chain consisting of a sequence as set forth in SEQ ID NO: 22; and
(ii) a light chain consisting of a sequence as set forth in SEQ ID NO: 26.

In some embodiments, the CD3-binding moiety comprises:
(i) a heavy chain comprising a sequence having at least 70%, at least 75%, at least 80%, at least 85%, at least 90%, at least 95%, at least 97%, at least 99%, or 100% identity to SEQ ID NO: 23; and
(ii) a light chain comprising a sequence having at least 70%, at least 75%, at least 80%, at least 85%, at least 90%, at least 95%, at least 97%, at least 99%, or 100% identity to SEQ ID NO: 27.

In some embodiments, the CD3-binding moiety comprises:
(i) a heavy chain consisting of a sequence having at least 70%, at least 75%, at least 80%, at least 85%, at least 90%, at least 95%, at least 97%, at least 99%, or 100% identity to SEQ ID NO: 23; and
(ii) a light chain consisting of a sequence having at least 70%, at least 75%, at least 80%, at least 85%, at least 90%, at least 95%, at least 97%, at least 99%, or 100% identity to SEQ ID NO: 27.

In some embodiments, the CD3-binding moiety comprises:
(i) a heavy chain comprising a sequence as set forth in SEQ ID NO: 23; and
(ii) a light chain comprising a sequence as set forth in SEQ ID NO: 27.

In some embodiments, the CD3-binding moiety comprises:
(i) a heavy chain consisting of a sequence as set forth in SEQ ID NO: 23; and
(ii) a light chain consisting of a sequence as set forth in SEQ ID NO: 27.

The CD3-binding moiety according to the invention shows a high affinity for CD3. In particular, the CD3-binding moiety of the invention has the ability to bind to a recombinant human CD3ε/δ heterodimer with an EC50 of less than 75 ng/ml, less than 50 ng/ml, less than 40 ng/ml, less than 30 ng/ml, less than 25 ng/ml, less than 20 ng/ml, less than 15 ng/ml, less than 10 ng/ml, less than 5 ng/ml, or less than 2 ng/ml.

In some embodiments, the EC50 is 4-75 ng/ml, 4-50 ng/ml, 4-40 ng/ml, 4-30 ng/ml, 4-25 ng/ml, 4-20 ng/ml, 4-15 ng/ml, 4-10 ng/ml or 4-5 ng/ml.

Furthermore, the CD3-binding moiety of the invention has the ability to bind to a recombinant human CD3ε/γ heterodimer with an EC50 of less than 20 ng/ml, less than 18 ng/ml, less than 16 ng/ml, less than 14 ng/ml, less than 12 ng/ml, less than 10 ng/ml, less than 8 ng/ml, less than 7 ng/ml, less than 6 ng/ml, or less than 2 ng/ml, such as 4-20 ng/ml, 4-18 ng/ml, 4-16 ng/ml, 4-14 ng/ml, 4-12 ng/ml, 4-10 ng/ml, 4-8 ng/ml, 4-7 ng/ml or 4-6 ng/ml.

The CD3-binding moiety of the invention also shows a high affinity for cells expressing CD3. In particular, the CD3-binding moiety of the invention binds to cells genetically modified to express human CD3 protein on the cell surface with an EC50 of less than 12 ng/ml, less than 11 ng/ml, less than 10 ng/ml, less than 9 ng/ml, less than 8 ng/ml, less than 7 ng/ml, less than 6 ng/ml, less than 5 ng/ml, less than 4 ng/ml, less than 3 ng/ml, or less than 2 ng/ml such as 1-12 ng/ml, 1-11 ng/ml, 1-10 ng/ml, 1-9 ng/ml, 1-8 ng/ml, 3-12 ng/ml, 3-11 ng/ml, 3-10 ng/ml, 3-9 ng/ml, 3-8 ng/ml, 6-12 ng/ml, 6-11 ng/ml, 6-10 ng/ml, 6-9 ng/ml or 6-8 ng/ml.

In some embodiments, the CD3-binding moiety binds to CD3ε. In some embodiments, the CD3ε is human CD3ε. Surprisingly, the inventors have shown that the CD3-binding moiety of the invention is capable of binding to cells expressing only CD3 and not the TCR. In other words, the antigen binding proteins of the invention are capable of binding to CD3 independently of the TCR.

In some embodiments, the CD3-binding moiety comprises a fragment thereof.

In some embodiments, the CD3-binding moiety comprises a fragment of a Fab.

In some embodiments, the CD3-binding moiety or a fragment thereof is non-human.

In some embodiments, the CD3-binding moiety or a fragment thereof is humanised.

In some embodiments, the CD3-binding moiety or a fragment thereof is chimeric.

In some embodiments, the CD3-binding moiety or a fragment thereof is fully human.

Techniques for obtaining antibodies and fragments thereof are well-known in the art. By way of example, it is well-known that antibodies may be obtained by standard techniques comprising immunizing an animal with a target antigen and isolating antibodies from serum.

It will be understood that a fragment of an antibody comprises an amino acid sequence that is shorter than the full-length sequence of an antibody, but retains the biological activity (e.g., binding specificity and/or affinity) of the full-length antibody.

The term "humanised antibody" typically refers to a genetically engineered antibody generated in a non-human animal that has been engineered to reduce immunogenicity when used in a human whilst retaining antigen specificity. The humanised antibody typically contains human antibody constant domains and non-human variable domains that are modified to contain a high level of sequence homology to human variable domains.

Suitable assays and techniques for measuring/quantifying binding activity of CD3-binding moiety according to the invention may include, but are not limited to, ELISA, surface plasmon resonance (SPR), bio-layer interferometry (BLI), quartz crystal microbalance (QCM), bioluminescence assays and flow cytometry. Other suitable techniques will be known in the art.

In particular, it will be understood that the ability of a binding molecule to bind to its cognate target may be assessed by flow cytometry, where the effectiveness of a binding molecule to bind to a cognate target expressed by a population of cells can be measured in a flow cytometry assay.

Moreover, it will be understood that EC₅₀ is a measure of the concentration of a binding molecule that induces a specific response that is 50% between the maximum response and the baseline response. As such, EC₅₀ can be used to assess the ability of a binding molecule to bind to a target.

### Fc region

In some embodiments, the bispecific binding molecule further comprises an Fc (fragment crystallisable) region.

In some embodiments, the Fc region comprises a first subunit and a second subunit.

In some embodiments, each subunit of the Fc region independently comprises one or more immunoglobulin constant domain(s).

In some embodiments, each subunit of the Fc region independently comprises a heavy chain constant domain. In some embodiments, the heavy chain constant domain is a heavy chain IgG constant domain. In some embodiments, the heavy chain IgG constant domain is a heavy chain IgG1 constant domain. In some embodiments, the heavy chain IgG constant domain is a heavy chain IgG2 constant domain. In some embodiments, the heavy chain IgG constant domain is a heavy chain IgG3 constant domain. In some embodiments, the heavy chain IgG constant domain is a heavy chain IgG4 constant domain.

In some embodiments, each subunit of the Fc region independently comprises a light chain constant domain. In some embodiments, the light chain constant domain is a light chain kappa constant domain. In some embodiments, the light chain constant domain is a light chain lambda constant domain.

In some embodiments, the, or each, immunoglobulin constant domain is selected from the list consisting of: a constant heavy 1 (CH1) domain, a constant heavy 2 (CH2) domain, and a constant heavy 3 (CH3) domain.

In some embodiments, the first subunit of the Fc region comprises a CH2 domain. In some embodiments, the second subunit of the Fc region comprises a CH2 domain. In some embodiments, the first subunit of the Fc region and the second subunit of the Fc region each independently comprises a CH2 domain.

In some embodiments, the first subunit of the Fc region comprises a CH3 domain. In some embodiments, the second subunit of the Fc region comprises a CH3 domain. In some embodiments, the first subunit of the Fc region and the second subunit of the Fc region each independently comprises a CH3 domain.

In some embodiments, the first subunit of the Fc region comprises a CH2 domain and a CH3 domain. In some embodiments, the second subunit of the Fc region comprises a CH2 domain and a CH3 domain. In some embodiments, the first subunit of the Fc region and the second subunit of the Fc region each independently comprises a CH2 domain and a CH3 domain.

In some embodiments, a hinge region is part of the Fc region. In some embodiments, a hinge region is separate to the Fc region. In some embodiments, a hinge region comprises part of the Fc region.

In some embodiments, a hinge region is part of the CH2 domain. In some embodiments, a hinge region is separate to the CH2 domain. In some embodiments, a hinge region comprises part of the CH2 domain.

In some embodiments, the Fc region is linked to a hinge region. In some embodiments, the Fc region comprises a hinge region.

In some embodiments, the Fc region comprises a full hinge region. In some embodiments, the Fc region comprises a part of a hinge region.

It will be understood that a hinge region can be present between a CH1 domain and a CH2 domain. It will also be understood that part of a CH1 domain and part of a CH2 domain can together form the hinge region.

In some embodiments, the Fc region comprises (i) a hinge region, (ii) a CH2 domain, and (iii) a CH3 domain.

In some embodiments, the Fc region comprises (i) part of a hinge region, (ii) a CH2 domain, and (iii) a CH3 domain.

In some embodiments, the Fc region comprises a CH2 domain and a CH3 domain, wherein a part of the CH2 domain is a part of a hinge region. In some embodiments, the Fc region comprises a CH2 domain and a CH3 domain, wherein an N-terminal part of the CH2 domain is a part of a hinge region.

In some embodiments, the hinge region is derived from IgG1 or IgG3. In some embodiments, the hinge region is derived from IgG1. In some embodiments, the hinge region is derived from IgG3.

In some embodiments, the hinge region is derived from an IgG1 hinge region or an IgG3 hinge region. In some embodiments, the hinge region is derived from an IgG1 hinge region. In some embodiments, the hinge region is derived from an IgG3 hinge region.

In some embodiments, the hinge region is an IgG1 hinge region or an IgG3 hinge region. In some embodiments, the hinge region is an IgG1 hinge region. In some embodiments, the hinge region is an IgG3 hinge region.

In some embodiments, the first subunit of the Fc region and the second subunit of the Fc region each independently comprise a CH2 domain and a CH3 domain, wherein the Fc region is linked to a hinge region.

In some embodiments, the first subunit of the Fc region and the second subunit of the Fc region each independently comprise a CH2 domain and a CH3 domain, wherein the CH2 domain is linked to a hinge region.

In some embodiments, the first subunit of the Fc region and the second subunit of the Fc region each independently comprise a CH2 domain and a CH3 domain, wherein the CH2 domain comprises part of a hinge region.

In some embodiments, the hinge region substantially results in the production of a single covalently bound molecular species when used to produce a binding molecule of the present invention.

In some embodiments, the hinge regions do not form intermolecular disulphide bonds.

In some embodiments, the lack of aggregation is determined by size exclusion chromatography, although any suitable means are known to the skilled person and may be used.

It will be understood that the Fc region may interact with Fc receptors presented on the surface of a cell. The Fc receptors may be Fc gamma receptors.

In some embodiments, the Fc region binds to one or more or all of the Fc receptors. In some embodiments, the Fc receptors comprise one or more or all of FcγRI (CD64), FcγRIIa (CD32A), FcγRIIb (CD32B), FcγRIIIa (CD16a), FcγRIIIb (CD16b), C1q and FcRn.

In some embodiments, the Fc region is capable of interacting with Fc receptors and/or be able to recruit immune cells.

In some embodiments, the Fc region binds to one or more Fc receptors. In some embodiments, the Fc region binds to one or more Fc gamma receptors.

In some embodiments, the Fc region is capable of binding to immune cells. In some embodiments, the Fc domain is capable of recruiting immune cells.

In some embodiments, the immune cells are cytotoxic immune cells. In some embodiments, the immune cells are capable of killing target cells. In some embodiments, the cytotoxic immune cells are selected from the list consisting of: NK cells, macrophages, neutrophils and eosinophils.

In some embodiments, the Fc region interacts with proteins of the complement system. The proteins of the complement system may include C1q.

In some embodiments, the Fc region is a ΔK447 Fc region. In some embodiments, the Fc region is an Fc region containing K447. In some embodiments, the Fc region is an Fc region containing K478.

In some embodiments, the Fc region maintains the same Fc functionality as an antibody comprising the same Fc region. In some embodiments, Fc-mediated effector functions include antibody-dependent cellular cytotoxicity (ADCC) and/or complement-dependent cytotoxicity (CDC) and/or antibody-dependent cellular phagocytosis (ADCP).

However, in some embodiments the Fc region is silenced to reduce, negate or abolish one or more Fc receptor binding and/or functionalities. In some embodiments, the Fc region of the bispecific binding molecule according to the invention is modified to negate one or more Fc receptor functionalities. In some embodiments, the Fc region of the bispecific binding molecule according to the invention is silenced in respect of one or more or all of FcγRI (CD64), FcγRIIa (CD32A), FcγRIIb (CD32B), FcγRIII (CD16) and C1q functionality.

In some embodiments, the Fc region comprises one or more STR mutations.

In some embodiments, the Fc region comprises L234S, L235T, and G236R (STR; according to EU numbering) mutations.

In some embodiments, the CH2 domain comprises one or more STR mutations.

In some embodiments, the CH2 domain comprises L234S, L235T, and G236R (STR, according to EU numbering) mutations.

In some embodiments, the CH3 domain comprises one or more STR mutations.

In some embodiments, the Fc region comprises one or more LALA mutations.

In some embodiments, the Fc region comprises L234A and L235A (according to EU numbering) mutations.

In some embodiments, the CH2 domain comprises one or more LALA mutations.

In some embodiments, the CH2 domain comprises L234A and L235A (according to EU numbering) mutations.

In some embodiments, the CH2 domain comprises the amino acid sequence according to SEQ ID NO: 59 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 59. In some embodiments, the CH2 domain consists of an amino acid sequence according to SEQ ID NO: 59.

In some embodiments, the CH2 domain comprises the amino acid sequence according to SEQ ID NO: 60 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 60. In some embodiments, the CH2 domain consists of an amino acid sequence according to SEQ ID NO: 60.

In some embodiments, the CH3 domain comprises the amino acid sequence according to SEQ ID NO: 61 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 61. In some embodiments, the CH3 domain consists of an amino acid sequence according to SEQ ID NO: 61.

In some embodiments, the CH3 domain comprises the amino acid sequence according to SEQ ID NO: 62 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 62. In some embodiments, the CH3 domain consists of an amino acid sequence according to SEQ ID NO: 62.

In some embodiments, the CH3 domain comprises the amino acid sequence according to SEQ ID NO: 63 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 63. In some embodiments, the CH3 domain consists of an amino acid sequence according to SEQ ID NO: 63.

In some embodiments, the hinge region comprises the amino acid sequence according to SEQ ID NO: 64 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 64. In some embodiments, the hinge region consists of an amino acid sequence according to SEQ ID NO: 64.

It will be understood that the first subunit of the Fc region may be present on a first polypeptide. It will also be understood that the second subunit of the Fc region may be present on a second polypeptide.

It will be understood that within the scope of the invention the first subunit and the second subunit may be the same or different, such that subunit may independently possess any combination of the features defined herein.

In some embodiments, the first subunit and/or the second subunit independently comprise one or more modifications that enhance the formation of the bispecific binding molecule according to the invention. In some embodiments, the modifications comprise knobs-into-holes modifications.

In some embodiments, the first and second subunits comprise modifications that enhance the formation of a dimer, comprising one monomer of the first subunit of the Fc region as described herein and one monomer of the second subunit of the Fc region as described herein.

It will be understood that dimerisation of the first and second subunit of the Fc region as described herein may result in dimerisation of the first and second polypeptide as described herein, thus forming the bispecific binding molecule according to the invention.

In some embodiments, the dimer is a heterodimer. In some embodiments, the dimer is a homodimer.

Challenges exist in producing bispecific antibody agents from a single composition (e.g., a composition comprising polyribonucleotides sufficient to encode multiple antibody agents), particularly because the random pairing of different antibody heavy and light chains can yield undesired antibody species. Due to the presence of mispaired byproducts, and significantly reduced production yields, sophisticated purification procedures are required to isolate the desired antibody agent in those situations (see e.g. Morrison, S.L., Nature Biotech. 25, 1233-1234, 2007, which is herein incorporated by reference). In general, the same problem of mispaired byproducts remains if recombinant expression techniques are used.

In some embodiments, the modifications can be those modifications which are widely known in the art of antibody technology for the purpose of (hetero)dimerisation between antibody chains (e.g. "knobs-into-holes" (KiH) modifications). In some embodiments, the modifications are present in the CH domains, e.g. the CH3 domains, of the first and second subunits.

In some embodiments, one or more of the CH domains of the first subunit of the Fc region as described herein and/or one or more of the CH domains of the second subunit of the Fc region as described herein comprise a modification that enhances the formation of a dimer comprising the first and second subunits of the Fc region.

In some embodiments wherein the first and second subunits of the Fc region each comprise more than one constant immunoglobulin domain, e.g. a CH2 domain and a CH3 domain, one or more or all of the CH domains may comprise such modifications.

In some embodiments, the modification is in the CH2 domain of the first and second subunits.

In some embodiments, the modification is in the CH3 domain of the first and second subunits.

As used herein, the term "enhances the formation" includes promoting dimerisation of the first and second subunits of the Fc region as described herein. Thus, in some embodiments, the modifications promote dimerisation of the first and second subunits of the Fc region as described herein, which in turn may promote dimerisation of the first and second polypeptide as described herein, thus forming the bispecific binding molecule according to the invention.

In some embodiments, the modifications promote heterodimerisation of the first and second subunits of the Fc region as described herein. By heterodimerisation it is meant that the first subunit of the Fc region is different to the second subunit of the Fc region, and the first subunit of the Fc region will form a dimer with the second subunit of the Fc region, but the first subunit of the Fc region will not form a dimer with the first subunit of the Fc region and the second subunit of the Fc region will not form a dimer with the subunit of the Fc region.

In some embodiments, the modifications promote homodimerisation of the first and second subunits of the Fc region. By homodimerisation it is meant that the first subunit of the Fc region is the same as the second subunit of the Fc region, and the first subunit of the Fc region will form a dimer with the second subunit of the Fc region.

Various means of promoting dimerisation of two domains (e.g. homo- or hetero-dimerisation) are known in the art. In some embodiments, the two domains may comprise a modification that enhances formation of dimer, e.g. a modification that increases the affinity of the two domains, promotes and/or enables the formation of one or more disulphide bonds, reduces steric hindrance to the dimerisation, promotes electrostatic and/or hydrophilic/hydrophobic interactions between the two domains, or any combination thereof.

In some embodiments, one or more of the CH domains of the first subunit of the Fc region and/or one or more of the CH domains of the second subunit of the Fc region comprise a modification that enhances the affinity between the first and second subunits of the Fc region.

In some embodiments, the modification enhances the affinity between the CH domains of the first and second subunits of the Fc region.

In some embodiments, one or more of the CH domains of the first subunit of the Fc region and/or one or more of the CH domains of the second subunit of the Fc region comprise a modification that reduces steric hindrance to the formation of the first and second polypeptide as described herein, thus forming the bispecific binding molecule according to the invention.

In some embodiments, the modification reduces steric hindrance between the CH domains of the first and second subunits of the Fc region.

In some embodiments, one or more of the CH domains of the first subunit of the Fc region and one or more of the CH domains of the second subunit of the Fc region comprise a 'knobs-into-holes' modification.

In some embodiments, one or more of the CH domains of the first subunit of the Fc region and one or more of the CH domains of the second subunit of the Fc region are associated via a 'knobs-into-holes' modification.

In some embodiments, one or more of the CH domains of the first binding molecule and/or one or more of the CH domains of the second binding molecule comprise a modification that promotes electrostatic interactions between the first and second subunits of the Fc region. In some embodiments, the modification promotes electrostatic interactions between the CH domains of the first and second subunits of the Fc region. In some embodiments, the electrostatic interactions that are promoted are favourable to the formation of a dimer (e.g. a heterodimer) comprising the first and second subunit of the Fc region.

In some embodiments, one or more of the CH domains of the first subunit of the Fc region and/or one or more of the CH domains of the second subunit of the Fc region comprise a modification that promotes hydrophilic/hydrophobic interactions between the first and second subunits of the Fc region. In some embodiments, the modification promotes hydrophilic/hydrophobic interactions between the CH domains of the first and second subunits of the Fc region. In some embodiments, the hydrophilic/hydrophobic interactions that are promoted are favourable to the formation of a dimer comprising the first and second subunits of the Fc region.

In some embodiments, a CH domain of the first subunit of the Fc region forms a disulphide bond with a CH domain of the second subunit of the Fc region. In some embodiments, a CH domain of the first subunit of the Fc region forms a disulphide bond with a CH domain of the second subunit of the Fc region, and in addition one or more of the CH domains of the first subunit of the Fc region and/or one or more of the CH domains of the second subunit of the Fc region comprise a modification that enhances the affinity between the first and second subunits of the Fc region.

Any suitable technology for promoting the dimerisation of CH domains, i.e. of at least one of the CH domains of the first subunit of the Fc region and at least one of the CH domains of the second subunit of the Fc region, may be employed in the practice of the invention. The modification that enhances the formation of a dimer between the CH domains may be KiH technology (Ridgeway et al., Protein Engineering, Design and Selection, 1996, 9: 617-621 and Merchant et al., Nat Biotechnol, 1998, 16: 677-681). The KiH technology is based on an engineered pair of CH domains that heterodimerises (CH heterodimer) in which asymmetric hydrophobic mutations are introduced between the homodimeric CH domain. KiH involves introducing mutations that create a protuberance ("knob") in the interface of the first CH domain and a corresponding cavity ("hole") in the interface of the second CH domain, such that the protuberance can be positioned in the cavity to promote heterodimer assembly and hinder homodimer formation. KiH variants therefore thermodynamically favour the formation of heterodimers rather than homodimers.

In some embodiments, one or more of the CH domains of the first subunit of the Fc region and one or more of the CH domains of the second subunit of the Fc region are associated via a KiH modification.

In some embodiments, the CH2 domain of the first subunit of the Fc region and the CH2 domain of the second subunit of the Fc region each comprise a KiH modification.

In some embodiments, the CH3 domain of the first subunit of the Fc region and the CH3 domain of the second subunit of the Fc region each comprise a KiH modification.

Any suitable KiH modifications known in the art may be employed in the practice of the present invention.

In some embodiments, the first subunit of the Fc region and the second subunit of the Fc region comprise a 'knobs-into-holes' modification.

In some embodiments, the CH2 domain of the first subunit of the Fc region and the CH2 domain of the second subunit of the Fc region comprise a 'knobs-into-holes' modification.

In some embodiments, the CH3 domain of the first subunit of the Fc region and the CH3 domain of the second subunit of the Fc region comprise a 'knobs-into-holes' modification.

It will be understood that the "Knob" modifications may comprise one or more mutations selected from the list consisting of: S354C,T366W.

It will be understood that the "Hole" modifications may comprise one or more mutations selected from the list consisting of: Y349C,T366S,L368A,Y407V.

In some embodiments, one subunit of the Fc region comprises one or more mutations selected from the list consisting of: Y349C, T366S, L368A, and Y407V (according to EU numbering). In some embodiments, the subunit comprises each of the following mutations: Y349C, T366S, L368A, and Y407V (according to EU numbering). Such a combination of mutations is referred to herein as "cah".

In some embodiments, one subunit of the Fc region comprises one or more mutations selected from the list consisting of: S354C and T366W (according to EU numbering). In some embodiments, the subunit comprises each of the following mutations: S354C and T366W (according to EU numbering). Such a combination of CH3 mutations is referred to herein as "cak".

In some embodiments, the CH3 domain of one subunit of the Fc region comprises one or more mutations selected from the list consisting of: Y349C, T366S, L368A, and Y407V (according to EU numbering). In some embodiments, the CH3 domain comprises each of the following mutations: Y349C, T366S, L368A, and Y407V (according to EU numbering). Such a combination of mutations is referred to herein as "cah".

In some embodiments, the CH3 domain of the one subunit of the Fc region comprises one or more mutations selected from the list consisting of: S354C and T366W (according to EU numbering). In some embodiments, the CH3 domain comprises each of the following mutations: S354C and T366W (according to EU numbering). Such a combination of CH3 mutations is referred to herein as "cak".

In some embodiments, the CH3 domain of the first subunit of the Fc region comprises one or more mutations selected from the list consisting of: Y349C, T366S, L368A, and Y407V (according to EU numbering). In some embodiments, the CH3 domain comprises each of the following mutations: Y349C, T366S, L368A, and Y407V (according to EU numbering). Such a combination of mutations is referred to herein as "cah".

In some embodiments, the CH3 domain of the second subunit of the Fc region comprises one or more mutations selected from the list consisting of: S354C and T366W (according to EU numbering). In some embodiments, the CH3 domain comprises each of the following mutations: S354C and T366W (according to EU numbering). Such a combination of CH3 mutations is referred to herein as "cak".

In some embodiments, the CH3 domain of the second subunit of the Fc region comprises one or more mutations selected from the list consisting of: Y349C, T366S, L368A, and Y407V (according to EU numbering). In some embodiments, the CH3 domain comprises each of the following mutations: Y349C, T366S, L368A, and Y407V (according to EU numbering). Such a combination of mutations is referred to herein as "cah".

In some embodiments, the CH3 domain of the first subunit of the Fc region comprises one or more mutations selected from the list consisting of: S354C and T366W (according to EU numbering). In some embodiments, the CH3 domain comprises each of the following mutations: S354C and T366W (according to EU numbering). Such a combination of CH3 mutations is referred to herein as "cak".

In some embodiments, the first subunit of the Fc region comprises knob mutations and Fc-silencing LALA mutations.

In some embodiments, the second subunit of the Fc region comprises hole mutations and Fc-silencing LALA mutations.

In some embodiments, the first subunit of the Fc region comprises knob mutations and Fc-silencing LALA mutations, and the second subunit of the Fc region comprises hole mutations and Fc-silencing LALA mutations.

In some embodiments, the first subunit of the Fc region comprises hole mutations and Fc-silencing LALA mutations.

In some embodiments, the second subunit of the Fc region comprises knob mutations and Fc-silencing LALA mutations.

In some embodiments, the first subunit of the Fc region comprises hole mutations and Fc-silencing LALA mutations, and the second subunit of the Fc region comprises knob mutations and Fc-silencing LALA mutations.

In some embodiments, the first subunit of the Fc region comprises knob mutations and Fc-silencing STR mutations.

In some embodiments, the second subunit of the Fc region comprises hole mutations and Fc-silencing STR mutations.

In some embodiments, the first subunit of the Fc region comprises knob mutations and Fc-silencing STR mutations, and the second subunit of the Fc region comprises hole mutations and Fc-silencing STR mutations.

In some embodiments, the first subunit of the Fc region comprises hole mutations and Fc-silencing STR mutations.

In some embodiments, the second subunit of the Fc region comprises knob mutations and Fc-silencing STR mutations.

In some embodiments, the first subunit of the Fc region comprises hole mutations and Fc-silencing STR mutations, and the second subunit of the Fc region comprises knob mutations and Fc-silencing STR mutations.

### Full-length molecule

In some embodiments, the bispecific binding molecule comprises:
(i) a first polypeptide comprising the following from N to C terminus:
   PSMA-binding VHH domain - (optional linker) - CD3-VH domain - CD3-CH1 domain - first subunit of the Fc region as described herein;
(ii) a second polypeptide comprising the following from N to C terminus:
   PSMA-binding VHH domain - (optional linker) - second subunit of the Fc region as described herein; and
(iii) a third polypeptide comprising the following from N to C terminus:
   CD3-VL domain - CD3-CL domain.

It will be understood that the hinge region may be formed from part of the CH1 domain and part of the Fc region described herein.

It will be also understood that the hinge region may be considered as separate to the CH1 domain and the Fc region described herein.

Accordingly, in some embodiments, the bispecific binding molecule comprises:
(i) a first polypeptide comprising the following from N to C terminus:
   PSMA-binding VHH domain - (optional linker) - CD3-VH domain - CD3-CH1 domain - hinge region - first subunit of the Fc region as described herein;
(ii) a second polypeptide comprising the following from N to C terminus:
   PSMA-binding VHH domain - (optional linker) - hinge region - second subunit of the Fc region as described herein; and
(iii) a third polypeptide comprising the following from N to C terminus:
   CD3-VL domain - CD3-CL domain.

In some embodiments, the first subunit of the Fc region comprises a CH2 domain as described herein and a CH3 domain as described herein.

In some embodiments, the second subunit of the Fc region comprises a CH2 domain as described herein and a CH3 domain as described herein.

In some embodiments, the bispecific binding molecule comprises:
(i) a first polypeptide comprising the following from N to C terminus:
   PSMA-binding VHH domain - (optional linker) - CD3-VH domain - CD3-CH1 domain - CH2 domain - CH3 domain;
(ii) a second polypeptide comprising the following from N to C terminus:
   PSMA-binding VHH domain - (optional linker) - CH2 domain - CH3 domain; and
(iii) a third polypeptide comprising the following from N to C terminus:
   CD3-VL domain - CD3-CL domain.

It will be understood that the hinge region may be formed from part of the CH1 domain and part of the CH2 domain described herein.

It will be also understood that the hinge region may be considered as separate to the CH1 domain and the CH2 domain described herein.

In some embodiments, the bispecific binding molecule comprises:
(i) a first polypeptide comprising the following from N to C terminus:
   PSMA-binding VHH domain - (optional linker) - CD3-VH domain - CD3-CH1 domain - hinge region - CH2 domain - CH3 domain;
(ii) a second polypeptide comprising the following from N to C terminus:
   PSMA-binding VHH domain - (optional linker) - hinge region - CH2 domain - CH3 domain; and
(iii) a third polypeptide comprising the following from N to C terminus:
   CD3-VL domain - CD3-CL domain.

In some embodiments, the first polypeptide comprises knob mutations and Fc-silencing LALA mutations.

In some embodiments, the second polypeptide comprises a hole mutations and Fc-silencing LALA mutations.

In some embodiments, the first polypeptide comprises knob mutations and Fc-silencing LALA mutations, and the second polypeptide comprises a hole mutations and Fc-silencing LALA mutations.

In some embodiments, the first polypeptide comprises a hole mutations and Fc-silencing LALA mutations.

In some embodiments, the second polypeptide comprises knob mutations and Fc-silencing LALA mutations.

In some embodiments, the first polypeptide comprises a hole mutations and Fc-silencing LALA mutations, and the second polypeptide comprises knob mutations and Fc-silencing LALA mutations.

In some embodiments, the first polypeptide comprises the amino acid sequence according to SEQ ID NO: 65 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 65. In some embodiments, the first polypeptide consists of an amino acid sequence according to SEQ ID NO: 65.

In some embodiments, the first polypeptide comprises the amino acid sequence according to SEQ ID NO: 66 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 66. In some embodiments, the first polypeptide consists of an amino acid sequence according to SEQ ID NO: 66.

In some embodiments, the first polypeptide comprises the amino acid sequence according to SEQ ID NO: 67 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 67. In some embodiments, the first polypeptide consists of an amino acid sequence according to SEQ ID NO: 67.

In some embodiments, the first polypeptide comprises the amino acid sequence according to SEQ ID NO: 68 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 68. In some embodiments, the first polypeptide consists of an amino acid sequence according to SEQ ID NO: 68.

In some embodiments, the first polypeptide comprises the amino acid sequence according to SEQ ID NO: 69 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 69. In some embodiments, the first polypeptide consists of an amino acid sequence according to SEQ ID NO: 69.

In some embodiments, the first polypeptide comprises the amino acid sequence according to SEQ ID NO: 70 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 70. In some embodiments, the first polypeptide consists of an amino acid sequence according to SEQ ID NO: 70.

In some embodiments, the second polypeptide comprises the amino acid sequence according to SEQ ID NO: 71 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 71. In some embodiments, the second polypeptide consists of an amino acid sequence according to SEQ ID NO: 71.

In some embodiments, the second polypeptide comprises the amino acid sequence according to SEQ ID NO: 72 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 72. In some embodiments, the second polypeptide consists of an amino acid sequence according to SEQ ID NO: 72.

In some embodiments, the third polypeptide comprises a third polypeptide comprising the amino acid sequence according to SEQ ID NO: 73 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 73. In some embodiments, the third polypeptide consists of an amino acid sequence according to SEQ ID NO: 73.

In some embodiments, the third polypeptide comprises a third polypeptide comprising the amino acid sequence according to SEQ ID NO: 74 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 74. In some embodiments, the third polypeptide consists of an amino acid sequence according to SEQ ID NO: 74.

In some embodiments, the third polypeptide comprises a third polypeptide comprising the amino acid sequence according to SEQ ID NO: 75 or a variant having at least 80% sequence identity thereto. In some embodiments, the variant has at least 85%, at least 90%, at least 95%, at least 96%, at least 97%, at least 98%, at least 99%, or 100% sequence identity to SEQ ID NO: 75. In some embodiments, the third polypeptide consists of an amino acid sequence according to SEQ ID NO: 75.

In some embodiments, the bispecific binding molecule comprises:
(i) a first polypeptide comprising the amino acid sequence according to SEQ ID NO: 65 or a variant having at least 80% sequence identity thereto;
(ii) a second polypeptide comprising the amino acid sequence according to SEQ ID NO: 71 or a variant having at least 80% sequence identity thereto; and
(iii) a third polypeptide comprising the amino acid sequence according to SEQ ID NO: 73 or a variant having at least 80% sequence identity thereto.

In some embodiments, each variant independently has at least 85%, at least 90%, at least 95%, at least 96%, at least 97%, at least 98%, at least 99%, or 100% sequence identity thereto.

In some embodiments, the bispecific binding molecule comprises:
(i) a first polypeptide comprising the amino acid sequence according to SEQ ID NO: 65;
(ii) a second polypeptide comprising the amino acid sequence according to SEQ ID NO: 71; and
(iii) a third polypeptide comprising the amino acid sequence according to SEQ ID NO: 73.

In some embodiments, the bispecific binding molecule consists of:
(i) a first polypeptide comprising the amino acid sequence according to SEQ ID NO: 65;
(ii) a second polypeptide comprising the amino acid sequence according to SEQ ID NO: 71; and
(iii) a third polypeptide comprising the amino acid sequence according to SEQ ID NO: 73.

In some embodiments, the bispecific binding molecule comprises:
(i) a first polypeptide comprising the amino acid sequence according to SEQ ID NO: 66 or a variant having at least 80% sequence identity thereto;
(ii) a second polypeptide comprising the amino acid sequence according to SEQ ID NO: 71 or a variant having at least 80% sequence identity thereto; and
(iii) a third polypeptide comprising the amino acid sequence according to SEQ ID NO: 74 or a variant having at least 80% sequence identity thereto.

In some embodiments, each variant independently has at least 85%, at least 90%, at least 95%, at least 96%, at least 97%, at least 98%, at least 99%, or 100% sequence identity thereto.

In some embodiments, the bispecific binding molecule comprises:
(i) a first polypeptide comprising the amino acid sequence according to SEQ ID NO: 66;
(ii) a second polypeptide comprising the amino acid sequence according to SEQ ID NO: 71; and
(iii) a third polypeptide comprising the amino acid sequence according to SEQ ID NO: 74.

In some embodiments, the bispecific binding molecule consists of:
(i) a first polypeptide comprising the amino acid sequence according to SEQ ID NO: 66;
(ii) a second polypeptide comprising the amino acid sequence according to SEQ ID NO: 71; and
(iii) a third polypeptide comprising the amino acid sequence according to SEQ ID NO: 74.

In some embodiments, the bispecific binding molecule comprises:
(i) a first polypeptide comprising the amino acid sequence according to SEQ ID NO: 67 or a variant having at least 80% sequence identity thereto;
(ii) a second polypeptide comprising the amino acid sequence according to SEQ ID NO: 71 or a variant having at least 80% sequence identity thereto; and
(iii) a third polypeptide comprising the amino acid sequence according to SEQ ID NO: 75 or a variant having at least 80% sequence identity thereto.

In some embodiments, each variant independently has at least 85%, at least 90%, at least 95%, at least 96%, at least 97%, at least 98%, at least 99%, or 100% sequence identity thereto.

In some embodiments, the bispecific binding molecule comprises:
(i) a first polypeptide comprising the amino acid sequence according to SEQ ID NO: 67;
(ii) a second polypeptide comprising the amino acid sequence according to SEQ ID NO: 71; and
(iii) a third polypeptide comprising the amino acid sequence according to SEQ ID NO: 75.

In some embodiments, the bispecific binding molecule consists of:
(i) a first polypeptide comprising the amino acid sequence according to SEQ ID NO: 67;
(ii) a second polypeptide comprising the amino acid sequence according to SEQ ID NO: 71; and
(iii) a third polypeptide comprising the amino acid sequence according to SEQ ID NO: 75.

In some embodiments, the bispecific binding molecule comprises:
(i) a first polypeptide comprising the amino acid sequence according to SEQ ID NO: 68 or a variant having at least 80% sequence identity thereto;
(ii) a second polypeptide comprising the amino acid sequence according to SEQ ID NO: 72 or a variant having at least 80% sequence identity thereto; and
(iii) a third polypeptide comprising the amino acid sequence according to SEQ ID NO: 73 or a variant having at least 80% sequence identity thereto.

In some embodiments, each variant independently has at least 85%, at least 90%, at least 95%, at least 96%, at least 97%, at least 98%, at least 99%, or 100% sequence identity thereto.

In some embodiments, the bispecific binding molecule comprises:
(i) a first polypeptide comprising the amino acid sequence according to SEQ ID NO: 68;
(ii) a second polypeptide comprising the amino acid sequence according to SEQ ID NO: 72; and
(iii) a third polypeptide comprising the amino acid sequence according to SEQ ID NO: 73.

In some embodiments, the bispecific binding molecule consists of:
(i) a first polypeptide comprising the amino acid sequence according to SEQ ID NO: 68;
(ii) a second polypeptide comprising the amino acid sequence according to SEQ ID NO: 72; and
(iii) a third polypeptide comprising the amino acid sequence according to SEQ ID NO: 73.

In some embodiments, the bispecific binding molecule comprises:
(i) a first polypeptide comprising the amino acid sequence according to SEQ ID NO: 69 or a variant having at least 80% sequence identity thereto;
(ii) a second polypeptide comprising the amino acid sequence according to SEQ ID NO: 72 or a variant having at least 80% sequence identity thereto; and
(iii) a third polypeptide comprising the amino acid sequence according to SEQ ID NO: 74 or a variant having at least 80% sequence identity thereto.

In some embodiments, each variant independently has at least 85%, at least 90%, at least 95%, at least 96%, at least 97%, at least 98%, at least 99%, or 100% sequence identity thereto.

In some embodiments, the bispecific binding molecule comprises:
(i) a first polypeptide comprising the amino acid sequence according to SEQ ID NO: 69;
(ii) a second polypeptide comprising the amino acid sequence according to SEQ ID NO: 72; and
(iii) a third polypeptide comprising the amino acid sequence according to SEQ ID NO: 74.

In some embodiments, the bispecific binding molecule consists of:
(i) a first polypeptide comprising the amino acid sequence according to SEQ ID NO: 69;
(ii) a second polypeptide comprising the amino acid sequence according to SEQ ID NO: 72; and
(iii) a third polypeptide comprising the amino acid sequence according to SEQ ID NO: 74.

In some embodiments, the bispecific binding molecule comprises:
(i) a first polypeptide comprising the amino acid sequence according to SEQ ID NO: 70 or a variant having at least 80% sequence identity thereto;
(ii) a second polypeptide comprising the amino acid sequence according to SEQ ID NO: 72 or a variant having at least 80% sequence identity thereto; and
(iii) a third polypeptide comprising the amino acid sequence according to SEQ ID NO: 75 or a variant having at least 80% sequence identity thereto.

In some embodiments, each variant independently has at least 85%, at least 90%, at least 95%, at least 96%, at least 97%, at least 98%, at least 99%, or 100% sequence identity thereto.

In some embodiments, the bispecific binding molecule comprises:
(i) a first polypeptide comprising the amino acid sequence according to SEQ ID NO: 70;
(ii) a second polypeptide comprising the amino acid sequence according to SEQ ID NO: 72; and
(iii) a third polypeptide comprising the amino acid sequence according to SEQ ID NO: 75.

In some embodiments, the bispecific binding molecule consists of:
(i) a first polypeptide comprising the amino acid sequence according to SEQ ID NO: 70;
(ii) a second polypeptide comprising the amino acid sequence according to SEQ ID NO: 72; and
(iii) a third polypeptide comprising the amino acid sequence according to SEQ ID NO: 75.

### POLYNUCLEOTIDE

The present invention provides a polynucleotide comprising a nucleic acid sequence encoding the bispecific binding molecule according to the invention.

In some embodiments, the polynucleotide comprises a nucleic acid sequence(s) as shown in Table 1.

The present invention provides one or more polynucleotide(s) comprising a nucleic acid sequence encoding the bispecific binding molecule according to the invention.

In some embodiments, the one or more polynucleotide(s) comprises one or more nucleic acid sequence(s) as shown in Table 1.

As used herein, the terms "nucleotide sequence", and "nucleic acid sequence" are intended to be synonymous with each other. The nucleic acid sequence may be a DNA or an RNA sequence. The nucleic acid sequence may be single-stranded or may be double-stranded. The nucleic acid sequence may be, for example, genomic, recombinant, cDNA or mRNA. The nucleic acid sequence may comprise synthetic nucleotides and/or modified nucleotides. These synthetic nucleotides and/or modified nucleotides may enhance the *in vivo* activity and/or stability of the polynucleotide.

In some embodiments, the, or each, nucleic acid sequence is independently a DNA sequence.

In some embodiments, the, or each, nucleic acid sequence is independently a cDNA sequence.

In some embodiments, the, or each, nucleic acid sequence is independently an RNA sequence.

In some embodiments, the, or each, nucleic acid sequence is independently an mRNA sequence.

In some embodiments, the, or each RNA sequence is independently *in vitro* transcribed RNA. Processes for producing *in vitro* transcribed RNA are known in the art. For example, CleanCap 413 ((m7(3'OMeG)(5')ppp(5')(2'OMeA)pG)-capped RNA can be produced following the process as disclosed, e.g., in Kreiter, S. et al. Cancer Immunol. Immunother. 56, 1577-87 (2007) and WO 2021214204A1, each of which is incorporated herein by reference in its entirety. Methyl pseudo-uridine can be used in the *in vitro* transcription reaction and incorporated into the produced RNA.

In some embodiments, the, or each RNA sequence is independently modRNA (N1-methyl-pseudouridine).

In some embodiments, the modRNA contains N1-methyl-pseudouridine instead of uridine.

In some embodiments, an additional purification step may be applied for modRNA to reduce double stranded RNA contaminants generated during the *in vitro* transcription reaction.

In some embodiments, the nucleic acid sequence(s) contain one or more structural elements optimized for maximal efficacy of the RNA or mRNA with respect to stability and translational efficiency, wherein the structural elements are selected from the list consisting of: a 5'-cap, a 5'-UTR, a 3'-UTR, and a poly(A)-tail.

In some embodiments, the nucleic acid sequence(s) comprise a 5'-cap, a 5'-UTR, a 3'-UTR, and a poly(A)-tail.

In some embodiments, the, or each RNA sequence independently comprises a 5'-cap.

In some embodiments, the 5'-cap is CC413. It will be understood that CC413 may be defined as CleanCap^{®} Reagent AG (3' OMe) - (N-7413), which has the molecular formula: C33H45N15O24P4 (free acid).

In some embodiments, the 5'-cap is m₂^{7,3'-O}Gppp(ₘ₁^{2'-O})ApG, which comprises the following structure:

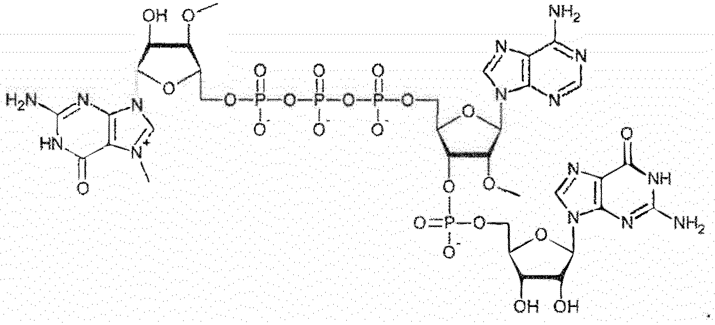

In some embodiments, the, or each RNA sequence independently comprises a poly adenine sequence (poly(A)-tail).

In some embodiments, the poly(A) tail may be about 10 to about 250 nucleotides in length.

In some embodiments, the poly(A) tail may be about 50 to about 200 nucleotides in length.

In some embodiments, the poly(A) tail may be about 50 to about 100 nucleotides in length.

In some embodiments, the poly(A) tail may be about 100 to about 200 nucleotides in length.

In some embodiments, the poly(A) tail may be about 100 to about 150 nucleotides in length.

In some embodiments, the poly(A) tail may be about 100 to about 120 nucleotides in length.

In some embodiments, the poly(A) tail may be poly A30LA70.

In some embodiments, the poly(A) tail comprises or consists of the nucleic acid sequence according to SEQ ID NO: 43. In some embodiments, the poly(A) tail may comprise a variant having at least 80% sequence identity to SEQ ID NO: 43. In some embodiments, the variant may have at least 85%, at least 90%, at least 95%, at least 96%, at least 97%, at least 98%, at least 99%, or 100% sequence identity to SEQ ID NO: 43. In some embodiments, the poly(A) tail may consist of the nucleic acid sequence according to SEQ ID NO: 43.

In some embodiments, the, or each RNA sequence independently comprises a 5'UTR.

In some embodiments, the 5'UTR comprises the nucleic acid sequence according to SEQ ID NO: 44. In some embodiments, the 5'UTR comprises a variant having at least 80% sequence identity to SEQ ID NO: 44. In some embodiments, the variant has at least 85%, at least 90%, at least 95%, at least 96%, at least 97%, at least 98%, at least 99%, or 100% sequence identity to SEQ ID NO: 44. In some embodiments, the 5'UTR consists of the nucleic acid sequence according to SEQ ID NO: 44.

In some embodiments, the, or each RNA sequence independently comprises a 3'UTR

In some embodiments, the 3'UTR comprises the nucleic acid sequence according to SEQ ID NO: 45. In some embodiments, the 3'UTR comprises a variant having at least 80% sequence identity to SEQ ID NO: 45. In some embodiments, the variant has at least 85%, at least 90%, at least 95%, at least 96%, at least 97%, at least 98%, at least 99%, or 100% sequence identity to SEQ ID NO: 45. In some embodiments, the 3'UTR consists of the nucleic acid sequence according to SEQ ID NO: 45.

In some embodiments, the nucleic acid sequence(s) comprises a Kozak sequence. Kozak sequences are well known to those in the art.

In some embodiments, the nucleic acid sequence(s) comprises a signal peptide sequence (e.g., husec2).

In some embodiments, one polynucleotide comprises a nucleic acid sequence encoding the following polypeptide chain from N to C terminus:
PSMA-binding moiety, preferably the PSMA-binding VHH domain, - (optional linker) - CD3-VH domain - CD3-CH1 domain - CH2 domain - CH3 domain

In some embodiments, one polynucleotide comprises a nucleic acid sequence encoding the following polypeptide chain from N to C terminus:
PSMA-binding moiety, preferably the PSMA-binding VHH domain, - (optional linker) - CH2 domain - CH3 domain

In some embodiments, one polynucleotide comprises a nucleic acid sequence encoding the following polypeptide chain from N to C terminus:
PSMA-binding moiety, preferably the PSMA-binding VHH domain, - (optional linker) - CD3-VH domain - CD3-CH1 domain - hinge region - CH2 domain - CH3 domain

In some embodiments, one polynucleotide comprises a nucleic acid sequence encoding the following polypeptide chain from N to C terminus:
PSMA-binding moiety, preferably the PSMA-binding VHH domain, - (optional linker) - hinge region - CH2 domain - CH3 domain

In some embodiments, one polynucleotide comprises a nucleic acid sequence encoding the following polypeptide chain from N to C terminus:
CD3-VL domain - CD3-CL domain

It will be understood that the PSMA-binding moiety, PSMA-binding VHH domain, CD3-VH domain, CD3-CH1 domain, CD3-VL domain, CD3-CL domain, CH2 domain, and CH3 domain may be defined as described herein.

In some embodiments, one polynucleotide comprises a nucleic acid sequence comprising in 5'-3' orientation:
5'-cap - 5'UTR - PSMA-binding moiety, preferably the PSMA-binding VHH domain, - (optional linker) - CD3-VH domain - CD3-CH1 domain - CH2 domain - CH3 domain - 3'UTR - Poly(A)

In some embodiments, one polynucleotide comprises a nucleic acid sequence comprising in 5'-3' orientation:
5'-cap - 5'UTR - PSMA-binding moiety, preferably the PSMA-binding VHH domain, - (optional linker) - CH2 domain - CH3 domain - 3'UTR - Poly(A)

In some embodiments, one polynucleotide comprises a nucleic acid sequence comprising in 5'-3' orientation:
5'-cap - 5'UTR - PSMA-binding moiety, preferably the PSMA-binding VHH domain, - (optional linker) - CD3-VH domain - CD3-CH1 domain - hinge region - CH2 domain - CH3 domain - 3'UTR - Poly(A)

In some embodiments, one polynucleotide comprises a nucleic acid sequence comprising in 5'-3' orientation:
5'-cap - 5'UTR - PSMA-binding moiety, preferably the PSMA-binding VHH domain, - (optional linker) - hinge region - CH2 domain - CH3 domain - 3'UTR - Poly(A)

In some embodiments, one polynucleotide comprises a nucleic acid sequence comprising in 5'-3' orientation:
5'-cap - 5'UTR - CD3-VL domain - CD3-CL domain - 3'UTR - Poly(A)

It will be understood that the PSMA-binding moiety, PSMA-binding VHH domain, CD3-VH domain, CD3-CH1 domain, CD3-VL domain, CD3-CL domain, CH2 domain, and CH3 domain may be defined as described herein.

It will be understood that the 5'-cap, the 5'UTR, the 3'UTR and the Poly(A) tail may be defined as described herein.

The present invention provides a plurality of polynucleotides encoding the bispecific -binding molecule according to the invention, wherein the plurality of polynucleotides independently comprise a nucleic acid sequence encoding part of the bispecific binding molecule according to the invention.

In other words, the bispecific binding molecule according to the invention may be encoded by a plurality of polynucleotides, such that expression of the plurality of polynucleotides results in polypeptides that form the bispecific binding molecule according to the invention.

In some embodiments, a plurality of polynucleotides comprise:
(i) a first polynucleotide comprising a nucleic acid sequence encoding the following polypeptide chain from N to C terminus:
   PSMA-binding moiety, preferably the PSMA-binding VHH domain, - (optional linker) - CD3-VH domain - CD3-CH1 domain - CH2 domain - CH3 domain;
(ii) a second polynucleotide comprising a nucleic acid sequence encoding the following polypeptide chain from N to C terminus:
   PSMA-binding moiety, preferably the PSMA-binding VHH domain, - (optional linker) - CH2 domain - CH3 domain; and
(iii) a third polynucleotide comprising a nucleic acid sequence encoding the following polypeptide chain from N to C terminus:
   CD3-VL domain - CD3-CL domain.

In some embodiments, a plurality of polynucleotides comprise:
(i) a first polynucleotide comprising a nucleic acid sequence encoding the following polypeptide chain from N to C terminus:
   PSMA-binding moiety, preferably the PSMA-binding VHH domain, - (optional linker) - CD3-VH domain - CD3-CH1 domain - hinge region - CH2 domain - CH3 domain;
(ii) a second polynucleotide comprising a nucleic acid sequence encoding the following polypeptide chain from N to C terminus:
   PSMA-binding moiety, preferably the PSMA-binding VHH domain, - (optional linker) - hinge region - CH2 domain - CH3 domain; and
(iii) a third polynucleotide comprising a nucleic acid sequence encoding the following polypeptide chain from N to C terminus:
   CD3-VL domain - CD3-CL domain.

It will be understood that the PSMA-binding moiety, PSMA-binding VHH domain, CD3-VH domain, CD3-CH1 domain, CD3-VL domain, CD3-CL domain, CH2 domain, and CH3 domain may be defined as described herein.

In some embodiments, the plurality of polynucleotides comprise one or more copies of the third polynucleotide according to part (iii) above.

In some embodiments, the plurality of polynucleotides comprise two copies of the third polynucleotide according to part (iii) above.

In some embodiments, a plurality of polynucleotides comprise:
(i) a first polynucleotide comprising a nucleic acid sequence comprising in 5'-3' orientation:
   5'-cap - 5'UTR - PSMA-binding moiety, preferably the PSMA-binding VHH domain, - (optional linker) - CD3-VH domain - CD3-CH1 domain - CH2 domain - CH3 domain - 3'UTR - Poly(A)
(ii) a second polynucleotide comprising a nucleic acid sequence comprising in 5'-3' orientation:
   5'-cap - 5'UTR - PSMA-binding moiety, preferably the PSMA-binding VHH domain, - (optional linker) - CH2 domain - CH3 domain - 3'UTR - Poly(A)
(iii) a third polynucleotide comprising a nucleic acid sequence comprising in 5'-3' orientation:
   5'-cap - 5'UTR - CD3-VL domain - CD3-CL domain - 3'UTR - Poly(A)

In some embodiments, a plurality of polynucleotides comprise:
(i) a first polynucleotide comprising a nucleic acid sequence comprising in 5'-3' orientation:
   5'-cap - 5'UTR - PSMA-binding moiety, preferably the PSMA-binding VHH domain, - (optional linker) - CD3-VH domain - CD3-CH1 domain - hinge region - CH2 domain - CH3 domain - 3'UTR - Poly(A)
(ii) a second polynucleotide comprising a nucleic acid sequence comprising in 5'-3' orientation:
   5'-cap - 5'UTR - PSMA-binding moiety, preferably the PSMA-binding VHH domain, - (optional linker) - hinge region - CH2 domain - CH3 domain - 3'UTR - Poly(A)
(iii) a third polynucleotide comprising a nucleic acid sequence comprising in 5'-3' orientation:
   5'-cap - 5'UTR - CD3-VL domain - CD3-CL domain - 3'UTR - Poly(A)

It will be understood that the PSMA-binding moiety, PSMA-binding VHH domain, CD3-VH domain, CD3-CH1 domain, CD3-VL domain, CD3-CL domain, CH2 domain, and CH3 domain may be defined as described herein.

It will be understood that the 5'-cap, the 5'UTR, the 3'UTR and the Poly(A) tail may be defined as described above.

In some embodiments, the plurality of polynucleotides may comprise one or more copies of the third polynucleotide according to part (iii) above.

In some embodiments, the plurality of polynucleotides may comprise two copies of the third polynucleotide according to part (iii) above.

In some embodiments, the first polynucleotide comprises the nucleic acid sequence according to SEQ ID NO: 46.

In some embodiments, the first polynucleotide comprises a variant having at least 80% sequence identity to SEQ ID NO: 46. In some embodiments, the variant has at least 85%, at least 90%, at least 95%, at least 96%, at least 97%, at least 98%, at least 99%, or 100% sequence identity to SEQ ID NO: 46.

In some embodiments, the first polynucleotide consists of the nucleic acid sequence according to SEQ ID NO: 46.

In some embodiments, the first polynucleotide comprises the nucleic acid sequence according to SEQ ID NO: 47.

In some embodiments, the first polynucleotide comprises a variant having at least 80% sequence identity to SEQ ID NO: 47. In some embodiments, the variant has at least 85%, at least 90%, at least 95%, at least 96%, at least 97%, at least 98%, at least 99%, or 100% sequence identity to SEQ ID NO: 47.

In some embodiments, the first polynucleotide consists of the nucleic acid sequence according to SEQ ID NO: 47.

In some embodiments, the first polynucleotide comprises the nucleic acid sequence according to SEQ ID NO: 48.

In some embodiments, the first polynucleotide comprises a variant having at least 80% sequence identity to SEQ ID NO: 48. In some embodiments, the variant has at least 85%, at least 90%, at least 95%, at least 96%, at least 97%, at least 98%, at least 99%, or 100% sequence identity to SEQ ID NO: 48.

In some embodiments, the first polynucleotide consists of the nucleic acid sequence according to SEQ ID NO: 48.

In some embodiments, the first polynucleotide comprises the nucleic acid sequence according to SEQ ID NO: 49.

In some embodiments, the first polynucleotide comprises a variant having at least 80% sequence identity to SEQ ID NO: 49. In some embodiments, the variant has at least 85%, at least 90%, at least 95%, at least 96%, at least 97%, at least 98%, at least 99%, or 100% sequence identity to SEQ ID NO: 49.

In some embodiments, the first polynucleotide consists of the nucleic acid sequence according to SEQ ID NO: 49.

In some embodiments, the first polynucleotide comprises the nucleic acid sequence according to SEQ ID NO: 50.

In some embodiments, the first polynucleotide comprises a variant having at least 80% sequence identity to SEQ ID NO: 50. In some embodiments, the variant has at least 85%, at least 90%, at least 95%, at least 96%, at least 97%, at least 98%, at least 99%, or 100% sequence identity to SEQ ID NO: 50.

In some embodiments, the first polynucleotide consists of the nucleic acid sequence according to SEQ ID NO: 50.

In some embodiments, the first polynucleotide comprises the nucleic acid sequence according to SEQ ID NO: 51.

In some embodiments, the first polynucleotide comprises a variant having at least 80% sequence identity to SEQ ID NO: 51. In some embodiments, the variant has at least 85%, at least 90%, at least 95%, at least 96%, at least 97%, at least 98%, at least 99%, or 100% sequence identity to SEQ ID NO: 51.

In some embodiments, the first polynucleotide consists of the nucleic acid sequence according to SEQ ID NO: 51.

In some embodiments, the second polynucleotide comprises the nucleic acid sequence according to SEQ ID NO: 52.

In some embodiments, the second polynucleotide comprises a variant having at least 80% sequence identity to SEQ ID NO: 52. In some embodiments, the variant has at least 85%, at least 90%, at least 95%, at least 96%, at least 97%, at least 98%, at least 99%, or 100% sequence identity to SEQ ID NO: 52.

In some embodiments, the second polynucleotide consists of the nucleic acid sequence according to SEQ ID NO: 52.

In some embodiments, the second polynucleotide comprises the nucleic acid sequence according to SEQ ID NO: 53.

In some embodiments, the second polynucleotide comprises a variant having at least 80% sequence identity to SEQ ID NO: 53. In some embodiments, the variant has at least 85%, at least 90%, at least 95%, at least 96%, at least 97%, at least 98%, at least 99%, or 100% sequence identity to SEQ ID NO: 53.

In some embodiments, the second polynucleotide consists of the nucleic acid sequence according to SEQ ID NO: 53.

In some embodiments, the third polynucleotide comprises the nucleic acid sequence according to SEQ ID NO: 54.

In some embodiments, the third polynucleotide comprises a variant having at least 80% sequence identity to SEQ ID NO: 54. In some embodiments, the variant has at least 85%, at least 90%, at least 95%, at least 96%, at least 97%, at least 98%, at least 99%, or 100% sequence identity to SEQ ID NO: 54.

In some embodiments, the third polynucleotide consists of the nucleic acid sequence according to SEQ ID NO: 54.

In some embodiments, the third polynucleotide comprises the nucleic acid sequence according to SEQ ID NO: 55.

In some embodiments, the third polynucleotide comprises a variant having at least 80% sequence identity to SEQ ID NO: 55. In some embodiments, the variant has at least 85%, at least 90%, at least 95%, at least 96%, at least 97%, at least 98%, at least 99%, or 100% sequence identity to SEQ ID NO: 55.

In some embodiments, the third polynucleotide consists of the nucleic acid sequence according to SEQ ID NO: 55.

In some embodiments, the third polynucleotide comprises the nucleic acid sequence according to SEQ ID NO: 56.

In some embodiments, the third polynucleotide comprises a variant having at least 80% sequence identity to SEQ ID NO: 56. In some embodiments, the variant has at least 85%, at least 90%, at least 95%, at least 96%, at least 97%, at least 98%, at least 99%, or 100% sequence identity to SEQ ID NO: 56.

In some embodiments, the third polynucleotide consists of the nucleic acid sequence according to SEQ ID NO: 56.

In some embodiments, the plurality of polynucleotides comprises:
(i) the first polynucleotide comprises a nucleic acid sequence according to SEQ ID NO: 46 or a variant having at least 80% sequence identity to SEQ ID NO: 46;
(ii) the second polynucleotide comprises a nucleic acid sequence according to SEQ ID NO: 52 or a variant having at least 80% sequence identity to SEQ ID NO: 52; and
(iii) the third polynucleotide comprises a nucleic acid sequence according to SEQ ID NO: 54 or a variant having at least 80% sequence identity to SEQ ID NO: 54.

In some embodiments, each variant independently has at least 85%, at least 90%, at least 95%, at least 96%, at least 97%, at least 98%, at least 99%, or 100% sequence identity thereto.

In some embodiments, the plurality of polynucleotides comprises:
(i) the first polynucleotide comprises a nucleic acid sequence according to SEQ ID NO: 46;
(ii) the second polynucleotide comprises a nucleic acid sequence according to SEQ ID NO: 52; and
(iii) the third polynucleotide comprises a nucleic acid sequence according to SEQ ID NO: 54.

In some embodiments, the plurality of polynucleotides comprises:
(i) the first polynucleotide comprises a nucleic acid sequence according to SEQ ID NO: 47 or a variant having at least 80% sequence identity to SEQ ID NO: 47;
(ii) the second polynucleotide comprises a nucleic acid sequence according to SEQ ID NO: 52 or a variant having at least 80% sequence identity to SEQ ID NO: 52; and
(iii) the third polynucleotide comprises a nucleic acid sequence according to SEQ ID NO: 55 or a variant having at least 80% sequence identity to SEQ ID NO: 55.

In some embodiments, each variant independently has at least 85%, at least 90%, at least 95%, at least 96%, at least 97%, at least 98%, at least 99%, or 100% sequence identity thereto.

In some embodiments, the plurality of polynucleotides comprises:
(i) the first polynucleotide comprises a nucleic acid sequence according to SEQ ID NO: 47;
(ii) the second polynucleotide comprises a nucleic acid sequence according to SEQ ID NO: 52; and
(iii) the third polynucleotide comprises a nucleic acid sequence according to SEQ ID NO: 55.

In some embodiments, the plurality of polynucleotides comprises:
(i) the first polynucleotide comprises a nucleic acid sequence according to SEQ ID NO: 48 or a variant having at least 80% sequence identity to SEQ ID NO: 48;
(ii) the second polynucleotide comprises a nucleic acid sequence according to SEQ ID NO: 52 or a variant having at least 80% sequence identity to SEQ ID NO: 52; and
(iii) the third polynucleotide comprises a nucleic acid sequence according to SEQ ID NO: 56 or a variant having at least 80% sequence identity to SEQ ID NO: 56.

In some embodiments, each variant independently has at least 85%, at least 90%, at least 95%, at least 96%, at least 97%, at least 98%, at least 99%, or 100% sequence identity thereto.

In some embodiments, the plurality of polynucleotides comprises:
(i) the first polynucleotide comprises a nucleic acid sequence according to SEQ ID NO: 48;
(ii) the second polynucleotide comprises a nucleic acid sequence according to SEQ ID NO: 52; and
(iii) the third polynucleotide comprises a nucleic acid sequence according to SEQ ID NO: 56.

In some embodiments, the plurality of polynucleotides comprises:
(i) the first polynucleotide comprises a nucleic acid sequence according to SEQ ID NO: 49 or a variant having at least 80% sequence identity to SEQ ID NO: 49;
(ii) the second polynucleotide comprises a nucleic acid sequence according to SEQ ID NO: 53 or a variant having at least 80% sequence identity to SEQ ID NO: 53; and
(iii) the third polynucleotide comprises a nucleic acid sequence according to SEQ ID NO: 54 or a variant having at least 80% sequence identity to SEQ ID NO: 54.

In some embodiments, each variant independently has at least 85%, at least 90%, at least 95%, at least 96%, at least 97%, at least 98%, at least 99%, or 100% sequence identity thereto.

In some embodiments, the plurality of polynucleotides comprises:
(i) the first polynucleotide comprises a nucleic acid sequence according to SEQ ID NO: 49;
(ii) the second polynucleotide comprises a nucleic acid sequence according to SEQ ID NO: 53; and
(iii) the third polynucleotide comprises a nucleic acid sequence according to SEQ ID NO: 54.

In some embodiments, the plurality of polynucleotides comprises:
(i) the first polynucleotide comprises a nucleic acid sequence according to SEQ ID NO: 50 or a variant having at least 80% sequence identity to SEQ ID NO: 50;
(ii) the second polynucleotide comprises a nucleic acid sequence according to SEQ ID NO: 53 or a variant having at least 80% sequence identity to SEQ ID NO: 53; and
(iii) the third polynucleotide comprises a nucleic acid sequence according to SEQ ID NO: 55 or a variant having at least 80% sequence identity to SEQ ID NO: 55.

In some embodiments, each variant independently has at least 85%, at least 90%, at least 95%, at least 96%, at least 97%, at least 98%, at least 99%, or 100% sequence identity thereto.

In some embodiments, the plurality of polynucleotides comprises:
(i) the first polynucleotide comprises a nucleic acid sequence according to SEQ ID NO: 50;
(ii) the second polynucleotide comprises a nucleic acid sequence according to SEQ ID NO: 53; and
(iii) the third polynucleotide comprises a nucleic acid sequence according to SEQ ID NO: 55.

In some embodiments, the plurality of polynucleotides comprises:
(i) the first polynucleotide comprises a nucleic acid sequence according to SEQ ID NO: 51 or a variant having at least 80% sequence identity to SEQ ID NO: 51;
(ii) the second polynucleotide comprises a nucleic acid sequence according to SEQ ID NO: 53 or a variant having at least 80% sequence identity to SEQ ID NO: 53; and
(iii) the third polynucleotide comprises a nucleic acid sequence according to SEQ ID NO: 56 or a variant having at least 80% sequence identity to SEQ ID NO: 56.

In some embodiments, each variant independently has at least 85%, at least 90%, at least 95%, at least 96%, at least 97%, at least 98%, at least 99%, or 100% sequence identity thereto.

In some embodiments, the plurality of polynucleotides comprises:
(i) the first polynucleotide comprises a nucleic acid sequence according to SEQ ID NO: 51;
(ii) the second polynucleotide comprises a nucleic acid sequence according to SEQ ID NO: 53; and
(iii) the third polynucleotide comprises a nucleic acid sequence according to SEQ ID NO: 56.

In some embodiments, the ratios relating to the mass of the first polynucleotide : second polynucleotide : third polynucleotide is: 1-4.5 : 1-3.5 : 1.

In some embodiments, the ratios relating to the mass of the first polynucleotide : second polynucleotide : third polynucleotide is: 2-3.5 : 1.5-2.5 : 1.

In some embodiments, the ratios relating to the mass of the first polynucleotide : second polynucleotide : third polynucleotide is: 2.8 : 1.9 : 1.

Due to the redundancy of the genetic code, variations in nucleic acid sequences are possible that encode for the same polypeptide. These sequences are encompassed by the present invention. Therefore, multiple polynucleotides are envisaged, each with a different nucleic acid sequence but which encodes a polypeptide according to the invention or a further polypeptide as described herein. It is known in the art how to design and produce such nucleic acid sequences.

In some embodiments, the nucleic acid sequence of the polynucleotide is codon optimised for production in the host cell of choice.

In some embodiments, the nucleic acid sequence of the polynucleotide is operably linked to a sequence such as a control sequence, e.g., a promoter sequence, an enhancer sequence or regulatory sequence, which controls transcription and/or translation. The polynucleotide may be in the form of an expression cassette.

The polynucleotide may be suitable for expression in prokaryotic cells or in eukaryotic cells, such as mammalian cells.

Any promoter may be used in the polynucleotide, such as a strong promoter that is functional in prokaryotic cells or in eukaryotic cells. Suitable promoters will be known in the art. The promoter may be a constitutive promoter. The promoter may be a tissue specific promoter.

### VECTOR

The present invention provides a vector comprising the polynucleotide according to the invention.

In some embodiments, the vector comprises a polynucleotide comprising a nucleic acid sequence which encodes the bispecific binding molecule according to the invention.

The present invention provides a vector comprising one or more polynucleotide(s) according to the invention.

In some embodiments, the vector comprises one or more polynucleotide(s) comprising one or more nucleic acid sequence(s) which encodes the bispecific binding molecule according to the invention.

The present invention provides a vector comprising the plurality of polynucleotides according to the invention.

In some embodiments, the vector comprises a plurality of polynucleotides comprising a plurality of nucleic acid sequences which together encodes the bispecific binding molecule according to the invention.

The vector may be used to introduce a polynucleotide or polynucleotides according to the invention into a cell so that the cell expresses and/or produces the bispecific binding molecule according to the invention.

As used herein, the term "vector" may be considered interchangeable with the term "expression vector" and "expression construct". The vector may be any vector that is suitable for introducing and/or expressing a nucleic acid sequence in a cell. The vector may comprise regulatory sequences, enhancer sequences and/or promoter sequences that promote expression of a nucleic acid sequence in a cell.

The vector according to the invention may be any agent capable of delivering a polynucleotide according to the invention to a cell.

The vector according to the invention may be any agent capable of expressing a polynucleotide or polynucleotides according to the invention in a cell.

Examples of suitable vectors include but are not limited to plasmids, cosmids, phages, viruses or artificial chromosomes.

In some embodiments, the vector is a plasmid or a viral vector. In some embodiments, the vector is a retroviral vector or a lentiviral vector.

The vector may be capable of transfecting or transducing a cell.

### COMPOSITION

The present invention provides a composition comprising the bispecific binding molecule according to the invention.

The present invention provides a composition comprising the polynucleotide according to the invention.

The present invention provides a composition comprising the polynucleotides according to the invention.

In some embodiments, the composition comprises the plurality of polynucleotides as described herein.

The present invention provides a composition comprising the vector according to the invention.

### PHARMACEUTICAL COMPOSITION

The present invention provides a pharmaceutical composition comprising the bispecific binding molecule according to the invention; together with a pharmaceutically acceptable carrier, diluent or excipient.

The present invention also provides a pharmaceutical composition comprising the polynucleotide according to the invention; together with a pharmaceutically acceptable carrier, diluent or excipient.

The present invention also provides a pharmaceutical composition comprising the polynucleotides according to the invention; together with a pharmaceutically acceptable carrier, diluent or excipient.

The present invention also provides a pharmaceutical composition comprising the vector according to the invention; together with a pharmaceutically acceptable carrier, diluent or excipient.

The present invention also provides a pharmaceutical composition comprising the composition according to the invention; together with a pharmaceutically acceptable carrier, diluent or excipient.

In some embodiments, the pharmaceutical composition described herein further comprises one or more selected from this list consisting of: a pharmaceutically acceptable adjuvant, salt, active polypeptide, buffer, compound, component and active agent.

In some embodiments, the pharmaceutical composition further comprises a vesicle, a nanoparticle, a lipid nanoparticle (LNP), a liposome or a polymeric mixture.

It will be understood that the vesicle, nanoparticle, LNP, liposome or polymeric mixture may enable delivery of the bispecific binding molecule according to the invention, the polynucleotide according to the invention, the polynucleotide(s) according to the invention, the vector according to the invention, or the composition according to the invention, to a cell.

In some embodiments, the pharmaceutical composition further comprises a vesicle. In some embodiments, the bispecific binding molecule, the polynucleotide, the polynucleotides, the vector, or the composition according to the invention is encapsulated within the vesicle.

In some embodiments, the pharmaceutical composition further comprises a nanoparticle. In some embodiments, the bispecific binding molecule, the polynucleotide, the polynucleotides, the vector, or the composition according to the invention is encapsulated within the nanoparticle.

In some embodiments, the pharmaceutical composition further comprises a lipid nanoparticle (LNP). In some embodiments, the bispecific binding molecule, the polynucleotide, the polynucleotides, the vector, or the composition according to the invention is encapsulated within the LNP.

In some embodiments, the pharmaceutical composition further comprises a liposome. In some embodiments, the bispecific binding molecule, the polynucleotide, the polynucleotides, the vector, or the composition according to the invention is encapsulated within the liposome.

In some embodiments, the pharmaceutical composition further comprises a polymeric mixture. In some embodiments, the bispecific binding molecule, the polynucleotide, the polynucleotides, the vector, or the composition according to the invention is encapsulated within the polymeric mixture.

Compositions typically should be sterile and stable under the conditions of manufacture and storage. The composition according to the invention may be produced using current good manufacturing practices (CGMP).

In some embodiments, the pharmaceutical composition is sterile.

In some embodiments, the pharmaceutical composition is sterile in aqueous buffer suitable for intravenous (IV) administration.

In some embodiments, the pharmaceutical composition may comprise an aqueous diluent or solvent. In some embodiments, the aqueous diluent or solvent may be a phosphate buffered saline solution, such as a sterile phosphate buffered saline solution.

The term "carrier", as used herein, may refer to a diluent, adjuvant, excipient, or vehicle. Such carriers can be sterile liquids, such as saline solutions in water and oils, including those of petroleum, animal, vegetable or synthetic origin, such as peanut oil, soybean oil, mineral oil, sesame oil. A sterile saline solution is a preferred carrier.

The term "nanoparticle" relates to a nano-sized particle, wherein all three external dimensions of the particle are in the nanoscale, i.e., at least about 1 nm and below about 1,000 nm. Preferably, the size of a particle is its diameter.

Saline solutions and aqueous dextrose and glycerol solutions can also be employed as liquid carriers. Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

The composition, if desired, can also contain minor amounts of wetting or emulsifying agents, or pH buffering agents. The composition of the invention can be formulated as neutral or salt forms. Salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

In some embodiments, the salt comprises a metal cation, such as a sodium salt or a potassium salt.

The pharmaceutical composition may be formulated to be suitable for administration to a patient in order to prevent and/or treat disease. Pharmaceutical compositions can be formulated for administration by different routes, for example, for oral, parenteral, topical, inhalative, intravenous, intramuscular, rectal, sublingual, transdermal, subcutaneous, intratumoral application routes, according to their chemical and physical properties.

In some embodiments, the pharmaceutical composition is in a form suitable for intravenous infusion. In some embodiments, the pharmaceutical composition is administered intravenously.

In some embodiments, the pharmaceutical composition is formulated for parenteral administration.

In some embodiments, the pharmaceutical composition is formulated for intravenous or intraarterial administration.

### DISEASE

The present invention provides the bispecific binding molecule according to the invention, the polynucleotide according to the invention, the polynucleotides according to the invention, the vector according to the invention, the composition according to the invention, or the pharmaceutical composition according to the invention, for use in treating and/or preventing a disease.

The present invention provides the bispecific binding molecule according to the invention for use in treating and/or preventing a disease.

The present invention provides the polynucleotide according to the invention for use in treating and/or preventing a disease.

The present invention provides the polynucleotides according to the invention for use in treating and/or preventing a disease.

The present invention provides the vector according to the invention for use in treating and/or preventing a disease.

The present invention provides the composition according to the invention for use in treating and/or preventing a disease.

The present invention provides the pharmaceutical composition according to the invention for use in treating and/or preventing a disease.

In some embodiments, the use is for treating a disease. In some embodiments, the use is for preventing a disease.

The present invention provides a method for treating and/or preventing a disease which comprises the step of administering the bispecific binding molecule according to the invention, the polynucleotide according to the invention, the polynucleotides according to the invention, the vector according to the invention, the composition according to the invention, or the pharmaceutical composition according to the invention, to a subject.

In some embodiments, the method is for treating a disease. In some embodiments, the method is for preventing a disease.

The present invention provides a use of the bispecific binding molecule according to the invention, the polynucleotide according to the invention, the polynucleotides according to the invention, the vector according to the invention, the composition according to the invention, or the pharmaceutical composition according to the invention, in the manufacture of a medicament for treating and/or preventing a disease.

In some embodiments, the medicament is for treating a disease. In some embodiments, the medicament is for preventing a disease.

In some embodiments, the disease is cancer.

In some embodiments, the disease is a tumour.

In some embodiments, the disease is associated with inflammation.

In some embodiments, the cancer is a tumour or cancer expressing PSMA.

In some embodiments, the tumour expresses PSMA.

In some embodiments, the expression of PSMA is increased compared to the expression of PSMA by a non-cancerous tissue. It will be understood that non-cancerous tissue typically has no detectable expression of PSMA or low expression compared to cancerous tissue.

In some embodiments, the cancer is prostate cancer.

In some embodiments, the cancer is castration resistant prostate cancer.

It will be understood that the terms "tumor" and "tumours" can be used interchangeably.

In some embodiments, the bispecific binding molecule according to the invention, the polynucleotide according to the invention, the polynucleotides according to the invention, the vector according to the invention, the composition according to the invention, or the pharmaceutical composition according to the invention, is administered to a subject systemically.

In some embodiments, the bispecific binding molecule according to the invention, the polynucleotide according to the invention, the polynucleotides according to the invention, the vector according to the invention, the composition according to the invention, or the pharmaceutical composition according to the invention, is administered intravenously (IV) to a subject.

The term "disease" and "disorder" are used interchangeably herein, referring to an abnormal condition, especially an abnormal medical condition such as an illness or injury, wherein a cell, a tissue, an organ, or an individual is not able to efficiently fulfil its function anymore. Typically, but not necessarily, a disease is associated with specific symptoms or signs indicating the presence of such disease. The presence of such symptoms or signs may thus, be indicative for a cell, a tissue, an organ, or an individual suffering from a disease. An alteration of these symptoms or signs may be indicative for the progression of such a disease.

A progression of a disease is typically characterised by an increase or decrease of such symptoms or signs which may indicate a "worsening" or "bettering" of the disease. The "worsening" of a disease is characterised by a decreasing ability of a cell, tissue, organ or individual/patient to fulfil its function efficiently, whereas the "bettering" of a disease is typically characterised by an increase in the ability of a cell, tissue, an organ or an individual/patient to fulfil its function efficiently.

The terms "treat", "treatment" and "treating" refers to lessening, reducing or improving at least one symptom associated with an existing disease or condition and/or to slow down, reduce or block the progression of the disease or condition and/or to delay or prevent the onset of symptoms (such as further symptoms) of the disease or condition.

The terms "prevent", "prevention" and "preventing" refers to preventing the onset of symptoms of a disease or condition, and as such encompasses prophylactic treatment.

The medical uses and methods of treatment described herein may be used in combination with additional treatments and/or medicaments.

### SUBJECT

In some embodiments, the subject is a mammal.

In some embodiments, the subject is a human.

In some embodiments, the subject is a non-human mammal, including for example, a dog, a cat, a horse, a cow, a sheep or a pig.

As used herein, the terms "patient" and "subject" may be used interchangeably.

In some embodiments, the subject is a patient. In some embodiments, the subject is a human patient.

In some embodiments, the subject is a human male. In some embodiments, the subject is a human female.

### IDENTITY

The terms "identity" and "% sequence identity" as used herein, may refer to the proportion of amino acids (expressed in percent) of an amino acid sequence in a peptide or protein, which across the amino acid sequence, are identical to a reference sequence.

The percentage of identity is thus calculated by counting the number of aligned amino acids that are identical (a Match) between two sequences (in the amino acids sequence of the peptide or protein of the invention and in the reference sequence), dividing that number by the total number of amino acids in the aligned region and multiplying by 100.

Therefore, Percentage of Identity = (Matches divided by Length of the aligned region) multiplied by 100.

Insertions and deletions are not allowed in the calculation the percentage of identity of an amino acid sequence.

Any method of calculating percentage identity is permitted within the scope of the invention.

### GENERAL TERMS AND DEFINITIONS

This disclosure is not limited by the exemplary methods and materials disclosed herein, and any methods and materials similar or equivalent to those described herein can be used in the practice or testing of embodiments of this disclosure.

The term "polypeptide" is used in the conventional sense to mean a series of amino acids, typically L-amino acids, connected one to the other, typically by peptide bonds between the α-amino and carboxyl groups of adjacent amino acids.

The term "polypeptide" is used interchangeably with the terms "amino acid sequence", "peptide" and/or "protein".

The term "residues" is used to refer to amino acids in an amino acid sequence.

Numeric ranges are inclusive of the numbers defining the range.

Unless otherwise indicated, any nucleic acid sequences are written left to right in 5' to 3' orientation; amino acid sequences are written left to right in amino to carboxy orientation, respectively.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of' also include the terms "consisting of' or "consisting essentially of".

As used herein, the term "variant" is synonymous with the term "mutant" and refers to an amino acid sequence or a nucleic acid sequence that differs in comparison to the corresponding wild-type sequence. The term "wild-type" is used to mean a protein comprising an amino acid sequence or a polynucleotide comprising an amino acid sequence, which is identical to the native protein or native polynucleotide (e.g. gene) respectively. The variant may have an equivalent function to the amino acid sequences or nucleic acid sequences described herein, but may include one or more amino acid or nucleic acid (respectively) substitutions, insertions or deletions. Amino acid substitutions, insertions and/or deletions may be considered as mutations. Nucleic acid substitutions, insertions and/or deletions may be considered as mutations.

The publications discussed herein are provided solely for their disclosure prior to the filing date of the present application. Nothing herein is to be construed as an admission that such publications constitute prior art to the claims appended hereto.

### SEQUENCES

**Table 1 - List of sequences**

| SEQ ID NO: | Name | Antibody | Sequence AA/NT |
|---|---|---|---|
| 1 | HCDR1 (Kabat) | CD3 (all) | NNYYMC |
| 2 | HCDR2 (Kabat) | CD3 (all) | CIYTNNIGSTWYASWAKG |
| 3 | HCDR3 (Kabat) | CD3 (all) | GIYSDSSDFIGNL |
| 4 | LCDR1 (Kabat) | CD3 (all) | QASQTIYNYLA |
| 5 | LCDR2 (Kabat) | CD3 (all) | KASTLAS |
| 6 | LCDR3 (Kabat) | CD3 (all) | QQYNTIINVDRT |
| 7 | HCDR1 (IMGT) | CD3 (all) | GFSFSNNYY |
| 8 | HCDR2 (IMGT) | CD3 (all) | IYTNNIGST |
| 9 | HCDR3 (IMGT) | CD3 (all) | ARGIYSDSSDFIGNL |
| 10 | LCDR1 (IMGT) | CD3 (all) | QTIYNY |
| 11 | LCDR2 (IMGT) | CD3 (all) | KAS |
| 12 | LCDR3 (IMGT) | CD3 (all) | QQYNTIINVDRT |
| 13 | CD3-VH CLONE 1 | CD3#1 | EVQLVESGGGLVKPGGSLGLSCTASGFSFSNNYY MCWVRRAPGKGLEWVGCIYTNNIGSTWYASWAK GRFTISRDDSKNTVYLQMNSLKTEDTAVYYCARGI YSDSSDFIGNLWGQGTLVTVSS |
| 14 | CD3-VH CLONE 2 | CD3#2 | QVQLVQSGGGLVQPGGPLRLSCTASGFSFSNNYY MCWVRQAPGKGLEWVSCIYTNNIGSTWYASWAK GRFTISRDNSKNTLYLQMNSLRAEDTAVYYCARGI YSDSSDFIGNLWGQGTLVTVSS |
| 15 | CD3-VH CLONE 3 | CD3#3 | EVQLVESGGGLVKPGGSLGLSCTASGFSFSNNYY MCWVRRAPGKGLEWVGCIYTNNIGSTWYASWAK GRFTISRDDSKNTVYLQMNSLKTEDTAVYYCARGI YSDSSDFIGNLWGQGTLVTVSS |
| 16 | CD3-VH consensus | CD3 (all) | XVQLVXSGGGLVXPGGXLXLSCTASGFSFSNNYY MCWVRXAPGKGLEWVXCIYTNNIGSTWYASWAK GRFTISRDXSKNTXYLQMNSLXXEDTAVYYCARGI YSDSSDFIGNLWGQGTLVTVSS |
| 17 | CD3-VL CLONE 1 | CD3#1 | EIVMTQSPATLSVSPGEKATLSCQASQTIYNYLAWY QQKPGQPPRLLIYKASTLASGIPARFSGSGSGTEF TLTISNLQSEDFAVYYCQQYNTIINVDRTFGGGTKV EIK |
| 18 | CD3-VL CLONE 2 | CD3#2 | AIQMTQSPSSLSASVGDRVTITCQASQTIYNYLAW YQQKPGKVPELLIYKASTLASGVPSRFMGTGFGTD FTLTIDSLQPEDAATYYCQQYNTIINVDRTFGGGTK VEIK |
| 19 | CD3-VL CLONE 3 | CD3#3 | DIQMTQSPSSLSASVGDRVTITCQASQTIYNYLAW YQQKPGKQPKLLIYKASTLASGVPSRFKGSGSGTD FTLTISSLQPEDVATYYCQQYNTIINVDRTFGGGTE VVVK |
| 20 | CD3-VL consensus | CD3 (all) | XIXMTQSPXXLSXSXGXXXTXXCQASQTIYNYLAW YQQKPGXXPXLLIYKASTLASGXPXRFXGXGXGTX FTLTIXXLQXEDXAXYYCQQYNTIINVDRTFGGGTX VXXK |
| 21 | CD3-Fab heavy chain CLONE 1 | CD3#1 | EVQLVESGGGLVKPGGSLGLSCTASGFSFSNNYY MCWVRRAPGKGLEWVGCIYTNNIGSTWYASWAK GRFTISRDDSKNTVYLQMNSLKTEDTAVYYCARGI YSDSSDFIGNLWGQGTLVTVSSASTKGPSVFPLAP SSKSTSGGTAALGCLVKDYFPEPVTVSWNSGALTS GVHTFPAVLQSSGLYSLSSVVTVPSSSLGTQTYICN VNHKPSNTKVDKKVEPKSC |
| 22 | CD3-Fab heavy chain CLONE 2 | CD3#2 | QVQLVQSGGGLVQPGGPLRLSCTASGFSFSNNYY MCWVRQAPGKGLEWVSCIYTNNIGSTWYASWAK GRFTISRDNSKNTLYLQMNSLRAEDTAVYYCARGI |
| | | | YSDSSDFIGNLWGQGTLVTVSSASTKGPSVFPLAP SSKSTSGGTAALGCLVKDYFPEPVTVSWNSGALTS GVHTFPAVLQSSGLYSLSSVVTVPSSSLGTQTYICN VNHKPSNTKVDKKVEPKSC |
| 23 | CD3-Fab heavy chain CLONE 3 | CD3#3 | EVQLVESGGGLVKPGGSLGLSCTASGFSFSNNYY MCWVRRAPGKGLEWVGCIYTNNIGSTWYASWAK GRFTISRDDSKNTVYLQMNSLKTEDTAVYYCARGI YSDSSDFIGNLWGQGTLVTVSSASTKGPSVFPLAP SSKSTSGGTAALGCLVKDYFPEPVTVSWNSGALTS GVHTFPAVLQSSGLYSLSSVVTVPSSSLGTQTYICN VNHKPSNTKVDKKVEPKSC |
| 24 | CD3-Fab heavy chain (consensus) | CD3 (all) | XVQLVXSGGGLVXPGGXLXLSCTASGFSFSNNYY MCWVRXAPGKGLEWVXCIYTNNIGSTWYASWAK GRFTISRDXSKNTXYLQMNSLXXEDTAVYYCARGI YSDSSDFIGNLWGQGTLVTVSSASTKGPSVFPLAP SSKSTSGGTAALGCLVKDYFPEPVTVSWNSGALTS GVHTFPAVLQSSGLYSLSSVVTVPSSSLGTQTYICN VNHKPSNTKVDKKVEPKSC |
| 25 | CD3-Fab light chain CLONE 1 | CD3#1 | EIVMTQSPATLSVSPGEKATLSCQASQTIYNYLAWY QQKPGQPPRLLIYKASTLASGIPARFSGSGSGTEF TLTISNLQSEDFAVYYCQQYNTIINVDRTFGGGTKV EIKRTVAAPSVFIFPPSDEQLKSGTASVVCLLNNFY PREAKVQWKVDNALQSGNSQESVTEQDSKDSTY SLSSTLTLSKADYEKHKVYACEVTHQGLSSPVTKS FNRGEC |
| 26 | CD3-Fab light chain CLONE 2 | CD3#2 | AIQMTQSPSSLSASVGDRVTITCQASQTIYNYLAW YQQKPGKVPELLIYKASTLASGVPSRFMGTGFGTD FTLTIDSLQPEDAATYYCQQYNTIINVDRTFGGGTK VEIKRTVAAPSVFIFPPSDEQLKSGTASVVCLLNNF YPREAKVQWKVDNALQSGNSQESVTEQDSKDST YSLSSTLTLSKADYEKHKVYACEVTHQGLSSPVTK SFNRGEC |
| 27 | CD3-Fab light chain CLONE 3 | CD3#3 | DIQMTQSPSSLSASVGDRVTITCQASQTIYNYLAW YQQKPGKQPKLLIYKASTLASGVPSRFKGSGSGTD FTLTISSLQPEDVATYYCQQYNTIINVDRTFGGGTE VVVKRTVAAPSVFIFPPSDEQLKSGTASVVCLLNNF YPREAKVQWKVDNALQSGNSQESVTEQDSKDST YSLSSTLTLSKADYEKHKVYACEVTHQGLSSPVTK SFNRGEC |
| 28 | CD3-Fab light chain (consensus) | CD3 (all) | XIXMTQSPXXLSXSXGXXXTXXCQASQTIYNYLAW YQQKPGXXPXLLIYKASTLASGXPXRFXGXGXGTX FTLTIXXLQXEDXAXYYCQQYNTIINVDRTFGGGTX VXXKRTVAAPSVFIFPPSDEQLKSGTASVVCLLNNF YPREAKVQWKVDNALQSGNSQESVTEQDSKDST YSLSSTLTLSKADYEKHKVYACEVTHQGLSSPVTK SFNRGEC |
| 29 | HCDR1 (Kabat) | PSMA VHH #1 | KYAMA |
| 30 | HCDR2 (Kabat) | PSMA VHH #1 | TITTAGDSTNYPDSVKG |
| 31 | HCDR3 (Kabat) | PSMA VHH #1 | YDYFGDNEIDF |
| 32 | HCDR1 (IMGT) | PSMA VHH #1 | GFGFSKYA |
| 33 | HCDR2 (IMGT) | PSMA VHH #1 | ITTAGDST |
| 34 | HCDR3 (IMGT) | PSMA VHH #1 | NAYDYFGDNEIDF |
| 35 | VHH | PSMA VHH #1 | QLQLVESGGGLVQPGGSLRLSCKASGFGFSKYAM AWHRQAPGNARELVATITTAGDSTNYPDSVKGRFT ASRDNAKSTAYLQMNSLKPEDTAVYLCNAYDYFGD NEIDFWGQGTQVTVSS |
| 36 | HCDR1 (Kabat) | PSMA VHH #2 | THAMA |
| 37 | HCDR2 (Kabat) | PSMA VHH #2 | LITSTDGTTKYADSVKG |
| 38 | HCDR3 (Kabat) | PSMA VHH #2 | YNYFGDYEDDY |
| 39 | HCDR1 (IMGT) | PSMA VHH #2 | GFTFSTHA |
| 40 | HCDR2 (IMGT) | PSMA VHH #2 | ITSTDGTT |
| 41 | HCDR3 (IMGT) | PSMA VHH #2 | NAYNYFGDYEDDY |
| 42 | VHH | PSMA VHH #2 | QLQLVESGGGLVQPGGPLRLSCAASGFTFSTHAM AWYRQAPGNERELVALITSTDGTTKYADSVKGRFTI SRDNAGNTVYLQMNSLKPEDTAVYYCNAYNYFGD YEDDYWGQGTLVTVSS |
| 43 | Poly(A)-tail | | AAAAAAAAAAAAAAAAAAAAAAAAAAAAAAAGCAU AUGACUAAAAAAAAAAAAAAAAAAAAAAAAAAAAA AAAAAAAAAAAAAAAAAAAAAAAAAAAAAAAAAAAA AAAAAAA |
| 44 | 5'-UTR | | AUUCUUCUGGUCCCCACAGACUCAGAGAGAAC CC |
| 45 | 3'-UTR | | CUGGUACUGCAUGCACGCAAUGCUAGCUGCCC CUUUCCCGUCCUGGGUACCCCGAGUCUCCCCC GACCUCGGGUCCCAGGUAUGCUCCCACCUCCA CCUGCCCCACUCACCACCUCUGCUAGUUCCAG ACACCUCCCAAGCACGCAGCAAUGCAGCUCAAA ACGCUUAGCCUAGCCACACCCCCACGGGAAAC AGCAGUGAUUAACCUUUAGCAAUAAACGAAAGU UUAACUAAGCUAUACUAACCCCAGGGUUGGUC AAUUUCGUGCCAGCCACACC |
| 46 | 5' Cap - 5'UTR - PSMA-binding VHH #1 domain - CD3-VH #1 domain - CD3-CH1 domain - CH2 domain - CH3 domain - 3'UTR - Poly(A) | PSMA VHH #1 x CD3 #1 | AGAAUAAACUAGUAUUCUUCUGGUCCCCACAG ACUCAGAGAGAACCCGCCACCAUGGAUUGGAU CUGGCGUAUCCUGUUCCUGGUGGGGGCCGCA ACUGGUGCUCAUAGUCAGCUUCAGCUCGUUGA GAGCGGCGGGGCCUUGUGCAGCCCGGUGGC UCUCUGCGCCUGUCCUGCAAGGCGUCCGGUU UCGGGUUCUCUAAGUAUGCCAUGGCGUGGCAC CGCCAGGCACCCGGCAACGCGUGAGCUGG UGGCCACCAUCACCACGGCGGGCGACAGCACA AAUUACCCCGAUUCCGUGAAAGGUCGGUUCAC CGCCAGUCGCGACAACGCUAAGAGUACCGCUU AUUUGCAGAUGAAUUCACUGAAGCCCGAGGAC ACCGCAGUCUACCUGUGUAAUGCUUACGACUA CUUUGGCGAUAACGAGAUCGAUUUCUGGGGCC AGGGCACCCAAGUUACCGUGUCCAGUGGGGGA GGGGGCUCCGGAGGCGGAGGCUCCGAAGUGC AGCUGGUCGAGUCCGGCGGAGGUCUUGUCAA GCCUGGCGGUAGCCUGGGAUUGUCAUGUACC GCAAGUGGUUUCAGCUUCUCUAAUAACUAUUA CAUGUGCUGGGUCCGUCGCGCCCCAGGCAAAG GACUGGAGUGGGUGGGGUGCAUCUAUACAAAU AACAUCGGUAGCACCUGGUACGCUAGCUGGGC UAAAGGCCGGUUCACCAUUAGUAGAGACGAUU CCAAGAACACCGUCUAUCUGCAGAUGAACAGU UUGAAGACCGAGGAUACCGCUGUGUACUAUUG UGCCCGUGGCAUUUAUUCCGAUUCCAGUGAUU UCAUCGGGAACCUGUGGGGCCAAGGUACCCUG GUGACAGUGUCCUCUGCUUCAACCAAAGGCCC UAGCGUUUUUCCACUUGCCCCUAGCUCAAAGU CUACCAGUGGAGGGACCGCGGCCCUUGGCUG UCUCGUGAAGGAUUACUUUCCUGAACCUGUGA CAGUCAGCUGGAAUAGCGGCGCUCUUACCAGC GGGGUCCAUACUUUUCCUGCCGUUCUUCAGUC CAGCGGGCUGUAUUCCCUCAGUUCUGUUGUGA CUGUGCCAUCAUCCAGCCUGGGUACCCAGACA UAUAUCUGCAAUGUGAACCACAAGCCGUCCAA UACCAAGGUGGACAAAAAGGUGGAGCCAAAAU CUUGUGACAAGACUCACACCUGCCCUCCGUGU CCAGCCCCGGAGUCCACCCGCGGCCCAUCCGU CUUCCUGUUUCCCCCAAAGCCAAAAGACACUC |
| | | | UGAUGAUUAGCCGCACCCCGGAGGUGACGUGC GUGGUUGUGGAUGUGAGUCAUGAAGAUCCCGA AGUGAAAUUUAACUGGUACGUGGACGGCGUUG AGGUGCACAAUGCCAAGACCAAGCCGCGUGAG GAGCAGUACAACUCAACUUAUCGCGUCGUGUC AGUCCUGACAGUGCUGCACCAGGACUGGCUGA AUGGCAAGGAAUACAAGUGCAAGGUGAGUAAC AAGGCGCUGCCGGCUCCUAUCGAGAAGACCAU UAGUAAGGCCAAGGGCCAGCCACGCGAGCCGC AGGUGUACACCCUGCCACCGUGUCGCGAUGAA UUGACUAAGAACCAGGUUAGCCUGUGGUGCCU GGUGAAAGGCUUCUAUCCUUCUGACAUCGCUG UGGAAUGGGAGUCUAACGGCCAGCCUGAAAAC AAUUAUAAGACCACACCUCCCGUGCUCGAUUC AGACGGCUCUUUUUUCCUGUACAGCAAACUCA CUGUCGACAAGAGCCGCUGGCAACAGGGGAAC GUGUUUUCCUGCUCUGUUAUGCACGAGGCCCU GCACAAUCACUACACUCAGAAAUCUCUCUCCCU CUCUCCUGGAAAGUGAUAAGGAUCCGAUCUGG UACUGCAUGCACGCAAUGCUAGCUGCCCCUUU CCCGUCCUGGGUACCCCGAGUCUCCCCCGACC UCGGGUCCCAGGUAUGCUCCCACCUCCACCUG CCCCACUCACCACCUCUGCUAGUUCCAGACAC CUCCCAAGCACGCAGCAAUGCAGCUCAAAACG CUUAGCCUAGCCACACCCCCACGGGAAACAGC AGUGAUUAACCUUUAGCAAUAAACGAAAGUUUA ACUAAGCUAUACUAACCCCAGGGUUGGUCAAU UUCGUGCCAGCCACACCCUCGAGCUAGCAAAA AAAAAAAAAAAAAAAAAAAAAAAAAAGCAUAUGA CUAAAAAAAAAAAAAAAAAAAAAAAAAAAAAAAA AAAAAAAAAAAAAAAAAAAAAAAAAAAAAAAAAA AA |
| 47 | 5' Cap - 5'UTR - PSMA-binding VHH #1 domain - CD3-VH #2 domain - CD3-CH1 domain - CH2 domain - CH3 domain - 3'UTR - Poly(A) | PSMA VHH #1 x CD3 #2 | AGAAUAAACUAGUAUUCUUCUGGUCCCCACAG ACUCAGAGAGAACCCGCCACCAUGGAUUGGAU UUGGCGUAUCCUGUUUCUGGUGGCGCUGCC ACCGGAGCCAUUCCCAGCUCCAGCUGGUUGA AUCCGGUGGCGGCUGGUGCAGCCUGGUGGC UCCCUGCGCCUUAGCUGCAAGGCCUCUGGCUU CGGAUUCAGUAAGUACGCCAUGGCCUGGCACC GUCAGGCCCCCGGAAACGCCCGCGAACUGGUU GCCACCAUCACCACAGCCGGCGACUCUACCAA CUACCCAGACAGCGUGAAAGGGCGGUUUACCG CCAGCAGGGACAACGCCAAAUCUACCGCCUAU CUGCAGAUGAAUUCUCUGAAACCCGAGGAUAC AGCCGUAUACCUGUGUAACGCCUAUGAUUACU UUGGCGAUAAUGAAAUCGACUUCUGGGGGCAA GGGACCCAGGUGACCGUCUCUAGCGGCGGUG GCGGUUCCGGAGGUGGCGGAAGCCAGGUCCA GCUGGUGCAGUCCGGGGGCGGUUUGGUGCAG CCCGGUGGGCCGCUGAGAUUGAGCUGCACAG CAUCCGGCUUUUCAUUUAGCAAUAACUACUAUA UGUGCUGGGUGCGCCAAGCUCCUGGUAAGGG UCUGGAGUGGGUUAGCUGCAUCUACACAAACA AUAUCGGCUCAACUUGGUACGCAUCUUGGGCU AAGGGACGCUUCACCAUCAGCCGCGAUAAUUC UAAAAACACCCUGUACCUUCAGAUGAACUCUCU GCGCGCUGAAGAUACCGCCGUGUAUUACUGCG CCCGUGGGAUUUACUCCGACAGUUCAGACUUC AUCGGGAACCUUUGGGGCCAAGGUACCCUGGU GACAGUGUCUAGUGCUUCUACCAAAGGCCCAA GCGUUUUCCCUCUCGCCCCCAGUUCCAAGAGC |
| | | | ACCUCUGGUGGCACAGCAGCCUUGGGCUGUCU GGUUAAGGACUACUUCCCAGAACCCGUCACAG UGUCUUGGAACAGCGGCGCUCUGACCUCUGGA GUGCACACCUUCCCAGCUGUGCUGCAGAGCAG UGGUCUCUACUCCUUGUCCAGUGUGGUAACCG UCCCGAGUUCUUCAUUGGGCACCCAGACCUAU AUUUGUAAUGUUAACCACAAACCUUCAAAUACC AAGGUGGACAAAAAGGUGGAGCCGAAGUCUUG UGACAAAACCCAUACUUGCCCUCCAUGCCCAG CUCCCGAGUCUACCCGUGGCCCAUCCGUUUUC CUGUUUCCUCCGAAGCCAAAGGAUACUCUGAU GAUCAGCAGAACCCCUGAGGUAACCUGUGUCG UGGUAGACGUGAGCCAUGAAGACCCUGAGGUC AAAUUCAACUGGUAUGUAGAUGGAGUUGAGGU UCACAACGCCAAGACCAAGCCGCGGGAGGAGC AGUAUAACAGCACAUACCGCGUGGUAUCCGUC CUGACUGUGCUGCAUCAGGACUGGCUGAAUGG GAAGGAAUACAAGUGUAAGGUGUCUAACAAGG CGCUGCCUGCCCCCAUCGAGAAGACUAUCAGC AAGGCCAAGGGCCAGCCUCGUGAGCCACAAGU GUACACCCUGCCCCCUUGCCGCGACGAGCUGA CAAAGAAUCAGGUUAGCCUGUGGUGCCUCGUG AAAGGCUUCUACCCGUCCGAUAUUGCCGUGGA AUGGGAAUCCAACGGCCAGCCCGAAAAUAACU ACAAGACCACACCUCCAGUGCUCGACAGCGAC GGUAGCUUUUUCCUCUACAGUAAGCUCACGGU UGACAAGUCCCGGUGGCAACAGGGGAACGUGU UCAGUUGCUCCGUUAUGCAUGAGGCCCUGCAC AACCAUUACACCCAGAAGUCACUGAGCCUCUC CCCCGGGAAAUGAUAAGGAUCCGAUCUGGUAC UGCAUGCACGCAAUGCUAGCUGCCCCUUUCCC GUCCUGGGUACCCCGAGUCUCCCCCGACCUCG GGUCCCAGGUAUGCUCCCACCUCCACCUGCCC CACUCACCACCUCUGCUAGUUCCAGACACCUC CCAAGCACGCAGCAAUGCAGCUCAAAACGCUU AGCCUAGCCACACCCCCACGGGAAACAGCAGU GAUUAACCUUUAGCAAUAAACGAAAGUUUAACU AAGCUAUACUAACCCCAGGGUUGGUCAAUUUC GUGCCAGCCACACCCUCGAGCUAGCAAAAAAA AAAAAAAAAAAAAAAAAAAAAAGCAUAUGACUAA AAAAAAAAAAAAAAAAAAAAAAAAAAAAAAAAAAAA AAAAAAAAAAAAAAAAAAAAAAAAAAAAAAAAAA |
| 48 | 5' Cap - 5'UTR - PSMA-binding VHH #1 domain - CD3-VH #3 domain - CD3-CH1 domain - CH2 domain - CH3 domain - 3'UTR - Poly(A) | PSMA VHH #1 x CD3 #3 | AGAAUAAACUAGUAUUCUUCUGGUCCCCACAG ACUCAGAGAGAACCCGCCACCAUGGAUUGGAU CUGGCGUAUCCUGUUCCUGGUGGGGGCCGCA ACUGGUGCUCAUAGUCAGCUUCAGCUCGUUGA GAGCGGCGGGGGCCUUGUGCAGCCCGGUGGC UCUCUGCGCCUGUCCUGCAAGGCGUCCGGUU UCGGGUUCUCUAAGUAUGCCAUGGCGUGGCAC CGCCAGGCACCCGGCAACGCGCUGAGCUGG UGGCCACCAUCACCACGGCGGGCGACAGCACA AAUUACCCCGAUUCCGUGAAAGGUCGGUUCAC CGCCAGUCGCGACAACGCUAAGAGUACCGCUU AUUUGCAGAUGAAUUCACUGAAGCCCGAGGAC ACCGCAGUCUACCUGUGUAAUGCUUACGACUA CUUUGGCGAUAACGAGAUCGAUUUCUGGGGCC AGGGCACCCAAGUUACCGUGUCCAGUGGGGA GGGGGCUCCGGAGGCGGAGGCUCCGAAGUGC AGCUGGUCGAGUCCGGCGGAGGUCUUGUCAA GCCUGGCGGUAGCCUGGGAUUGUCAUGUACC GCAAGUGGUUUCAGCUUCUCUAAUAACUAUUA |
| | | | CAUGUGCUGGGUCCGUCGCGCCCCAGGCAAAG GACUGGAGUGGGUGGGGUGCAUCUAUACAAAU AACAUCGGUAGCACCUGGUACGCUAGCUGGGC UAAAGGCCGGUUCACCAUUAGUAGAGACGAUU CCAAGAACACCGUCUAUCUGCAGAUGAACAGU UUGAAGACCGAGGAUACCGCUGUGUACUAUUG UGCCCGUGGCAUUUAUUCCGAUUCCAGUGAUU UCAUCGGGAACCUGUGGGCCAAGGUACCCUG GUGACAGUGUCCUCUGCUUCAACCAAAGGCCC UAGCGUUUUUCCACUUGCCCCUAGCUCAAAGU CUACCAGUGGAGGGACCGCGGCCCUUGGCUG UCUCGUGAAGGAUUACUUUCCUGAACCUGUGA CAGUCAGCUGGAAUAGCGGCGCUCUUACCAGC GGGGUCCAUACUUUUCCUGCCGUUCUUCAGUC CAGCGGGCUGUAUUCCCUCAGUUCUGUUGUGA CUGUGCCAUCAUCCAGCCUGGGUACCCAGACA UAUAUCUGCAAUGUGAACCACAAGCCGUCCAA UACCAAGGUGGACAAAAAGGUGGAGCCAAAAU CUUGUGACAAGACUCACACCUGCCCUCCGUGU CCAGCCCCGGAGUCCACCCGCGGCCCAUCCGU CUUCCUGUUCCCCCAAAGCCAAAAGACACUC UGAUGAUUAGCCGCACCCCGGAGGUGACGUGC GUGGUUGUGGAUGUGAGUCAUGAAGAUCCCGA AGUGAAAUUUAACUGGUACGUGGACGGCGUUG AGGUGCACAAUGCCAAGACCAAGCCGCGUGAG GAGCAGUACAACUCAACUUAUCGCGUCGUGUC AGUCCUGACAGUGCUGCACCAGGACUGGCUGA AUGGCAAGGAAUACAAGUGCAAGGUGAGUAAC AAGGCGCUGCCGGCUCCUAUCGAGAAGACCAU UAGUAAGGCCAAGGGCCAGCCACGCGAGCCGC AGGUGUACACCCUGCCACCGUGUCGCGAUGAA UUGACUAAGAACCAGGUUAGCCUGUGGUGCCU GGUGAAAGGCUUCUAUCCUUCUGACAUCGCUG UGGAAUGGGAGUCUAACGGCCAGCCUGAAAAC AAUUAUAAGACCACACCUCCCGUGCUCGAUUC AGACGGCUCUUUUUUCCUGUACAGCAAACUCA CUGUCGACAAGAGCCGCUGGCAACAGGGGAAC GUGUUUUCCUGCUCUGUUAUGCACGAGGCCCU GCACAAUCACUACACUCAGAAAUCUCUCUCCCU CUCUCCUGGAAAGUGAUAAGGAUCCGAUCUGG UACUGCAUGCACGCAAUGCUAGCUGCCCCUUU CCCGUCCUGGGUACCCCGAGUCUCCCCCGACC UCGGGUCCCAGGUAUGCUCCCACCUCCACCUG CCCCACUCACCACCUCUGCUAGUUCCAGACAC CUCCCAAGCACGCAGCAAUGCAGCUCAAAACG CUUAGCCUAGCCACACCCCCACGGGAAACAGC AGUGAUUAACCUUUAGCAAUAAACGAAAGUUUA ACUAAGCUAUACUAACCCCAGGGUUGGUCAAU UUCGUGCCAGCCACACCCUCGAGCUAGCAAAA AAAAAAAAAAAAAAAAAAAAAAAAAAGCAUAUGA CUAAAAAAAAAAAAAAAAAAAAAAAAAAAAAAAAA AAAAAAAAAAAAAAAAAAAAAAAAAAAAAAAAAA AA |
| 49 | 5' Cap - 5'UTR - PSMA-binding VHH #2 domain - CD3-VH #1 domain - CD3-CH1 domain - CH2 domain - | PSMA VHH #2 x CD3 #1 | AGAAUAAACUAGUAUUCUUCUGGUCCCCACAG ACUCAGAGAGAACCCGCCACCAUGGACUGGAU UUGGCGCAUCCUGUUCCUGGUCGGCGCCGCG ACUGGUGCCCACUCCCAGCUGCAACUUGUGGA GUCAGGCGGUGGCCUGGUGCAGCCCGGCGGU CCUCUCCGCCUGUCCUGCGCUGCCUCCGGCU UCACCUUCUCCACCCAUGCGAUGGCCUGGUAC AGACAGGCCCCUGGCAAUGAGCGUGAACUGGU |
| | CH3 domain - 3'UTR - Poly(A) | | CGCCCUGAUCACCAGCACGGACGGCACCACUA AGUACGCAGAUAGUGUGAAGGUCGGUUCACC AUCUCCAGAGACAACGCAGGUAACACCGUGUA CCUGCAGAUGAACUCCCUUAAGCCUGAGGAUA CAGCUGUGUAUUACUGUAAUGCCUAUAACUAC UUCGGUGACUAUGAAGACGAUUAUUGGGGCCA GGGCACACUUGUCACAGUGAGUUCUGGAGGUG GCGGUUCCGGAGGCGGAGGCUCCGAAGUGCA GCUGGUCGAGUCCGGCGGAGGUCUUGUCAAG CCUGGCGGUAGCCUGGGAUUGUCAUGUACCG CAAGUGGUUUCAGCUUCUCUAAUAACUAUUAC AUGUGCUGGGUCCGUCGCGCCCCAGGCAAAG GACUGGAGUGGGUGGGGUGCAUCUAUACAAAU AACAUCGGUAGCACCUGGUACGCUAGCUGGGC UAAAGGCCGGUUCACCAUUAGUAGAGACGAUU CCAAGAACACCGUCUAUCUGCAGAUGAACAGU UUGAAGACCGAGGAUACCGCUGUGUACUAUUG UGCCCGUGGCAUUUAUUCCGAUUCCAGUGAUU UCAUCGGGAACCUGUGGGGCCAAGGUACCCUG GUGACAGUGUCCUCUGCUUCAACCAAAGGCCC UAGCGUUUUUCCACUUGCCCCUAGCUCAAAGU CUACCAGUGGAGGGACCGCGGCCCUUGGCUG UCUCGUGAAGGAUUACUUUCCUGAACCUGUGA CAGUCAGCUGGAAUAGCGGCGCUCUUACCAGC GGGGUCCAUACUUUUCCUGCCGUUCUUCAGUC CAGCGGGCUGUAUUCCCUCAGUUCUGUUGUGA CUGUGCCAUCAUCCAGCCUGGGUACCCAGACA UAUAUCUGCAAUGUGAACCACAAGCCGUCCAA UACCAAGGUGGACAAAAAGGUGGAGCCAAAAU CUUGUGACAAGACUCACACCUGCCCUCCGUGU CCAGCCCCGGAGUCCACCCGCGGCCCAUCCGU CUUCCUGUUUCCCCCAAAGCCAAAAGACACUC UGAUGAUUAGCCGCACCCCGGAGGUGACGUGC GUGGUUGUGGAUGUGAGUCAUGAAGAUCCCGA AGUGAAAUUUAACUGGUACGUGGACGGCGUUG AGGUGCACAAUGCCAAGACCAAGCCGCGUGAG GAGCAGUACAACUCAACUUAUCGCGUCGUGUC AGUCCUGACAGUGCUGCACCAGGACUGGCUGA AUGGCAAGGAAUACAAGUGCAAGGUGAGUAAC AAGGCGCUGCCGGCUCCUAUCGAGAAGACCAU UAGUAAGGCCAAGGGCCAGCCACGCGAGCCGC AGGUGUACACCCUGCCACCGUGUCGCGAUGAA UUGACUAAGAACCAGGUUAGCCUGUGGUGCCU GGUGAAAGGCUUCUAUCCUUCUGACAUCGCUG UGGAAUGGGAGUCUAACGGCCAGCCUGAAAAC AAUUAUAAGACCACACCUCCCGUGCUCGAUUC AGACGGCUCUUUUUUCCUGUACAGCAAACUCA CUGUCGACAAGAGCCGCUGGCAACAGGGGAAC GUGUUUUCCUGCUCUGUUAUGCACGAGGCCCU GCACAAUCACUACACUCAGAAAUCUCUCUCCCU CUCUCCUGGAAAGUGAUAAGGAUCCGAUCUGG UACUGCAUGCACGCAAUGCUAGCUGCCCCUUU CCCGUCCUGGGUACCCCGAGUCUCCCCCGACC UCGGGUCCCAGGUAUGCUCCCACCUCCACCUG CCCCACUCACCACCUCUGCUAGUUCCAGACAC CUCCCAAGCACGCAGCAAUGCAGCUCAAAACG CUUAGCCUAGCCACACCCCCACGGGAAACAGC AGUGAUUAACCUUUAGCAAUAAACGAAAGUUUA ACUAAGCUAUACUAACCCCAGGGUUGGUCAAU UUCGUGCCAGCCACACCCUCGAGCUAGCAAAA AAAAAAAAAAAAAAAAAAAAAAAAAAGCAUAUGA |
| | | | CUAAAAAAAAAAAAAAAAAAAAAAAAAAAAAAAAAAA AAAAAAAAAAAAAAAAAAAAAAAAAAAAAAAAAAAAA AA |
| 50 | 5' Cap - 5'UTR - PSMA-binding VHH #2 domain - CD3-VH #2 domain - CD3-CH1 domain - CH2 domain - CH3 domain - 3'UTR - Poly(A) | PSMA VHH #2 x CD3 #2 | AGAAUAAACUAGUAUUCUUCUGGUCCCCACAG ACUCAGAGAGAACCCGCCACCAUGGACUGGAU UUGGCGCAUCCUGUUCCUGGUCGGCGCCGCG ACUGGUGCCCACUCCCAGCUGCAACUUGUGGA GUCAGGCGGUGGCCUGGUGCAGCCCGGCGGU CCUCUCCGCCUGUCCUGCGCUGCCUCCGGCU UCACCUUCUCCACCCAUGCGAUGGCCUGGUAC AGACAGGCCCCUGGCAAUGAGCGUGAACUGGU CGCCCUGAUCACCAGCACGGACGGCACCACUA AGUACGCAGAUAGUGUGAAGGGUCGGUUCACC AUCUCCAGAGACAACGCAGGUAACACCGUGUA CCUGCAGAUGAACUCCCUUAAGCCUGAGGAUA CAGCUGUGUAUUACUGUAAUGCCUAUAACUAC UUCGGUGACUAUGAAGACGAUUAUUGGGGCCA GGGCACACUUGUCACAGUGAGUUCUGGAGGUG GCGGUUCCGGAGGUGGCGGAAGCCAGGUCCA GCUGGUGCAGUCCGGGGGCGGUUUGGUGCAG CCCGGUGGGCCGCUGAGAUUGAGCUGCACAG CAUCCGGCUUUUCAUUUAGCAAUAACUACUAUA UGUGCUGGGUGCGCCAAGCUCCUGGUAAGGG UCUGGAGUGGGUUAGCUGCAUCUACACAAACA AUAUCGGCUCAACUUGGUACGCAUCUUGGGCU AAGGGACGCUUCACCAUCAGCCGCGAUAAUUC UAAAAACACCCUGUACCUUCAGAUGAACUCUCU GCGCGCUGAAGAUACCGCCGUGUAUUACUGCG CCCGUGGGAUUUACUCCGACAGUUCAGACUUC AUCGGGAACCUUUGGGGCCAAGGUACCCUGGU GACAGUGUCUAGUGCUUCUACCAAAGGCCCAA GCGUUUUCCCUCUCGCCCCCAGUUCCAAGAGC ACCUCUGGUGGCACAGCAGCCUUGGGCUGUCU GGUUAAGGACUACUUCCCAGAACCCGUCACAG UGUCUUGGAACAGCGGCGCUCUGACCUCUGGA GUGCACACCUUCCCAGCUGUGCUGCAGAGCAG UGGUCUCUACUCCUUGUCCAGUGUGGUAACCG UCCCGAGUUCUUCAUUGGGCACCCAGACCUAU AUUUGUAAUGUUAACCACAAACCUUCAAAUACC AAGGUGGACAAAAAGGUGGAGCCGAAGUCUUG UGACAAAACCCAUACUUGCCCUCCAUGCCCAG CUCCCGAGUCUACCCGUGGCCCAUCCGUUUUC CUGUUUCCUCCGAAGCCAAAGGAUACUCUGAU GAUCAGCAGAACCCCUGAGGUAACCUGUGUCG UGGUAGACGUGAGCCAUGAAGACCCUGAGGUC AAAUUCAACUGGUAUGUAGAUGGAGUUGAGGU UCACAACGCCAAGACCAAGCCGCGGGAGGAGC AGUAUAACAGCACAUACCGCGUGGUAUCCGUC CUGACUGUGCUGCAUCAGGACUGGCUGAAUGG GAAGGAAUACAAGUGUAAGGUGUCUAACAAGG CGCUGCCUGCCCCCAUCGAGAAGACUAUCAGC AAGGCCAAGGGCCAGCCUCGUGAGCCACAAGU GUACACCCUGCCCCCUUGCCGCGACGAGCUGA CAAAGAAUCAGGUUAGCCUGUGGUGCCUCGUG AAAGGCUUCUACCCGUCCGAUAUUGCCGUGGA AUGGGAAUCCAACGGCCAGCCCGAAAAUAACU ACAAGACCACACCUCCAGUGCUCGACAGCGAC GGUAGCUUUUUCCUCUACAGUAAGCUCACGGU UGACAAGUCCCGGUGGCAACAGGGGAACGUGU UCAGUUGCUCCGUUAUGCAUGAGGCCCUGCAC AACCAUUACACCCAGAAGUCACUGAGCCUCUC |
| | | | CCCCGGGAAAUGAUAAGGAUCCGAUCUGGUAC UGCAUGCACGCAAUGCUAGCUGCCCCUUUCCC GUCCUGGGUACCCCGAGUCUCCCCCGACCUCG GGUCCCAGGUAUGCUCCCACCUCCACCUGCCC CACUCACCACCUCUGCUAGUUCCAGACACCUC CCAAGCACGCAGCAAUGCAGCUCAAAACGCUU AGCCUAGCCACACCCCCACGGGAAACAGCAGU GAUUAACCUUUAGCAAUAAACGAAAGUUUAACU AAGCUAUACUAACCCCAGGGUUGGUCAAUUUC GUGCCAGCCACACCCUCGAGCUAGCAAAAAAA AAAAAAAAAAAAAAAAAAAAAGCAUAUGACUAA AAAAAAAAAAAAAAAAAAAAAAAAAAAAAAAAA AAAAAAAAAAAAAAAAAAAAAAAAAAAAAAAA |
| 51 | 5' Cap - 5'UTR - PSMA-binding VHH #2 domain - CD3-VH #3 domain - CD3-CH1 domain - CH2 domain - CH3 domain - 3'UTR - Poly(A) | PSMA VHH #2 x CD3 #3 | AGAAUAAACUAGUAUUCUUCUGGUCCCCACAG ACUCAGAGAGAACCCGCCACCAUGGACUGGAU UUGGCGCAUCCUGUUCCUGGUCGGCGCCGCG ACUGGUGCCCACUCCCAGCUGCAACUUGUGGA GUCAGGCGGUGGCCUGGUGCAGCCCGGCGGU CCUCUCCGCCUGUCCUGCGCUGCCUCCGGCU UCACCUUCUCCACCCAUGCGAUGGCCUGGUAC AGACAGGCCCCUGGCAAUGAGCGUGAACUGGU CGCCCUGAUCACCAGCACGGACGGCACCACUA AGUACGCAGAUAGUGUGAAGGGUCGGUUCACC AUCUCCAGAGACAACGCAGGUAACACCGUGUA CCUGCAGAUGAACUCCCUUAAGCCUGAGGAUA CAGCUGUGUAUUACUGUAAUGCCUAUAACUAC UUCGGUGACUAUGAAGACGAUUAUUGGGGCCA GGGCACACUUGUCACAGUGAGUUCUGGAGGUG GCGGUUCCGGAGGCGGAGGCUCCGAAGUGCA GCUGGUCGAGUCCGGCGGAGGUCUUGUCAAG CCUGGCGGUAGCCUGGGAUUGUCAUGUACCG CAAGUGGUUUCAGCUUCUCUAAUAACUAUUAC AUGUGCUGGGUCCGUCGCGCCCCAGGCAAAG GACUGGAGUGGGUGGGGUGCAUCUAUACAAAU AACAUCGGUAGCACCUGGUACGCUAGCUGGGC UAAAGGCCGGUUCACCAUUAGUAGAGACGAUU CCAAGAACACCGUCUAUCUGCAGAUGAACAGU UUGAAGACCGAGGAUACCGCUGUGUACUAUUG UGCCCGUGGCAUUUAUUCCGAUUCCAGUGAUU UCAUCGGGAACCUGUGGGGCCAAGGUACCCUG GUGACAGUGUCCUCUGCUUCAACCAAAGGCCC UAGCGUUUUUCCACUUGCCCCUAGCUCAAAGU CUACCAGUGGAGGGACCGCGGCCCUUGGCUG UCUCGUGAAGGAUUACUUUCCUGAACCUGUGA CAGUCAGCUGGAAUAGCGGCGCUCUUACCAGC GGGGUCCAUACUUUUCCUGCCGUUCUUCAGUC CAGCGGGCUGUAUUCCCUCAGUUCUGUUGUGA CUGUGCCAUCAUCCAGCCUGGGUACCCAGACA UAUAUCUGCAAUGUGAACCACAAGCCGUCCAA UACCAAGGUGGACAAAAAGGUGGAGCCAAAAU CUUGUGACAAGACUCACACCUGCCCUCCGUGU CCAGCCCCGGAGUCCACCCGCGGCCCAUCCGU CUUCCUGUUUCCCCCAAAGCCAAAAGACACUC UGAUGAUUAGCCGCACCCCGGAGGUGACGUGC GUGGUUGUGGAUGUGAGUCAUGAAGAUCCCGA AGUGAAAUUUAACUGGUACGUGGACGGCGUUG AGGUGCACAAUGCCAAGACCAAGCCGCGUGAG GAGCAGUACAACUCAACUUAUCGCGUCGUGUC AGUCCUGACAGUGCUGCACCAGGACUGGCUGA AUGGCAAGGAAUACAAGUGCAAGGUGAGUAAC AAGGCGCUGCCGGCUCCUAUCGAGAAGACCAU |
| | | | UAGUAAGGCCAAGGGCCAGCCACGCGAGCCGC AGGUGUACACCCUGCCACCGUGUCGCGAUGAA UUGACUAAGAACCAGGUUAGCCUGUGGUGCCU GGUGAAAGGCUUCUAUCCUUCUGACAUCGCUG UGGAAUGGGAGUCUAACGGCCAGCCUGAAAAC AAUUAUAAGACCACACCUCCCGUGCUCGAUUC AGACGGCUCUUUUUUCCUGUACAGCAAACUCA CUGUCGACAAGAGCCGCUGGCAACAGGGGAAC GUGUUUUCCUGCUCUGUUAUGCACGAGGCCCU GCACAAUCACUACACUCAGAAAUCUCUCUCCCU CUCUCCUGGAAAGUGAUAAGGAUCCGAUCUGG UACUGCAUGCACGCAAUGCUAGCUGCCCCUUU CCCGUCCUGGGUACCCCGAGUCUCCCCCGACC UCGGGUCCCAGGUAUGCUCCCACCUCCACCUG CCCCACUCACCACCUCUGCUAGUUCCAGACAC CUCCCAAGCACGCAGCAAUGCAGCUCAAAACG CUUAGCCUAGCCACACCCCCACGGGAAACAGC AGUGAUUAACCUUUAGCAAUAAACGAAAGUUUA ACUAAGCUAUACUAACCCCAGGGUUGGUCAAU UUCGUGCCAGCCACACCCUCGAGCUAGCAAAA AAAAAAAAAAAAAAAAAAAAAAAAAGCAUAUGA CUAAAAAAAAAAAAAAAAAAAAAAAAAAAAAAA AAAAAAAAAAAAAAAAAAAAAAAAAAAAAAAAA AA |
| 52 | 5' Cap - 5'UTR - PSMA-binding VHH #1 domain - CH2 domain - CH3 domain - 3'UTR - Poly(A) | PSMA VHH #1 | AGAAUAAACUAGUAUUCUUCUGGUCCCCACAG ACUCAGAGAGAACCCGCCACCAUGGACUGGAU CUGGAGAAUCCUGUUUCUGGUAGGCGCGGCCA CAGGCGCCCACUCCCAGUUGCAACUGGUGGAG AGUGGUGGCGGGCUGGUGCAGCCCGGCGGGU CUCUGCGCCUGUCUUGCAAAGCCUCAGGUUUU GGUUUCUCAAAAUAUGCCAUGGCCUGGCACCG UCAGGCCCCGGGUAACGCUCGCGAGCUGGUG GCAACCAUCACUACGGCCGGCGACUCUACCAA CUACCCGGAUUCCGUGAAGGGGAGAUUCACAG CCUCUCGCGAUAACGCCAAGAGUACCGCCUAU CUGCAGAUGAAUUCUCUGAAACCCGAGGACAC CGCGGUCUACCUGUGUAACGCCUACGAUUACU UCGGAGACAACGAAAUUGACUUCUGGGGCCAG GGUACGCAGGUCACCGUUUCUAGCGGCGGUG GGGGUUCCGGAGGCGGAGGUUCUGAUAAAACC CACACAUGUCCUCCGUGCCCCGCGCCUGAAAG CACCCGCGGCCCCAGCGUGUUCCUCUUUCCGC CCAAGCCCAAAGACACUCUUAUGAUUUCUCGC ACCCCCGAGGUGACCUGUGUGGUAGUUGACGU UAGCCACGAGGACCCUGAGGUGAAGUUCAACU GGUAUGUCGAUGGAGUGGAGGUACAUAAUGCC AAGACUAAGCCUCGUGAGGAGCAGUACAACUC CACCUACCGUGUCGUUAGCGUGCUGACAGUGC UUCAUCAGGAUUGGUUGAACGGCAAGGAGUAC AAGUGCAAGGUUUCCAAUAAGGCACUUCCCGC GCCAAUCGAGAAAACCAUCAGUAAGGCAAAGG GACAACCGCGCGAGCCUCAGGUCUGUACCCUU CCUCCGAGCAGAGACGAGCUGACGAAGAAUCA GGUCUCCCUGUCCUGCGCUGUGAAGGGCUUC UACCCCUCCGACAUCGCCGUCGAGUGGGAAAG CAAUGGACAACCCGAGAAUAACUACAAGACUAC CCCUCCCGUGCUGGACUCCGAUGGAAGUUUUU UCCUGGUGUCUAAGCUCACCGUGGACAAGUCC CGCUGGCAGCAAGGGAACGUCUUCAGCUGUUC CGUCAUGCAUGAAGCUCUGCAUAACCACUACA CUCAGAAAUCCCUGUCUCUGUCCCCGGGGAAG |
| | | | UAAUAAGGAUCCGAUCUGGUACUGCAUGCACG CAAUGCUAGCUGCCCCUUUCCCGUCCUGGGUA CCCCGAGUCUCCCCCGACCUCGGGUCCCAGGU AUGCUCCCACCUCCACCUGCCCCACUCACCAC CUCUGCUAGUUCCAGACACCUCCCAAGCACGC AGCAAUGCAGCUCAAAACGCUUAGCCUAGCCA CACCCCCACGGGAAACAGCAGUGAUUAACCUU UAGCAAUAAACGAAAGUUUAACUAAGCUAUACU AACCCCAGGGUUGGUCAAUUUCGUGCCAGCCA CACCCUCGAGCUAGCAAAAAAAAAAAAAAAAAA AAAAAAAAAAAAGCAUAUGACUAAAAAAAAAAAA AAAAAAAAAAAAAAAAAAAAAAAAAAAAAAAAAA AAAAAAAAAAAAAAAAAAAAAAAA |
| 53 | 5' Cap - 5'UTR - PSMA-binding VHH #2 domain - CH2 domain - CH3 domain - 3'UTR - Poly(A) | PSMA VHH #2 | AGAAUAAACUAGUAUUCUUCUGGUCCCCACAG ACUCAGAGAGAACCCGCCACCAUGGACUGGAU CUGGCGUAUUUUGUUCCUGGUGGUGCCGCU ACCGGAGCUCACUCCCAGCUUCAGCUGGUCGA GUCUGGAGGUGGGCUGGUCCAGCCGGGAGGC CCUCUGCGCCUGUCCUGCGCAGCUUCAGGAUU CACUUUUAGCACACACGCGAUGGCGUGGUAUC GUCAGGCUCCUGGCAAUGAGCGCGAGUUGGU GGCCCUGAUCACCUCCACCGACGGCACCACAA AGUAUGCUGACUCUGUGAAGGGCCGUUUCACG AUCUCCCGUGAUAACGCUGGCAAUACUGUUUA CUUGCAGAUGAACUCCCUUAAGCCGGAAGAUA CCGCCGUGUAUUACUGCAACGCUUAUAACUAC UUCGGCGACUAUGAGGAUGACUAUUGGGGCCA GGGCACCCUGGUCACCGUGUCAUCCGGCGGA GGGGGCUCCGGAGGCGGUGGCUCUGAUAAGA CCCACACAUGUCCCCCAUGUCCGGCCCCGGAG UCUACCCGCGGCCCCUCCGUAUUUCUGUUCCC UCCGAAGCCCAAGGACACCCUCAUGAUCAGUC GGACUCCCGAAGUGACAUGUGUGGUCGUGGAC GUGAGUCACGAAGACCCUGAGGUGAAGUUCAA CUGGUACGUGGACGGGGUUGAGGUCCACAAC GCUAAGACCAAGCCUCGCGAGGAACAAUAUAA UUCUACCUACAGGGUGGUCUCUGUGCUUACCG UGCUGCAUCAGGACUGGCUCAACGGAAGGAG UACAAAUGUAAGGUCAGCAACAAGGCCCUGCC GGCGCCAAUCGAAAAAACAAUCUCAAAGGCCAA AGGCCAGCCAAGAGAACCCCAGGUGUGCACAC UGCCCCCUUCCCGUGACGAGCUCACCAAGAAC CAGGUCUCCCUGAGCUGCGCCGUUAAGGGCUU UUACCCCUCCGAUAUCGCCGUGGAAUGGGAGU CCAACGGCCAGCCCGAGAAUAACUACAAGACG ACUCCCCCGGUGCUUGACUCUGAUGGGUCAUU UUUCCUGGUGUCUAAGCUGACGGUGGACAAGU CCCGGUGGCAACAGGGCAACGUGUUUCAUGU UCAGUCAUGCAUGAGGCCCUUCACAACCACUA CACACAAAAAAGCCUGUCCCUGUCCCCUGGCA AGUGAUAAGGAUCCGAUCUGGUACUGCAUGCA CGCAAUGCUAGCUGCCCCUUUCCCGUCCUGGG UACCCCGAGUCUCCCCCGACCUCGGGUCCCAG GUAUGCUCCCACCUCCACCUGCCCCACUCACC ACCUCUGCUAGUUCCAGACACCUCCCAAGCAC GCAGCAAUGCAGCUCAAAACGCUUAGCCUAGC CACACCCCCACGGGAAACAGCAGUGAUUAACC UUUAGCAAUAAACGAAAGUUUAACUAAGCUAUA CUAACCCCAGGGUUGGUCAAUUUCGUGCCAGC CACACCCUCGAGCUAGCAAAAAAAAAAAAAAA AAAAAAAAAAAAAAAGCAUAUGACUAAAAAAAAAA |
| | | | AAAAAAAAAAAAAAAAAAAAAAAAAAAAAAAAAAAAAAAA AAAAAAAAAAAAAAAAAAAAAAAAAAAA |
| 54 | 5' Cap - 5'UTR - CD3 #1-VL domain - CD3-CL domain - 3'UTR - Poly(A) | CD3 #1 | AGAAUAAACUAGUAUUCUUCUGGUCCCCACAG ACUCAGAGAGAACCCGCCACCAUGGAUUGGAU CUGGCGGAUCCUGUUCCUUGUGGGAGCUGCC ACAGGCGCCCACUCUGAGAUUGUGAUGACACA GAGCCCCGCCACACUGUCCGUUUCUCCAGGCG AAAAAGCCACACUGAGCUGCCAGGCCAGCCAG ACCAUCUACAAUUACCUGGCCUGGUAUCAGCA GAAGCCCGGCCAACCUCCUCGGCUGCUGAUCU ACAAAGCCAGCACACUGGCCAGCGGCAUCCCU GCCAGAUUUUCUGGCAGCGGCUCUGGCACCGA GUUCACCCUGACAAUCAGCAACCUGCAGAGCG AGGACUUCGCCGUGUACUACUGCCAGCAGUAC AACACCAUCAUCAACGUGGACCGGACCUUCGG CGGAGGCACCAAGGUGGAAAUCAAGAGAACAG UGGCCGCUCCUAGCGUGUUCAUCUUCCCACCU UCCGACGAGCAGCUGAAAAGCGGCACAGCCUC UGUCGUGUGCCUGCUGAACAACUUCUACCCCA GAGAAGCCAAGGUGCAGUGGAAGGUGGACAAC GCCCUGCAGUCCGGCAAUAGCCAAGAGAGCGU GACCGAGCAGGACAGCAAGGACUCUACCUACA GCCUGAGCAGCACCCUGACUCUGAGCAAGGCC GACUACGAGAAGCACAAAGUGUACGCCUGCGA AGUGACCCACCAGGGCCUUUCUAGCCCUGUGA CCAAGAGCUUCAACCGGGGCGAGUGUUGAUAA GGAUCCGAUCUGGUACUGCAUGCACGCAAUGC UAGCUGCCCCUUUCCCGUCCUGGGUACCCCGA GUCUCCCCCGACCUCGGGUCCCAGGUAUGCUC CCACCUCCACCUGCCCCACUCACCACCUCUGC UAGUUCCAGACACCUCCCAAGCACGCAGCAAU GCAGCUCAAAACGCUUAGCCUAGCCACACCCC CACGGGAAACAGCAGUGAUUAACCUUUAGCAA UAAACGAAAGUUUAACUAAGCUAUACUAACCCC AGGGUUGGUCAAUUUCGUGCCAGCCACACCCU CGAGCUAGCAAAAAAAAAAAAAAAAAAAAAAAA AAAAAGCAUAUGACUAAAAAAAAAAAAAAAAAA AAAAAAAAAAAAAAAAAAAAAAAAAAAAAAAAAA AAAAAAAAAAAAAAAAA |
| 55 | 5' Cap - 5'UTR - CD3 #2-VL domain - CD3-CL domain - 3'UTR - Poly(A) | CD3 #2 | AGAAUAAACUAGUAUUCUUCUGGUCCCCACAG ACUCAGAGAGAACCCGCCACCAUGGAUUGGAU CUGGCGGAUCCUGUUCCUUGUGGGAGCUGCC ACAGGCGCCCAUUCUGCCAUUCAGAUGACACA GAGCCCCAGCAGCCUGUCUGCCUCUGUGGGA GACAGAGUGACCAUCACCUGUCAGGCCAGCCA GACCAUCUACAACUACCUGGCCUGGUAUCAGC AGAAACCCGGCAAGGUGCCAGAGCUGCUGAUC UACAAGGCCAGCACACUGGCUAGCGGCGUGCC CUCUAGAUUCAUGGGCACAGGCUUCGGCACCG ACUUCACCCUGACCAUCGAUAGCCUGCAGCCU GAAGAUGCCGCCACCUACUACUGCCAGCAGUA CAACACCAUCAUCAACGUGGACCGGACCUUCG GCGGAGGCACCAAGGUGGAAAUCAAGAGAACA GUGGCCGCUCCUAGCGUGUUCAUCUUCCCACC UUCCGACGAGCAGCUGAAGUCUGGCACAGCCU CUGUCGUGUGCCUGCUGAACAACUUCUACCCC AGAGAAGCCAAGGUGCAGUGGAAGGUGGACAA CGCCCUGCAGAGCGGCAAUAGCCAAGAGAGCG UGACCGAGCAGGACAGCAAGGACUCUACCUAC AGCCUGAGCAGCACCCUGACACUGAGCAAGGC CGACUACGAGAAGCACAAAGUGUACGCCUGCG |
| | | | AAGUGACCCACCAGGGCCUUUCUAGCCCUGUG ACCAAGAGCUUCAACCGGGGCGAGUGUUGAUA AGGAUCCGAUCUGGUACUGCAUGCACGCAAUG CUAGCUGCCCCUUUCCCGUCCUGGGUACCCCG AGUCUCCCCCGACCUCGGGUCCCAGGUAUGCU CCCACCUCCACCUGCCCCACUCACCACCUCUG CUAGUUCCAGACACCUCCCAAGCACGCAGCAA UGCAGCUCAAAACGCUUAGCCUAGCCACACCC CCACGGGAAACAGCAGUGAUUAACCUUUAGCA AUAAACGAAAGUUUAACUAAGCUAUACUAACCC CAGGGUUGGUCAAUUUCGUGCCAGCCACACCC UCGAGCUAGCAAAAAAAAAAAAAAAAAAAAAA AAAAAAGCAUAUGACUAAAAAAAAAAAAAAAAAA AAAAAAAAAAAAAAAAAAAAAAAAAAAAAAAAAA AAAAAAAAAAAAAAAAAA |
| 56 | 5' Cap - 5'UTR - CD3 #3-VL domain - CD3-CL domain - 3'UTR - Poly(A) | CD3 #3 | AGAAUAAACUAGUAUUCUUCUGGUCCCCACAG ACUCAGAGAGAACCCGCCACCAUGGAUUGGAU CUGGCGGAUCCUGUUCCUUGUGGGAGCUGCC ACAGGCGCCCACAGCGAUAUUCAGAUGACACA GAGCCCCAGCAGCCUGUCUGCCUCUGUGGGA GACAGAGUGACCAUCACCUGUCAGGCCAGCCA GACCAUCUACAACUACCUGGCCUGGUAUCAGC AGAAGCCCGGCAAGCAGCCUAAGCUGCUGAUC UACAAGGCCAGCACACUGGCCAGCGGAGUGCC CUCUAGAUUCAAAGGCUCUGGCAGCGGCACCG ACUUCACCCUGACCAUAUCUAGCCUGCAGCCU GAGGACGUGGCCACCUACUACUGCCAGCAGUA CAACACCAUCAUCAACGUGGACCGGACCUUCG GCGGAGGAACAGAGGUGGUGGUCAAGAGAACA GUGGCCGCUCCUAGCGUGUUCAUCUUCCCACC UUCCGACGAGCAGCUGAAGUCUGGCACAGCCU CUGUCGUGUGCCUGCUGAACAACUUCUACCCC AGAGAAGCCAAGGUGCAGUGGAAGGUGGACAA CGCCCUGCAGAGCGGCAAUAGCCAAGAGAGCG UGACCGAGCAGGACAGCAAGGACUCUACCUAC AGCCUGUCCUCCACACUGACCCUGAGCAAGGC CGACUACGAGAAGCACAAAGUGUACGCCUGCG AAGUGACCCACCAGGGCCUUUCUAGCCCUGUG ACCAAGAGCUUCAACCGGGGCGAGUGUUGAUA AGGAUCCGAUCUGGUACUGCAUGCACGCAAUG CUAGCUGCCCCUUUCCCGUCCUGGGUACCCCG AGUCUCCCCCGACCUCGGGUCCCAGGUAUGCU CCCACCUCCACCUGCCCCACUCACCACCUCUG CUAGUUCCAGACACCUCCCAAGCACGCAGCAA UGCAGCUCAAAACGCUUAGCCUAGCCACACCC CCACGGGAAACAGCAGUGAUUAACCUUUAGCA AUAAACGAAAGUUUAACUAAGCUAUACUAACCC CAGGGUUGGUCAAUUUCGUGCCAGCCACACCC UCGAGCUAGCAAAAAAAAAAAAAAAAAAAAAAA AAAAAAGCAUAUGACUAAAAAAAAAAAAAAAAA AAAAAAAAAAAAAAAAAAAAAAAAAAAAAAAAAA AAAAAAAAAAAAAAAAAA |
| 57 | Human PSMA (FOLH1/GCPII) | Uniprot: Q04609 | MWNLLHETDSAVATARRPRWLCAGALVLAGGFFLL GFLFGWFIKSSNEATNITPKHNMKAFLDELKAENIK KFLYNFTQIPHLAGTEQNFQLAKQIQSQWKEFGLD SVELAHYDVLLSYPNKTHPNYISIINEDGNEIFNTSL FEPPPPGYENVSDIVPPFSAFSPQGMPEGDLVYVN YARTEDFFKLERDMKINCSGKIVIARYGKVFRGNKV KNAQLAGAKGVILYSDPADYFAPGVKSYPDGWNL PGGGVQRGNILNLNGAGDPLTPGYPANEYAYRRGI AEAVGLPSIPVHPIGYYDAQKLLEKMGGSAPPDSS |
| | | | WRGSLKVPYNVGPGFTGNFSTQKVKMHIHSTNEV TRIYNVIGTLRGAVEPDRYVILGGHRDSWVFGGIDP QSGAAVVHEIVRSFGTLKKEGWRPRRTILFASWDA EEFGLLGSTEWAEENSRLLQERGVAYINADSSIEG NYTLRVDCTPLMYSLVHNLTKELKSPDEGFEGKSL YESWTKKSPSPEFSGMPRISKLGSGNDFEVFFQR LGIASGRARYTKNWETNKFSGYPLYHSVYETYELV EKFYDPMFKYHLTVAQVRGGMVFELANSIVLPFDC RDYAVVLRKYADKIYSISMKHPQEMKTYSVSFDSLF SAVKNFTEIASKFSERLQDFDKSNPIVLRMMNDQL MFLERAFIDPLGLPDRPFYRHVIYAPSSHNKYAGES FPGIYDALFDIESKVDPSKAWGEVKRQIYVAAFTVQ AAAETLSEVA |
| 58 | Linker | (G4S)₂ | GGGGSGGGGS |
| 59 | CH2 domain (STR) | Human IgG1 (Fc-silenced) | DKTHTCPPCPAPESTRGPSVFLFPPKPKDTLMISR TPEVTCVVVDVSHEDPEVKFNWYVDGVEVHNAKT KPREEQYNSTYRVVSVLTVLHQDWLNGKEYKCKV SNKALPAPIEKTISKAK |
| 60 | CH2 domain wt | Human IgG1 | DKTHTCPPCPAPELLGGPSVFLFPPKPKDTLMISRT PEVTCVVVDVSHEDPEVKFNWYVDGVEVHNAKTK PREEQYNSTYRVVSVLTVLHQDWLNGKEYKCKVS NKALPAPIEKTISKAK |
| 61 | CH3 domain advanced knob | Human IgG1 (for heterodimerizati on) | GQPREPQVYTLPPCRDELTKNQVSLWCLVKGFYP SDIAVEWESNGQPENNYKTTPPVLDSDGSFFLYSK LTVDKSRWQQGNVFSCSVMHEALHNHYTQKSLSL SPGK |
| 62 | CH3 domain advanced hole | Human IgG1 (for heterodimerizati on) | GQPREPQVCTLPPSRDELTKNQVSLSCAVKGFYP SDIAVEWESNGQPENNYKTTPPVLDSDGSFFLVSK LTVDKSRWQQGNVFSCSVMHEALHNHYTQKSLSL SPGK |
| 63 | CH3 domain wildtype | Human IgG1 | GQPREPQVYTLPPSRDELTKNQVSLTCLVKGFYPS DIAVEWESNGQPENNYKTTPPVLDSDGSFFLYSKL TVDKSRWQQGNVFSCSVMHEALHNHYTQKSLSL SPGK |
| 64 | Human IgG1 Hinge region | Human IgG1 | EPKSCDKTHTCPPCP |
| 65 | PSMA-binding VHH domain - CD3-VH domain - CD3-CH1 domain - hinge region - CH2 domain - CH3 domain | PSMA VHH #1 x CD3 #1 | QLQLVESGGGLVQPGGSLRLSCKASGFGFSKYAM AWHRQAPGNARELVATITTAGDSTNYPDSVKGRFT ASRDNAKSTAYLQMNSLKPEDTAVYLCNAYDYFGD NEIDFWGQGTQVTVSSGGGGSGGGGSEVQLVES GGGLVKPGGSLGLSCTASGFSFSNNYYMCWVRR APGKGLEWVGCIYTNNIGSTWYASWAKGRFTISRD DSKNTVYLQMNSLKTEDTAVYYCARGIYSDSSDFI GNLWGQGTLVTVSSASTKGPSVFPLAPSSKSTSG GTAALGCLVKDYFPEPVTVSWNSGALTSGVHTFPA VLQSSGLYSLSSVVTVPSSSLGTQTYICNVNHKPS NTKVDKKVEPKSCDKTHTCPPCPAPESTRGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPCRDELTKNQVSLWCLVKGFYPSDIAVEW ESNGQPENNYKTTPPVLDSDGSFFLYSKLTVDKSR WQQGNVFSCSVMHEALHNHYTQKSLSLSPGK |
| 66 | PSMA-binding VHH domain - CD3-VH domain - CD3-CH1 domain - hinge region - CH2 domain - CH3 domain | PSMA VHH #1 x CD3 #2 | QLQLVESGGGLVQPGGSLRLSCKASGFGFSKYAM AWHRQAPGNARELVATITTAGDSTNYPDSVKGRFT ASRDNAKSTAYLQMNSLKPEDTAVYLCNAYDYFGD NEIDFWGQGTQVTVSSGGGGSGGGGSQVQLVQS GGGLVQPGGPLRLSCTASGFSFSNNYYMCWVRQ APGKGLEWVSCIYTNNIGSTWYASWAKGRFTISRD NSKNTLYLQMNSLRAEDTAVYYCARGIYSDSSDFI GNLWGQGTLVTVSSASTKGPSVFPLAPSSKSTSG |
| | | | GTAALGCLVKDYFPEPVTVSWNSGALTSGVHTFPA VLQSSGLYSLSSVVTVPSSSLGTQTYICNVNHKPS NTKVDKKVEPKSCDKTHTCPPCPAPESTRGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPCRDELTKNQVSLWCLVKGFYPSDIAVEW ESNGQPENNYKTTPPVLDSDGSFFLYSKLTVDKSR WQQGNVFSCSVMHEALHNHYTQKSLSLSPGK |
| 67 | PSMA-binding VHH domain - CD3-VH domain - CD3-CH1 domain - hinge region - CH2 domain - CH3 domain | PSMA VHH #1 x CD3 #3 | QLQLVESGGGLVQPGGSLRLSCKASGFGFSKYAM AWHRQAPGNARELVATITTAGDSTNYPDSVKGRFT ASRDNAKSTAYLQMNSLKPEDTAVYLCNAYDYFGD NEIDFWGQGTQVTVSSGGGGSGGGGSEVQLVES GGGLVKPGGSLGLSCTASGFSFSNNYYMCWVRR APGKGLEWVGCIYTNNIGSTWYASWAKGRFTISRD DSKNTVYLQMNSLKTEDTAVYYCARGIYSDSSDFI GNLWGQGTLVTVSSASTKGPSVFPLAPSSKSTSG GTAALGCLVKDYFPEPVTVSWNSGALTSGVHTFPA VLQSSGLYSLSSVVTVPSSSLGTQTYICNVNHKPS NTKVDKKVEPKSCDKTHTCPPCPAPESTRGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPCRDELTKNQVSLWCLVKGFYPSDIAVEW ESNGQPENNYKTTPPVLDSDGSFFLYSKLTVDKSR WQQGNVFSCSVMHEALHNHYTQKSLSLSPGK |
| 68 | PSMA-binding VHH domain - CD3-VH domain - CD3-CH1 domain - hinge region - CH2 domain - CH3 domain | PSMA VHH #2 x CD3 #1 | QLQLVESGGGLVQPGGPLRLSCAASGFTFSTHAM AWYRQAPGNERELVALITSTDGTTKYADSVKGRFTI SRDNAGNTVYLQMNSLKPEDTAVYYCNAYNYFGD YEDDYWGQGTLVTVSSGGGGSGGGGSEVQLVES GGGLVKPGGSLGLSCTASGFSFSNNYYMCWVRR APGKGLEWVGCIYTNNIGSTWYASWAKGRFTISRD DSKNTVYLQMNSLKTEDTAVYYCARGIYSDSSDFI GNLWGQGTLVTVSSASTKGPSVFPLAPSSKSTSG GTAALGCLVKDYFPEPVTVSWNSGALTSGVHTFPA VLQSSGLYSLSSVVTVPSSSLGTQTYICNVNHKPS NTKVDKKVEPKSCDKTHTCPPCPAPESTRGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPCRDELTKNQVSLWCLVKGFYPSDIAVEW ESNGQPENNYKTTPPVLDSDGSFFLYSKLTVDKSR WQQGNVFSCSVMHEALHNHYTQKSLSLSPGK |
| 69 | PSMA-binding VHH domain - CD3-VH domain - CD3-CH1 domain - hinge region - CH2 domain - CH3 domain | PSMA VHH #2 x CD3 #2 | QLQLVESGGGLVQPGGPLRLSCAASGFTFSTHAM AWYRQAPGNERELVALITSTDGTTKYADSVKGRFTI SRDNAGNTVYLQMNSLKPEDTAVYYCNAYNYFGD YEDDYWGQGTLVTVSSGGGGSGGGGSQVQLVQS GGGLVQPGGPLRLSCTASGFSFSNNYYMCWVRQ APGKGLEWVSCIYTNNIGSTWYASWAKGRFTISRD NSKNTLYLQMNSLRAEDTAVYYCARGIYSDSSDFI GNLWGQGTLVTVSSASTKGPSVFPLAPSSKSTSG GTAALGCLVKDYFPEPVTVSWNSGALTSGVHTFPA VLQSSGLYSLSSVVTVPSSSLGTQTYICNVNHKPS NTKVDKKVEPKSCDKTHTCPPCPAPESTRGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPCRDELTKNQVSLWCLVKGFYPSDIAVEW ESNGQPENNYKTTPPVLDSDGSFFLYSKLTVDKSR WQQGNVFSCSVMHEALHNHYTQKSLSLSPGK |
| 70 | PSMA-binding VHH domain - CD3-VH domain - CD3-CH1 domain - hinge region - CH2 domain - CH3 domain | PSMA VHH #2 x CD3 #3 | QLQLVESGGGLVQPGGPLRLSCAASGFTFSTHAM AWYRQAPGNERELVALITSTDGTTKYADSVKGRFTI SRDNAGNTVYLQMNSLKPEDTAVYYCNAYNYFGD YEDDYWGQGTLVTVSSGGGGSGGGGSEVQLVES GGGLVKPGGSLGLSCTASGFSFSNNYYMCWVRR APGKGLEWVGCIYTNNIGSTWYASWAKGRFTISRD DSKNTVYLQMNSLKTEDTAVYYCARGIYSDSSDFI GNLWGQGTLVTVSSASTKGPSVFPLAPSSKSTSG GTAALGCLVKDYFPEPVTVSWNSGALTSGVHTFPA VLQSSGLYSLSSVVTVPSSSLGTQTYICNVNHKPS NTKVDKKVEPKSCDKTHTCPPCPAPESTRGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPCRDELTKNQVSLWCLVKGFYPSDIAVEW ESNGQPENNYKTTPPVLDSDGSFFLYSKLTVDKSR WQQGNVFSCSVMHEALHNHYTQKSLSLSPGK |
| 71 | PSMA-binding VHH domain - hinge region - CH2 domain - CH3 domain | PSMA VHH #1 | QLQLVESGGGLVQPGGSLRLSCKASGFGFSKYAM AWHRQAPGNARELVATITTAGDSTNYPDSVKGRFT ASRDNAKSTAYLQMNSLKPEDTAVYLCNAYDYFGD NEIDFWGQGTQVTVSSGGGGSGGGGSDKTHTCP PCPAPESTRGPSVFLFPPKPKDTLMISRTPEVTCV VVDVSHEDPEVKFNWYVDGVEVHNAKTKPREEQ YNSTYRVVSVLTVLHQDWLNGKEYKCKVSNKALP APIEKTISKAKGQPREPQVCTLPPSRDELTKNQVSL SCAVKGFYPSDIAVEWESNGQPENNYKTTPPVLDS DGSFFLVSKLTVDKSRWQQGNVFSCSVMHEALHN HYTQKSLSLSPGK |
| 72 | PSMA-binding VHH domain - hinge region - CH2 domain - CH3 domain | PSMA VHH #2 | QLQLVESGGGLVQPGGPLRLSCAASGFTFSTHAM AWYRQAPGNERELVALITSTDGTTKYADSVKGRFTI SRDNAGNTVYLQMNSLKPEDTAVYYCNAYNYFGD YEDDYWGQGTLVTVSSGGGGSGGGGSDKTHTCP PCPAPESTRGPSVFLFPPKPKDTLMISRTPEVTCV VVDVSHEDPEVKFNWYVDGVEVHNAKTKPREEQ YNSTYRVVSVLTVLHQDWLNGKEYKCKVSNKALP APIEKTISKAKGQPREPQVCTLPPSRDELTKNQVSL SCAVKGFYPSDIAVEWESNGQPENNYKTTPPVLDS DGSFFLVSKLTVDKSRWQQGNVFSCSVMHEALHN HYTQKSLSLSPGK |
| 73 | CD3-VL domain - CD3-CL domain | CD3 #1 | EIVMTQSPATLSVSPGEKATLSCQASQTIYNYLAWY QQKPGQPPRLLIYKASTLASGIPARFSGSGSGTEF TLTISNLQSEDFAVYYCQQYNTIINVDRTFGGGTKV EIKRTVAAPSVFIFPPSDEQLKSGTASVVCLLNNFY PREAKVQWKVDNALQSGNSQESVTEQDSKDSTY SLSSTLTLSKADYEKHKVYACEVTHQGLSSPVTKS FNRGEC |
| 74 | CD3-VL domain - CD3-CL domain | CD3 #2 | AIQMTQSPSSLSASVGDRVTITCQASQTIYNYLAW YQQKPGKVPELLIYKASTLASGVPSRFMGTGFGTD FTLTIDSLQPEDAATYYCQQYNTIINVDRTFGGGTK VEIKRTVAAPSVFIFPPSDEQLKSGTASVVCLLNNF YPREAKVQWKVDNALQSGNSQESVTEQDSKDST YSLSSTLTLSKADYEKHKVYACEVTHQGLSSPVTK SFNRGEC |
| 75 | CD3-VL domain - CD3-CL domain | CD3 #3 | DIQMTQSPSSLSASVGDRVTITCQASQTIYNYLAW YQQKPGKQPKLLIYKASTLASGVPSRFKGSGSGTD FTLTISSLQPEDVATYYCQQYNTIINVDRTFGGGTE VVVKRTVAAPSVFIFPPSDEQLKSGTASVVCLLNNF YPREAKVQWKVDNALQSGNSQESVTEQDSKDST YSLSSTLTLSKADYEKHKVYACEVTHQGLSSPVTK SFNRGEC |
| 76 | Kozak | | GCCACC |
| 77 | F-element | | CUGGUACUGCAUGCACGCAAUGCUAGCUGCCC CUUUCCCGUCCUGGGUACCCCGAGUCUCCCCC GACCUCGGGUCCCAGGUAUGCUCCCACCUCCA CCUGCCCCACUCACCACCUCUGCUAGUUCCAG ACACCUCC |
| 78 | I-element | | CAAGCACGCAGCAAUGCAGCUCAAAACGCUUA GCCUAGCCACACCCCCACGGGAAACAGCAGUG AUUAACCUUUAGCAAUAAACGAAAGUUUAACUA AGCUAUACUAACCCCAGGGUUGGUCAAUUUCG UGCCAGCCACACC |
| 79 | PolyA-Linker | | GCAUAUGACU |
| 80 | FI-Element + polyA30LA70 | | CUGGUACUGCAUGCACGCAAUGCUAGCUGCCC CUUUCCCGUCCUGGGUACCCCGAGUCUCCCCC GACCUCGGGUCCCAGGUAUGCUCCCACCUCCA CCUGCCCCACUCACCACCUCUGCUAGUUCCAG ACACCUCCCAAGCACGCAGCAAUGCAGCUCAAA ACGCUUAGCCUAGCCACACCCCCACGGGAAAC AGCAGUGAUUAACCUUUAGCAAUAAACGAAAGU UUAACUAAGCUAUACUAACCCCAGGGUUGGUC AAUUUCGUGCCAGCCACACCCUCGAGCUAGCA AAAAAAAAAAAAAAAAAAAAAAAAAAAAAGCAUA UGACUAAAAAAAAAAAAAAAAAAAAAAAAAAAAA AAAAAAAAAAAAAAAAAAAAAAAAAAAAAAAAAA AAAAAA |
| 81 | husec2 | Human secretion signal peptide | MDWIWRILFLVGAATGAHS |

### NUMBERED PARAGRAPHS

The present invention will now be described by way of the following paragraphs:
1. A bispecific binding molecule, preferably an antigen-binding molecule, more preferably a bispecific antibody, comprising a CD3-binding moiety and a first prostate-specific membrane antigen (PSMA)-binding moiety.
2. The bispecific binding molecule according to paragraph 1, wherein the CD3-binding moiety comprises a CD3 heavy chain variable (CD3-VH) domain and/or a CD3 light chain variable (CD3-VL) domain.
3. The bispecific binding molecule according to paragraph 1 or paragraph 2, wherein the CD3-binding moiety comprises a Fab, wherein the Fab comprises:
   (i) a heavy chain comprising a CD3 heavy chain variable (CD3-VH) domain and a CD3 heavy chain constant (CD3-CH1) domain; and
   (ii) a light chain comprising a CD3 light chain variable (CD3-VL) domain and a CD3 light chain constant (CD3-CL) domain.
4. The bispecific binding molecule according to any of paragraphs 1-3, wherein the first PSMA-binding moiety comprises a PSMA-binding VHH domain.
5. The bispecific binding molecule according to any of paragraphs 1-4, wherein the bispecific binding molecule further comprises a second PSMA-binding moiety.
6. The bispecific binding molecule according to paragraph 5, wherein each PSMA-binding moiety comprises a PSMA-binding VHH domain.
7. The bispecific binding molecule according to paragraph 5 or paragraph 6, wherein the first PSMA-binding moiety and the second PSMA-binding moiety are the same.
8. The bispecific binding molecule according to any of paragraphs 1-7, wherein the first PSMA-binding moiety is linked to the CD3-binding moiety.
9. The bispecific binding molecule according to paragraph 8, wherein:
   (i) the C-terminus of the first PSMA-binding moiety is linked to the N-terminus of the CD3-VH domain; or
   (ii) the C-terminus of the first PSMA-binding moiety is linked to the N-terminus of the CD3-VL domain.
10. The bispecific binding molecule according to any of paragraphs 1-9, wherein the bispecific binding molecule further comprises an Fc region comprising a first subunit and a second subunit.
11. The bispecific binding molecule according to paragraph 10, wherein the CD3-binding moiety is linked to the Fc region.
12. The bispecific binding molecule according to paragraph 10 or paragraph 11, wherein the second PSMA-binding moiety is linked to the Fc region.
13. The bispecific binding molecule according to any of paragraphs 10-12, wherein:
   (i) the C-terminus of the CD3-CH1 domain is linked to the first subunit of the Fc region; or
   (ii) the C-terminus of the CD3-CL domain is linked to the first subunit of the Fc region.
14. The bispecific binding molecule according to any of paragraphs 10-13, wherein the C-terminus of the second PSMA-binding moiety is linked to the second subunit of the Fc region.
15. The bispecific binding molecule according to paragraph 13 or paragraph 14, wherein:
   (i) the C-terminus of the CD3-CH1 domain is linked to a hinge region of the first subunit of the Fc region; or
   (ii) the C-terminus of the CD3-CL domain is linked to a hinge region of the first subunit of the Fc region.
16. The bispecific binding molecule according to paragraph 14 or paragraph 15, wherein the C-terminus of the second PSMA-binding moiety is linked to a hinge region of the second subunit of the Fc region.
17. The bispecific binding molecule according to any of paragraphs 10-16, wherein the first subunit of the Fc region and the second subunit of the Fc region each comprise a CH2 domain and/or a CH3 domain.
18. The bispecific binding molecule according to any of paragraphs 1-17, wherein:
   (i) the CD3-binding moiety comprises a Fab, wherein the Fab comprises:
      (a) a heavy chain comprising the CD3-VH domain and a heavy chain constant (CD3-CH1) domain; and
      (b) a light chain comprising the CD3-VL domain and a light chain constant (CD3-CL) domain;
   (ii) the bispecific binding molecule further comprises a second PSMA-binding moiety, wherein the first PSMA-binding moiety and the second PSMA-binding moiety are the same, wherein the first PSMA-binding moiety and the second PSMA-binding moiety each comprise a PSMA-binding VHH domain;
   (iii) the bispecific binding molecule further comprises an Fc region comprising a first subunit and a second subunit; wherein the first subunit of the Fc region and the second subunit of the Fc region each comprise a CH2 domain and a CH3 domain; optionally wherein the CH3 domain of one subunit of the Fc region comprises one or more mutations selected from the list consisting of: Y349C, T366S, L368A, and Y407V; and the CH3 domain of the other subunit of the Fc region comprises one or more mutations selected from the list consisting of: S354C and T366W;
   (iv) the C-terminus of the PSMA-binding VHH domain of the first PSMA-binding moiety is linked to the N-terminus of the CD3-VH domain;
   (v) the C-terminus of the CD3-CH1 domain is linked to the N-terminus of the CH2 domain of the first subunit of the Fc region, preferably to the N-terminus of a hinge region of the CH2 domain of the first subunit of the Fc region; and
   (vi) the C-terminus of the PSMA-binding VHH domain of the second PSMA-binding moiety is linked to the N-terminus of the CH2 domain of the second subunit of the Fc region, preferably to the N-terminus of a hinge region of the CH2 domain of the second subunit of the Fc region.
19. The bispecific binding molecule according to any of paragraphs 1-18, wherein the CD3-binding moiety comprises heavy chain complementarity determining regions (HCDRs) 1-3, and/or light chain complementarity determining regions (LCDRs) 1-3, wherein
   (i) (according to Kabat numbering)
      HCDR1 comprises the amino acid sequence according to SEQ ID NO: 1,
      HCDR2 comprises the amino acid sequence according to SEQ ID NO: 2, and
      HCDR3 comprises the amino acid sequence according to SEQ ID NO: 3,
         and/or
      LCDR1 comprises the amino acid sequence according to SEQ ID NO: 4,
      LCDR2 comprises the amino acid sequence according to SEQ ID NO: 5, and
      LCDR3 comprises the amino acid sequence according to SEQ ID NO: 6;
      or
   (ii) (according to IMGT numbering)
      HCDR1 comprises the amino acid sequence according to SEQ ID NO: 7,
      HCDR2 comprises the amino acid sequence according to SEQ ID NO: 8,
      HCDR3 comprises the amino acid sequence according to SEQ ID NO: 9,
         and/or
      LCDR1 comprises the amino acid sequence according to SEQ ID NO: 10,
      LCDR2 comprises the amino acid sequence according to SEQ ID NO: 11, and
      LCDR3 comprises the amino acid sequence according to SEQ ID NO: 12;
      optionally wherein one or more of the CDRs comprises one, two or three amino acid mutations relative to the recited sequences.
20. The bispecific binding molecule according to paragraph 19, wherein the HCDR1, HCDR2 and HCDR3 sequences consist of:
   (i) SEQ ID NO: 1, SEQ ID NO: 2, and SEQ ID NO: 3, respectively; or
   (ii) SEQ ID NO: 7, SEQ ID NO: 8, and SEQ ID NO: 9, respectively.
21. The bispecific binding molecule according to paragraph 19 or paragraph 20, wherein the LCDR1, LCDR2 and LCDR3 sequences consist of:
   (i) SEQ ID NO: 4, SEQ ID NO: 5, and SEQ ID NO: 6, respectively; or
   (ii) SEQ ID NO: 10, SEQ ID NO: 11, and SEQ ID NO: 12, respectively.
22. The bispecific binding molecule according to any of paragraphs 1 to 21, wherein the CD3-binding moiety comprises a CD3-VH domain sequence having at least 70%, at least 75%, at least 80%, at least 85%, at least 90%, at least 95%, at least 97%, at least 99%, or 100% identity to any one of SEQ ID NO: 13, SEQ ID NO: 14, or SEQ ID NO: 15.
23. The bispecific binding molecule according to any of paragraphs 2 to 22, wherein the CD3-VH domain comprises or consists of a sequence as set forth in any one of SEQ ID NO: 13, SEQ ID NO: 14, or SEQ ID NO: 15.
24. The bispecific binding molecule according to any of paragraphs 1 to 23, wherein the CD3-binding moiety comprises a CD3-VL domain sequence having at least 70%, at least 75%, at least 80%, at least 85%, at least 90%, at least 95%, at least 97%, at least 99%, or 100% identity to any one of SEQ ID NO: 17, SEQ ID NO: 18, or SEQ ID NO: 19.
25. The bispecific binding molecule according to any of paragraphs 2 to 24, wherein the CD3-VL domain comprises or consists of a sequence as set forth in any one of SEQ ID NO: 17, SEQ ID NO: 18, or SEQ ID NO: 19.
26. The bispecific binding molecule according to any of paragraphs 1 to 25, wherein the CD3-binding moiety comprises a heavy chain comprising a sequence having at least 70%, at least 75%, at least 80%, at least 85%, at least 90%, at least 95%, at least 97%, at least 99%, or 100% identity to any one of SEQ ID NO: 21, SEQ ID NO: 22, or SEQ ID NO: 23.
27. The bispecific binding molecule according to any of paragraphs 1 to 26, wherein the CD3-binding moiety comprises a heavy chain comprising or consisting of a sequence as set forth in any one of SEQ ID NO: 21, SEQ ID NO: 22, or SEQ ID NO: 23.
28. The bispecific binding molecule according to any of paragraphs 1 to 27, wherein the CD3-binding moiety comprises a light chain comprising a sequence having at least 70%, at least 75%, at least 80%, at least 85%, at least 90%, at least 95%, at least 97%, at least 99%, or 100% identity to any one of SEQ ID NO: 25, SEQ ID NO: 26, or SEQ ID NO: 27.
29. The bispecific binding molecule according to any of paragraphs 1 to 28, wherein the CD3-binding moiety comprises a light chain comprising or consisting of a sequence as set forth in any one of SEQ ID NO: 25, SEQ ID NO: 26, or SEQ ID NO: 27.
30. The bispecific binding molecule according to any of paragraphs 1 to 29, wherein CD3 is human CD3, optionally wherein CD3 is CD3ε.
31. The bispecific binding molecule according to any of paragraphs 1 to 30, wherein the, or each, PSMA-binding moiety independently comprises heavy chain complementarity determining regions (HCDRs) 1-3, wherein (according to Kabat numbering)
   (i)
      HCDR1 comprises the amino acid sequence according to SEQ ID NO: 29,
      HCDR2 comprises the amino acid sequence according to SEQ ID NO: 30, and
      HCDR3 comprises the amino acid sequence according to SEQ ID NO: 31;
      optionally wherein one or more of the CDRs comprises one, two or three amino acid mutations relative to the recited sequences; or
   (ii)
      HCDR1 comprises the amino acid sequence according to SEQ ID NO: 36,
      HCDR2 comprises the amino acid sequence according to SEQ ID NO: 37, and
      HCDR3 comprises the amino acid sequence according to SEQ ID NO: 38;
      optionally wherein one or more of the CDRs comprises one, two or three amino acid mutations relative to the recited sequences.
32. The bispecific binding molecule according to any of paragraphs 1 to 30, wherein the, or each, PSMA-binding moiety independently comprises heavy chain complementarity determining regions (HCDRs) 1-3, wherein (according to IMGT numbering)
   (i)
      HCDR1 comprises the amino acid sequence according to SEQ ID NO: 32,
      HCDR2 comprises the amino acid sequence according to SEQ ID NO: 33, and
      HCDR3 comprises the amino acid sequence according to SEQ ID NO: 34;
      optionally wherein one or more of the CDRs comprises one, two or three amino acid mutations relative to the recited sequences; or
   (ii)
      HCDR1 comprises the amino acid sequence according to SEQ ID NO: 39,
      HCDR2 comprises the amino acid sequence according to SEQ ID NO: 40, and
      HCDR3 comprises the amino acid sequence according to SEQ ID NO: 41;
      optionally wherein one or more of the CDRs comprises one, two or three amino acid mutations relative to the recited sequences.
33. The bispecific binding molecule according to any of paragraphs 1 to 31 (i) and 32 (i), wherein the, or each, PSMA-binding moiety independently comprises a sequence having at least 70%, at least 75%, at least 80%, at least 85%, at least 90%, at least 95%, at least 97%, at least 99%, or 100% identity to SEQ ID NO: 35.
34. The bispecific binding molecule according to any of paragraphs 1 to 30, 31 (i), 32 (ii) and 33, wherein the, or each, PSMA-binding moiety independently comprises or consists of a sequence as set forth in SEQ ID NO: 35.
35. The bispecific binding molecule according to any of paragraphs 1 to 30, 31 (ii) and 32 (ii), wherein the, or each, PSMA-binding moiety independently comprises a sequence having at least 70%, at least 75%, at least 80%, at least 85%, at least 90%, at least 95%, at least 97%, at least 99%, or 100% identity to SEQ ID NO: 42.
36. The bispecific binding molecule according to any of paragraphs 1 to 30, 31 (ii), 32 (ii) and 35, wherein the, or each, PSMA-binding moiety independently comprises or consists of a sequence as set forth in SEQ ID NO: 42.
37. The bispecific binding molecule according to any of paragraphs 1 to 36, wherein the bispecific binding molecule comprises:
   (i) a first polypeptide comprising the amino acid sequence according to SEQ ID NO: 65 or a variant having at least 80% sequence identity thereto;
   (ii) a second polypeptide comprising the amino acid sequence according to SEQ ID NO: 71 or a variant having at least 80% sequence identity thereto; and/or
   (iii) a third polypeptide comprising the amino acid sequence according to SEQ ID NO: 73 or a variant having at least 80% sequence identity thereto.
38. The bispecific binding molecule according to any of paragraphs 1 to 36, wherein the bispecific binding molecule comprises:
   (i) a first polypeptide comprising the amino acid sequence according to SEQ ID NO: 66 or a variant having at least 80% sequence identity thereto;
   (ii) a second polypeptide comprising the amino acid sequence according to SEQ ID NO: 71 or a variant having at least 80% sequence identity thereto; and/or
   (iii) a third polypeptide comprising the amino acid sequence according to SEQ ID NO: 74 or a variant having at least 80% sequence identity thereto.
39. The bispecific binding molecule according to any of paragraphs 1 to 36, wherein the bispecific binding molecule comprises:
   (i) a first polypeptide comprising the amino acid sequence according to SEQ ID NO: 67 or a variant having at least 80% sequence identity thereto;
   (ii) a second polypeptide comprising the amino acid sequence according to SEQ ID NO: 71 or a variant having at least 80% sequence identity thereto; and/or
   (iii) a third polypeptide comprising the amino acid sequence according to SEQ ID NO: 75 or a variant having at least 80% sequence identity thereto.
40. The bispecific binding molecule according to any of paragraphs 1 to 36, wherein the bispecific binding molecule comprises:
   (i) a first polypeptide comprising the amino acid sequence according to SEQ ID NO: 68 or a variant having at least 80% sequence identity thereto;
   (ii) a second polypeptide comprising the amino acid sequence according to SEQ ID NO: 72 or a variant having at least 80% sequence identity thereto; and/or
   (iii) a third polypeptide comprising the amino acid sequence according to SEQ ID NO: 73 or a variant having at least 80% sequence identity thereto.
41. The bispecific binding molecule according to any of paragraphs 1 to 36, wherein the bispecific binding molecule comprises:
   (i) a first polypeptide comprising the amino acid sequence according to SEQ ID NO: 69 or a variant having at least 80% sequence identity thereto;
   (ii) a second polypeptide comprising the amino acid sequence according to SEQ ID NO: 72 or a variant having at least 80% sequence identity thereto; and/or
   (iii) a third polypeptide comprising the amino acid sequence according to SEQ ID NO: 74 or a variant having at least 80% sequence identity thereto.
42. The bispecific binding molecule according to any of paragraphs 1 to 36, wherein the bispecific binding molecule comprises:
   (i) a first polypeptide comprising the amino acid sequence according to SEQ ID NO: 70 or a variant having at least 80% sequence identity thereto;
   (ii) a second polypeptide comprising the amino acid sequence according to SEQ ID NO: 72 or a variant having at least 80% sequence identity thereto; and/or
   (iii) a third polypeptide comprising the amino acid sequence according to SEQ ID NO: 75 or a variant having at least 80% sequence identity thereto.
43. One or more polynucleotide(s) comprising a nucleic acid sequence encoding the bispecific binding molecule according to any of paragraphs 1 to 42.
44. The one or more polynucleotide(s) according to paragraph 43, wherein the nucleic acid sequence is selected from the list consisting of: a DNA sequence, a cDNA sequence, an RNA sequence, and an mRNA sequence.
45. The one or more polynucleotide(s) according to paragraph 44, wherein the nucleic acid sequence is an RNA sequence.
46. The one or more polynucleotide(s) according to paragraph 45, wherein the RNA sequence is an mRNA sequence.
47. The one or more polynucleotide(s) according to any of paragraphs 44 to 46, wherein the RNA is modRNA (N1-methyl-pseudouridine).
48. The one or more polynucleotide(s) according to any one of paragraphs 43 to 47, wherein the RNA sequence comprises a 5'-cap.
49. The one or more polynucleotide(s) according to paragraph 48, wherein the 5'-cap is CC413, further optionally wherein the 5'-cap is m₂^{7,3'-O}Gppp(ₘ₁^{2'-O})ApG, which comprises the following structure: 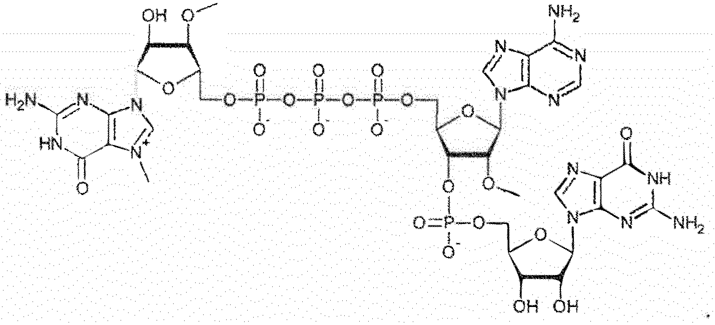
50. The one or more(s) polynucleotide according to any one of paragraphs 43 to 49, wherein the RNA sequence comprises a poly adenine sequence, optionally wherein the poly adenine sequence comprises the nucleic acid sequence according to SEQ ID NO: 43 or a variant having at least 80% sequence identity thereto.
51. The one or more polynucleotide(s) or composition according to any one of paragraphs 43 to 50, wherein the RNA sequence comprises a 5'UTR; optionally wherein the 5'UTR comprises the nucleic acid sequence according to SEQ ID NO: 44 or a variant having at least 80% sequence identity thereto.
52. The one or more polynucleotide(s) or composition according to any one of paragraphs 43 to 51, wherein the RNA sequence comprises a 3'UTR; optionally wherein the 3'UTR comprises the nucleic acid sequence according to SEQ ID NO: 45 or a variant having at least 80% sequence identity thereto.
53. The one or more polynucleotide(s) according to any one of paragraphs 43 to 52, wherein a plurality of polynucleotides comprises:
   (i) a first polynucleotide comprising a nucleic acid sequence encoding the following polypeptide chain from N to C terminus:
      PSMA-binding moiety, preferably the PSMA-binding VHH domain, - (optional linker) - CD3-VH domain - CD3-CH1 domain - CH2 domain - CH3 domain;
   (ii) a second polynucleotide comprising a nucleic acid sequence encoding the following polypeptide chain from N to C terminus:
      PSMA-binding moiety, preferably the PSMA-binding VHH domain, - (optional linker) - CH2 domain - CH3 domain; and/or
   (iii) a third polynucleotide comprising a nucleic acid sequence encoding the following polypeptide chain from N to C terminus:
      CD3-VL domain - CD3-CL domain.
54. The one or more polynucleotide(s) according to paragraphs 53, wherein:
   (i) the first polynucleotide comprises a nucleic acid sequence comprising in 5'-3' orientation:
      5'-cap - 5'UTR - PSMA-binding moiety, preferably the PSMA-binding VHH domain, - (optional linker) - CD3-VH domain -CD3-CH1 domain - CH2 domain - CH3 domain - 3'UTR - Poly(A)
   (ii) the second polynucleotide comprises a nucleic acid sequence comprising in 5'-3' orientation:
      5'-cap - 5'UTR - PSMA-binding moiety, preferably the PSMA-binding VHH domain, - (optional linker) - CH2 domain - CH3 domain - 3'UTR - Poly(A); and/or
   (ii) the third polynucleotide comprises a nucleic acid sequence comprising in 5'-3' orientation:
      5'-cap - 5'UTR - CD3-VL domain - CD3-CL domain - 3'UTR - Poly(A).
55. The one or more polynucleotide(s) according to paragraph 53 or paragraph 54, wherein:
   (i) the first polynucleotide comprises a nucleic acid sequence according to SEQ ID NO: 46 or a variant having at least 80% sequence identity to SEQ ID NO: 46;
   (ii) the second polynucleotide comprises a nucleic acid sequence according to SEQ ID NO: 52 or a variant having at least 80% sequence identity to SEQ ID NO: 52; and
   (iii) the third polynucleotide comprises a nucleic acid sequence according to SEQ ID NO: 54 or a variant having at least 80% sequence identity to SEQ ID NO: 54.
56. The one or more polynucleotide(s) according to paragraph 53 or paragraph 54, wherein:
   (i) the first polynucleotide comprises a nucleic acid sequence according to SEQ ID NO: 47 or a variant having at least 80% sequence identity to SEQ ID NO: 47;
   (ii) the second polynucleotide comprises a nucleic acid sequence according to SEQ ID NO: 52 or a variant having at least 80% sequence identity to SEQ ID NO: 52; and
   (iii) the third polynucleotide comprises a nucleic acid sequence according to SEQ ID NO: 55 or a variant having at least 80% sequence identity to SEQ ID NO: 55.
57. The one or more polynucleotide(s) according to paragraphs 53 or paragraph 54, wherein:
   (i) the first polynucleotide comprises a nucleic acid sequence according to SEQ ID NO: 48 or a variant having at least 80% sequence identity to SEQ ID NO: 48;
   (ii) the second polynucleotide comprises a nucleic acid sequence according to SEQ ID NO: 52 or a variant having at least 80% sequence identity to SEQ ID NO: 52; and
   (iii) the third polynucleotide comprises a nucleic acid sequence according to SEQ ID NO: 56 or a variant having at least 80% sequence identity to SEQ ID NO: 56.
58. The one or more polynucleotide(s) according to paragraphs 53 or paragraph 54, wherein:
   (i) the first polynucleotide comprises a nucleic acid sequence according to SEQ ID NO: 49 or a variant having at least 80% sequence identity to SEQ ID NO: 49;
   (ii) the second polynucleotide comprises a nucleic acid sequence according to SEQ ID NO: 53 or a variant having at least 80% sequence identity to SEQ ID NO: 53; and
   (iii) the third polynucleotide comprises a nucleic acid sequence according to SEQ ID NO: 54 or a variant having at least 80% sequence identity to SEQ ID NO: 54.
59. The one or more polynucleotide(s) according to paragraphs 53 or paragraph 54, wherein:
   (i) the first polynucleotide comprises a nucleic acid sequence according to SEQ ID NO: 50 or a variant having at least 80% sequence identity to SEQ ID NO: 50;
   (ii) the second polynucleotide comprises a nucleic acid sequence according to SEQ ID NO: 53 or a variant having at least 80% sequence identity to SEQ ID NO: 53; and
   (iii) the third polynucleotide comprises a nucleic acid sequence according to SEQ ID NO: 55 or a variant having at least 80% sequence identity to SEQ ID NO: 55.
60. The one or more polynucleotide(s) according to paragraphs 53 or paragraph 54, wherein:
   (i) the first polynucleotide comprises a nucleic acid sequence according to SEQ ID NO: 51 or a variant having at least 80% sequence identity to SEQ ID NO: 51;
   (ii) the second polynucleotide comprises a nucleic acid sequence according to SEQ ID NO: 53 or a variant having at least 80% sequence identity to SEQ ID NO: 53; and
   (iii) the third polynucleotide comprises a nucleic acid sequence according to SEQ ID NO: 56 or a variant having at least 80% sequence identity to SEQ ID NO: 56.
61. A vector which comprises one or more polynucleotide(s) according to any of paragraphs 43 to 60.
62. A composition comprising
   (i) the bispecific binding molecule according to any of paragraphs 1 to 42;
   (ii) the one or more polynucleotide(s) according to any of paragraphs 43 to 60; or
   (iii) the vector according to paragraph 61.
63. A pharmaceutical composition comprising:
   (i)
      (a) the bispecific binding molecule according to any of paragraphs 1 to 42; or
      (b) the one or more polynucleotide(s) according to any of paragraphs 43 to 60; or
      (c) the vector according to paragraph 61; or
      (d) the composition according to paragraph 62;
      and
   (ii) a pharmaceutically acceptable carrier, diluent or excipient,
      optionally wherein the pharmaceutical composition further comprises
   (iii) a vesicle, a nanoparticle, a lipid nanoparticle (LNP), a liposome or a polymeric mixture wherein the bispecific binding molecule, the one or more polynucleotide(s), the vector, or the composition is encapsulated within the vesicle, nanoparticle, LNP, liposome or polymeric mixture.
64. The pharmaceutical composition according to paragraph 63, wherein (iii) is an LNP.
65. The pharmaceutical composition according to paragraph 63 or paragraph 64, which is formulated for parenteral administration.
66. The pharmaceutical composition according to any of paragraphs 63 to 65, which is formulated for intravenous or intraarterial administration.
67. A method for treating and/or preventing a disease which comprises the step of administering the bispecific binding molecule according to any of paragraphs 1 to 42; or the one or more polynucleotide(s) according to any of paragraphs 43 to 60; or the vector according to paragraph 61; or the composition according to paragraph 62, or the pharmaceutical composition according to any of paragraphs 63 to 66, to a subject.
68. Use of the bispecific binding molecule according to any of paragraphs 1 to 42; or the one or more polynucleotide(s) according to any of paragraphs 43 to 60; or the vector according to paragraph 61; or the composition according to paragraph 62, or the pharmaceutical composition according to any of paragraphs 63 to 66, in the manufacture of a medicament for treating and/or preventing a disease.
69. The bispecific binding molecule according to any of paragraphs 1 to 42; or the one or more polynucleotide(s) according to any of paragraphs 43 to 60; or the vector according to paragraph 61; or the composition according to paragraph 62, or the pharmaceutical composition according to any of paragraphs 63 to 66, for use in the treatment and/or prevention of a disease.
70. The method according to paragraph 67, the use according to paragraph 68, or the bispecific binding molecule, the one or more polynucleotide(s), the vector, the composition, or the pharmaceutical composition for use according to paragraph 69, wherein the disease is cancer.
71. The method, the use, or the bispecific binding molecule, the one or more polynucleotide(s), the vector, the composition, or the pharmaceutical composition for use according to paragraph 59, wherein the cancer is prostate cancer.

### EXAMPLES

### Example 1: Determination of optimal IVT-RNA chain ratios for RiboMab expression

### Generation of polyribonucleotides encoding bispecific antibody agents

The present Example demonstrates the generation of polyribonucleotide sequences encoding bispecific anti-PSMA and anti-CD3 antibody agents. Specifically, ribonucleic acid sequences encoding PSMA VHH-directed PSMA clones and CD3-directed CD3 clones (or variants thereof) were combined with other immunoglobulin domains to form polyribonucleotides that encode the PSMA Clone and bispecific PSMA Clone-CD3 clones (or variants thereof) antibody agent immunoglobulin chains, respectively. The respective polydeoxyribonucleotides were cloned into a DNA plasmid. The polyribonucleotides were designed to encode a bispecific, asymmetric 2+1-Fab-(VHH)₂-Fc format (see FIG. 1). Exemplary antibody agent configurations and component immunoglobulin chains are shown in Table 1.

### Cloning of exemplary antibody agent-encoding DNA template vectors:

DNA sequences encoding antibody fragments were synthesized either internally or externally (Thermo Fischer Scientific). These DNA fragments were cloned into a DNA plasmid appropriate to use for RNA expression according to standard procedures well known by the person skilled in the art. In short, the parental plasmid was digested with restriction enzymes. The resulting product was analyzed via agarose gel, purified and its concentration measured by UV spectroscopy. The antibody encoding light chain and heavy chain sequences were mixed and cloned into the plasmid by transforming competent Escherichia coli cells via heat shock and subsequent in vivo assembly (Garcia-Nafria, "IVA cloning: A single-tube universal cloning system exploiting bacterial In Vivo Assembly," Scientific Reports volume 6, Article number: 27459 (2016), which is incorporated herein by reference in its entirety). Single bacterial clones were sequence verified by Sanger sequencing. Sequencing results were aligned to the reference sequence

### Plasmid DNA preparation of exemplary antibody agent-encoding DNA template vectors:

Plasmid DNA was prepared by selecting *E. coli* clones for inoculation in Luria-Bertani (LB) medium containing kanamycin. The cultures were grown overnight at 37 °C and ~ 150 to 200 rpm. Following cell harvest, purification was done using the QIAGEN Plasmid Plus Maxi or Giga Kit according to the manufacturer's instructions. Finally, the concentration and purity was determined by UV spectroscopy. DNA was stored in certified RNase- and DNase-free reaction tubes.

### Linearization and DNA purification of exemplary antibody agent-encoding DNA template vectors:

Linearization of plasmid DNA was performed using appropriate restriction enzymes, followed by purification of the linearized DNA template using magnetic beads (Dynabeads^{™} MyOne^{™} Carboxylic Acid) according to the manufacturer's protocol. The DNA concentration and purity was measured by UV spectroscopy.

### In vitro transcription of exemplary antibody agent-encoding DNA template vectors:

CleanCap 413 ((m7(3'OMeG)(5')ppp(5')(2'OMeA)pG)-capped RNA was produced following the process as disclosed, e.g., in Kreiter, S. et al. Cancer Immunol. Immunother. 56, 1577-87 (2007) and WO 2021214204A1, each of which is incorporated herein by reference in its entirety. Methyl pseudo-uridine was used in the *in vitro* transcription reaction and incorporated into the produced RNA. Purification of the resulting RNA was performed to isolate single-stranded RNA, followed by concentration measurement by UV spectroscopy. The RNA integrity was determined by microfluidic-based electrophoresis and the amount of doublestranded RNA impurities via RNA dot blot.

### Electroporation of K-562 cells with IVT-RNA

RiboMabs were generated by electroporation of K-562 cells with the IVT-RNAs for the corresponding chains according to methods known in the art. The three RiboMabs to be evaluated were generated using different ratios of IVT-RNA chains coding for the first heavy chain (HC1), the second heavy chain (HC2) and the light chain (LC). K-562 were washed twice with X-Vivo medium (Biozym) and were briefly adjusted to a density of 10 x 10⁶/mL in X-Vivo. 200 µL of cells were transferred to electroporation plates with 0.4 mm gap (BTX) and electroporated with a 25 µL RNA-mix diluted in 10 mM Hepes buffer with 1 mM EDTA at final concentrations of 25 µg/mL. Samples containing only Hepes buffer served as negative control. For electroporation, an ECM830 device (BTX Harvard Apparatus) was used. Conditions were set to 200 Volt, 3 pulses and 8 milliseconds. The 225 µL cell suspensions were seeded in 6-well cell culture plates (Greiner Bio-One) with 775 µL Expi293TM medium (Gibco) to approximately 2 x 10⁶ viable cells per mL. The cell culture supernatant was harvested after 48 hours, centrifuged via centrifugation for 10 minutes at 300 x g and stored at 2-8 °C until analysis by Western Blot (WB), quantitation via Gyros ELISA.

### Gyros ELISA for RiboMab quantitation

RiboMabs in serum or cell culture supernatant were quantified using a Gyros xPand^{™} ELISA device (Gyros Protein Technologies AB). The Gyrolab hulgG Kit - Low Titer in a dynamic range of 4 to 3000 ng/mL was used for the detection of RiboMabs in cell culture supernatant.

A sandwich assay format was performed in a Gyrolab Bioaffy 1000 HC for Low Titer Kit with ready-to-use reagents. All samples, standard protein (a purified IgG-(VHH)₂ antibody) and reagents were centrifuged for 4 min at 12,000 x g to sediment any aggregates. The cell culture supernatant samples were diluted 13.11-fold in Reagent E buffer (Gyrolab hulgG Kit - Low Titer) and 10 % mock transfection supernatant. Reagents, standards, controls, samples and wash buffers were loaded onto a 96-well plate (Gyros Protein Technologies AB). Data were generated with the Gyrolab hulgG Low Titer method v2. Results were evaluated using the Gyrolab Evaluator software.

### Western blot analysis

To evaluate RiboMab integrity, the supernatant of cells transfected with IVT-RNA was analyzed via Western blot. SDS-PAGE was performed by heating 20 µL (maximum 5-10 ng total amount) of supernatant as well as 1 ng and 5 ng of the reference protein in Laemmli buffer to 95°C for 10 min under non-reducing conditions. Supernatant from cells electroporated only with the corresponding HEPES buffer was used as negative control. The samples were separated by polyacrylamide gel electrophoresis (Mini-Protean 4-15% TGX Precast Gel, Bio-Rad), followed by Western blot analysis and incubated with a mixture of horseradish peroxidase (HRP) anti-human AB in a 5% milk- and 0.5% TBS-Tween buffer: HRP coupled Goat Anti-Human IgG Fd (Ads)-HRP, 1:2000 (full IgG and free HC detection, Hölzel Diagnostika Handels GmbH) and Goat anti-human Kappa LC antibody, HRP, 1:500 (full IgG and free LC detection, Life Technologies). Visualization was performed using the ClarityTM Western ECL Substrate (Bio-Rad), monitoring with a Vilber Fusion X imaging device (Vilber) and data were analyzed with the Image Lab Software (Bio-Rad). For the determination of band signal intensities, the analysis tool of the Imabe Lab Software was utilized and the ratio of fully assembled antibody to the HMW and LMW counterparts was estimated by calculating the ratio of the so determined band signals.

### Results of the IVT-RNA chain ratio optimization analysis

The general structure of the different 2+1-Fab-(VHH)₂-Fc Ribomabs evaluated is shown in Figure 1. They each possess three individual chains, two heavy chains, HC1 and HC2, and a light chain (LC), coded by individual IVT-RNA chains. To decide on an optimal RNA-ratio related to mass for the individual chains, both the total expression of the RiboMabs was determined via automated ELISA and the ratio of fully assembled antibody (monomer), compared to high molecular weight species (HMWs), not fully assembled antibody species and individual free chains (LMWs) via Western Blot. The results of this analysis are shown in Figure 2. All RiboMabs show a tendency to express more monomeric antibody when the ratio of the heavy chains to the light chain is higher. However, this effect is minimal and over the range of evaluated ratios the differences in expression titers are relatively low. While almost no HMWs were visible in any of the Western Blots, least free individual chains could be detected when the HC2:LC ratio was 2.8 and the HC1:LC ratio was in the range of 1.4 to 2.4, for RiboMab_PSMA-VHH#2xCD3#2 this was optimal for a HC1:LC ratio of 1.9. This ratio of HC1:HC2:LC of 1.9:2.8:1 was chosen as optimal for subsequent transfections.

### Example 2: Production titers of anti-PSMAxCD3 RiboMabs after transfection of HEK cells

### Ribofection of HEK cells with IVT-RNA

RiboMabs were generated via ribofection with HEK Expi293F cells, which are cultivated in culture flasks at 140 rpm, at 37 °C and 5% CO₂ in an incubator. 24 hours prior to the transfection cells were seeded at 2.5 x 10⁶ cells per mL. On the day of the transfection, viability and cell density were checked and if over 95% viability was achieved the cells were seeded at 6 x 10⁶ cells per mL. Per mL cell suspension 1 µg RNA was diluted in 100 µL serum-free OptiMEM medium (Gibco). 2 µL mRNA RiboJuice Boost Reagent (Merck) and 2 µL RiboJuice mRNA Reagent (Merck) are added to the diluted RNA in immediate succession and the resulting solutions each gently mixed in between. An incubation time of at least 3 min but not more than 5 min is executed at room temperature after which the RNA-mix is added dropwise to the cells. The transfected cells are then incubated in CF50 culture tubes (TPP) at 180 rpm, 37 °C and 5% CO₂ for four days. The cells are harvested by centrifugation at 300 x g for 4 min and the supernatant is decanted. The resulting supernatant is separated from remaining cells by centrifugation at 3000 x g for another 10 min. The collected supernatant is either concentrated with via centrifugation in a 50 kDa MWCO concentrator (Amicon) or directly stored at 4 °C until further analysis.

### Gyros ELISA for RiboMab quantitation

RiboMabs in serum or cell culture supernatant were quantified using a Gyros xPand^{™} ELISA device (Gyros Protein Technologies AB). The Gyrolab hulgG Kit - Low Titer in a dynamic range of 4 to 3000 ng/mL was used for the detection of RiboMabs in cell culture supernatant. A sandwich assay format was performed in a Gyrolab Bioaffy 1000 HC for Low Titer Kit with ready-to-use reagents. All samples, standard protein (a purified IgG-(VHH)₂ antibody) and reagents were centrifuged for 4 min at 12,000 x g to sediment any aggregates. The cell culture supernatant samples were diluted 13.58-fold in Reagent E buffer (Gyrolab hulgG Kit - Low Titer) and 10 % mock transfection supernatant. Reagents, standards, controls, samples and wash buffers were loaded onto a 96-well plate (Gyros Protein Technologies AB). Data were generated with the Gyrolab hulgG Low Titer method v2. Results were evaluated using the Gyrolab Evaluator software.

### Western blot analysis

To evaluate RiboMab integrity, the supernatant of cells transfected with IVT-RNA was analyzed via Western blot. SDS-PAGE was performed by heating 30 µL (prediluted for application of exactly 3 ng of fully assembled antibody) of supernatant as well as 3 ng of the control proteinin Laemmli buffer to 95°C for 10 min under non-reducing conditions. Supernatant from cells electroporated only with the corresponding HEPES buffer was used as negative control. The samples were separated by polyacrylamid gel electrophoresis (Mini-Protean 4-15% TGX Precast Gel, Bio-Rad), followed by Western blot analysis and incubated with a mixture of horseradish peroxidase (HRP) anti-human AB in a 5% milk- and 0.5% TBS-Tween buffer: Peroxidase-conjugated AffiniPure goat anti-human IgG, Fcγ-Fragment Specific, 1:1000 (full IgG and free HC detection, Hölzel Diagnostika Handels GmbH) and Goat anti-human Kappa LC antibody, HRP, 1:500 (full IgG and free LC detection, Life Technologies). Visualization was performed using the ClarityTM Western ECL Substrate (Bio-Rad), monitoring with a Vilber Fusion X imaging device (Vilber) and data were analyzed with the Image Lab Software (Bio-Rad).

### RiboMab in vitro expression and protein integrity

Expression of the tested RiboMabs after transient transfection via ribofection was in all cases sufficient for further *in vitro* evaluation. RiboMabs were analyzed on a Western Blot both unconcentrated and five times concentrated. The Western blot results (Figure 4) show a dominant band of the fully assembled monomeric antibody construct. No high molecular weight species (HMWs) can be detected, meaning a favorable aggregation profile. All three chains are visible in all reduced samples. These results indicate good protein integrity for use as RiboMabs, since higher ratios of HMWs and LMWs are undesirable, due to decreased functionality and potential unspecificity. Furthermore, the concentrated samples (5x) show the same integrity as the unconcentrated samples (1x), suggesting that even in higher concentrations the RiboMab protein might still be stable and that *in vitro* analysis in higher concentrations is possible.

### Example 3: RiboMab binding to the TCR/CD3 receptor and activation of recombinant Jurkat cells

### T-cell activation bioreporter assay

RiboMab containing supernatants were generated via electroporation of K-562 cells with corresponding IVT-RNA chains. The mass chain ratios of HC1:HC2:LC of all RiboMabs constructs during electroporation was 1.9:2.2:1. The supernatants were concentrated ten times (10x) after times via centrifugation with 50 kDa MWCO concentrator (Amicon), analyzed via sandwich ELISA and Western blot and stored at 4 °C until further analysis. The T-cell activation bioreporter assay was performed based on the T Cell Activation Bioassay (Promega). As effector cells, TCR/CD3-positive Jurkat_NFAT_luc cells expressing a luciferase reporter under the control of the NFAT promotor will be used. RiboMab binding to CD3 on the cell surface of these cells will induce intracellular signalling events resulting in NFAT-mediated luminescence. Briefly, 1.0 x 10⁶ luciferase-expressing Jurkat cells (25 µL of 4.0 x 10⁶ cells/mL stock suspension) were added to 1.0 x 10⁴ PSMA-positive LNCaP clone FGC target cells (50 µL of the 0.2 x 10⁶ cells/mL stock suspension) on a 96-well plate (Thermo Fisher) to achieve an effector-to-target (E:T) ratio of 10:1. RiboMab containing cell culture supernatant (5-fold, 10-point dilution, with the first two dilutions being 3-fold: 1000 to 0.0014 ng/mL) was added to the test wells. The purified CD3 x (PSMA)₂ tribody protein named "Positive Control" served as both a positive control and an interplate control with the same dilution steps as the test item. Further positive control was the purified OKT3 antibody (BioXCell). Supernatant from the cells of the mock transfection served as negative control. Further negative controls included Jurkat cells with HEK293/T17 cells not expressing PSMA and Jurkat cells plus the highest concentrations (dilution 1) per sample without target cells. Dilutions were performed in assay medium, except for the first dilution of the cell culture supernatant (1000 ng/mL), which included 10x mock cell culture supernatant to harmonize the matrices of the samples. Assay plates were incubated for 6 hours at 37°C with 5% CO₂. After a sample cool down for 15 min at RT, 75 µL of luciferin solution (Promega) were added followed by 20 min of incubation at RT in the dark to trigger luciferase activity and bioluminescence production. Bioluminescence was measured as relative light units (RLU) on a microplate reader (Infinite M200 Pro, Tecan). Results expressed as EC₅₀ values were derived from the dose-response curves of bioluminescence versus RiboMab or control concentration as calculated by the GraphPad Prism 9 software's "agonist vs response (three parameters)" fit. In the final graph, the luminescence values were normalized according to the lower and upper baseline values calculated by the dose-response curve fit of the individual sample.

The graphs show the results of the T-cell activation bioreporter assay. The abscissa represents the RiboMab concentration in the supernatant as determined by ELISA and the ordinate value is the luminescence normalized to the upper and lower baseline value determined by dose-response curve fit for each individual sample. The error bars are calculated from the standard deviation of three individual test well replicates. The assay was conducted with (A) PSMA-positive LNCaP cells or (B) PSMA-negative HEK293/T17 cells. (C) The positive controls on the different 96-well plates yield similar EC50 values and the negative controls show no increased luminescence.

### Results of the T-cell activation reporter bioassay

Evaluation of all RiboMabs, shown in Figure 5, yields a sigmoidal dose-response curve with concentration dependent activation of the recombinant Jurkat cells, via binding to TCR/CD3. This dose-dependent effect can only be seen when Jurkat cells and RiboMabs are co-cultured with PSMA-positive cells (Figure 5A). Figure 5B shows the results of co-culturing Jurkat cells with PSMA-negative HEK293/T17 cells (Figure 5B). Both positive controls show an increased luminescence signal from the recombinant Jurkat cells, while all controls with Mock and without target cells show no increase in luminescence (Figure 5C). This indicates that both the anti-PSMA VHH part of the RiboMab antibodies binds to its respective target on the LNCap cells specifically, but also that the anti-CD3 part of the RiboMab is able bind CD3 and through formation of an immune synapse also able to activate the recombinant Jurkat cells (Table 2).

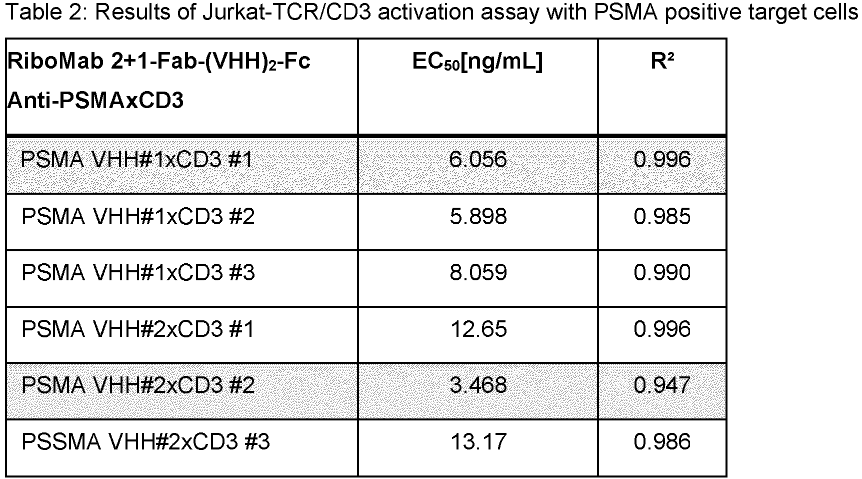

### Example 4: Anti-PSMAxCD3 RiboMabs show T-cell dependent cellular cytotoxicity specifically in the presence of PSMA-positive cells

### T-cell dependent cell-mediated cytotoxicity (TDCC) assay

RiboMab containing supernatants were generated via electroporation of K-562 cells with corresponding IVT-RNA chains. The mass chain ratios of HC1:HC2:LC of all RiboMabs constructs during electroporation was 1.9:2.2:1. The supernatants were concentrated ten times (10x) after times via centrifugation with 50 kDa MWCO concentrator (Amicon), analyzed via sandwich ELISA and Western Blot and stored at 4 °C until further analysis. PSMA-positive LNCaP_clone_FGC_luc target cells or PSMA-negative PA1-SC12_luc control cells were seeded in white 96-well plates (BRAND) (8 x 10³ cells in 40 µL per well) and cultured at 37 °C and 5 % CO₂ for 4 h. Both target cells express luciferase to monitor cell viability. Next, previously thawed PBMC effector cells were added to the target cells at an effector-to-target (E:T) ratio of 20:1 (1.6 x 10⁵ cells in 40 µL per well). RPMI1600-Medium (Gibco) + 10 % FBS (Gibco) was used as assay medium. Additionally, anti-PSMAxCD3 RiboMab containing cell culture supernatants were added in different concentrations (5-fold, 10-point dilution: 1500 ng/mL to 0.000768 ng/mL, except for RiboMab_PSMA-VHH#2xCD3#2 and RiboMab_PSMA-VHH#2xCD3#3 with 3-fold steps for the first two dilutions (550 ng/mL to 0.000552 ng/mL). The purified anti-PSMAxCD3 tribody protein "Positive Control" served as both a positive control and an interplate control with similar dilution steps as the test item (5-fold, 9-point dilution: 2000 ng/mL to 0.00512 ng/mL). A purified anti-CLDN6xCD3 tribody protein (bsAb control) was used as a negative control, with a concentration of 2000 ng/mL, on PSMA-positive cells and a killing control on the PSMA-negative, CLDN6 expressing PA1-SC12_luc cells. Furthermore, mock supernatant was used as a negative control. Maximum and minimum lysis (Lₘₐₓ/Lₘᵢₙ) wells were supplemented with mock supernatant only and assay medium only, respectively. Cells were treated in triplicates and incubated at 37°C and 5% CO₂ for 24 h. After the incubation period, cells in the Lₘₐₓ wells were lysed by adding 10 µL of 20% Triton X-100 (BioChemica) and incubated at 37°C for 40 min. Samples were equilibrated for 10 min at RT. Then, 75 µL of Luciferase reagent (Promega) was added to the wells and samples were incubated at RT for 20 min in the dark to induce luciferase activity and bioluminescence production. Bioluminescence was measured in a microplate reader (CLARIOstar) and the percentage of specific target-cell lysis was calculated as follows: $Specific lysis \left(%\right) = \left(1-\left[\frac{{L}_{exp} - {L}_{max}}{{L}_{min} - {L}_{max}}\right]\right) \times 100$

Specific lysis refers to PSMA-dependent tumor-cell killing augmented by the RiboMab candidates, with normalization to the Lₘᵢₙ and Lₘₐₓ readouts. The data is then summarized in a dose-response graph. Luciferin oxidation by luciferase is ATP-dependent; dying tumor cells emit lower bioluminescence due to reduced viability. Thus, lower bioluminescence values reflect a lower number of viable tumor cells.

The graphs show the increase in specific lysis as it relates to increasing RiboMab concentrations in cell culture supernatant, as determined by sandwich ELISA. The error bars are calculated from the standard deviation of three individual test well replicates. The assay was conducted with (A) PSMA-positive LNCaP cells or (B) PSMA-negative HEK293/T17 cells. (C) The positive controls on the different 96-well plates yield similar EC₅₀ values and the negative controls show no lysis.

### Results of the T-cell dependent cell-mediated cytotoxicity (TDCC) assay

Evaluation of all RiboMabs, shown in Figure 6, yields a sigmoidal dose-response curve with concentration dependent increase of specific lysis of PSMA expressing target cells, reaching lysis rates of 90 to 100 %. This dose-dependent effect can only be seen when PBMCs and RiboMabs are co-cultured with PSMA-positive LNCaP clone FGC_luc cells (Figures 6A and 6B). The positive controls showed a concentration dependent increase in tumor cell lysis with similar EC₅₀ values on all plates (Figure 6C). The anti-CLDN6xCD3 control antibody showed no lysis on PSMA-positive cells, but showed lysis of PSMA-negative, CLDN6 expressing cells. Mock controls show minimal influence of mock on cell growth and luciferase readout.

These results show that both the anti-PSMA VHH part and the anti-CD3 part of the RiboMab antibodies bind to their respective target and specifically initiate an immune synapse between PSMA-positive cells and T-cells which leads to increased tumor cell lysis (Table 3).

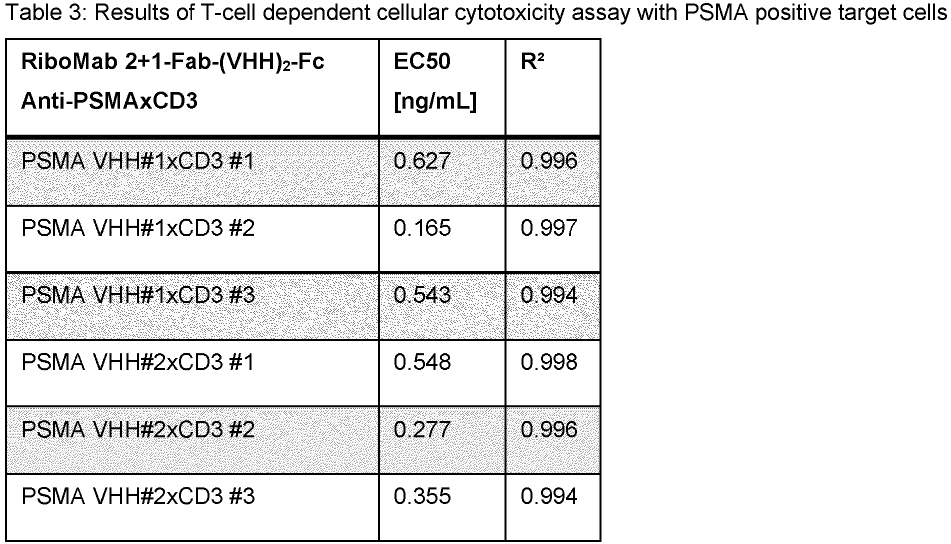

### Example 5: Anti-PSMAxCD3 RiboMabs induce T-cell activation via CD3 binding, specifically in the presence of PSMA expressing cells

### T-Cell Activation Assay

The assay was performed on a 48-well tissue culture plate. Cell suspensions were prepared in assay medium: 5.0 x 10⁵ cells/mL of the PSMA-positive LNCaP clone FGC target cells and 5.0 x 10⁶ cell/mL of PBMCs (effector cells). 100 µL of the target cells (5.0 x 10⁴) followed by 100 µL of the effector cells (5.0 x 10⁵) were added to the corresponding wells to achieve a 10:1 E:T ratio. The same was prepared with effector cells only, serving as a negative control. The first sample dilution was prepared in assay medium and 10x Mock to adjust samples matrices and 10-fold serial dilutions of the PSMAxCD3 targeting RiboMabs were prepared in assay medium (10-point; end concentration 2,000; 200; 20; 2; 0.2; 0.02; 0.002; 0.0002, 0.00002, 0.000002 ng/mL). 50 µL of the dilutions were added in triplicate to the test wells, respectively. 10 ng/mL OKT3 (BioXCell) was added to the positive control wells, also in triplicate, to serve as reference for the maximal stimulation of T cells. As negative control mock supernatant from the RiboMab production was used. The matrices of the control samples were adjusted to match those of the test items as described above. The assay plate was then incubated at 37°C with 5% CO₂ for 48 h. At the end of the 48-h incubation period, cell culture supernatants from the assay plates were collected by transfer to a fresh 96-well round-bottom microtiter plate to be stored for cytokine analysis. The cells were transferred to different 96-well round-bottom microtiter plates and centrifuged at 460 x g for 5 min. After dislodging the resulting cell pellet by gentle vortexing the microtiter plates, 50 µL of FACS buffer containing PE-Cy7 Mouse Anti-Human CD5 (Abcam), PE Mouse Anti-Human CD25 (BD Biosciences), APC Mouse Anti-Human CD69 (BD Biosciences) antibodies and eFluor506 (eBiosciences) was added to each suspension to stain at 2 to 8°C for 30 min in the dark. Following two washing steps with plain FACS buffer (DPBS, 5 % heat inactivated FBS and 5 mM EDTA), the stained cells were fixated by adding 30 µL of fixative solution (BD Biosciences) and incubation for 30 min at 4°C. The data were acquired on a FACS Celesta flow cytometer (BD Biosciences) and collected via FACS Diva software (both from BD Biosciences). Data analysis was performed with FlowJo v10 software (TreeStar Inc/BD Biosciences).

### Results of the T-Cell Activation Assay

T-cell activation after induction with anti-PSMAxCD3 RiboMabs was assessed via CD5 staining for T-cells and CD25 and CD69 staining for activation markers. Figure 7A shows that concentration dependent T-cell activation could be achieved with all RiboMabs in the presence of PSMA-expressing cells. This induction was specific, as in the absence of PSMA-expressing cells no increased CD25 or CD69 expression could be detected in the T-cell population. In a more detailed illustration of activation marker analysis (Figure 7B), more of the cells showed an earlier activation profile (CD69+/CD25-) than the later activation profile (CD69+/CD25+). The ratio of CD25 only activated cells stayed roughly the same over the concentration range. OKT3 control indicated a potential activation of 80 % of the all CD5 positive cells, while mock supernatant from the RiboMab production did not seem to have any influence on T-cell activation. Calculated EC₅₀ values of all RiboMabs were similar (Table 4).

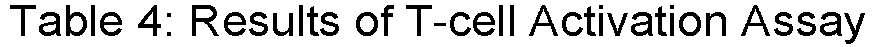

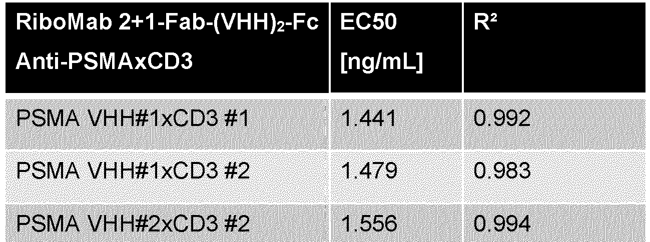

### Example 6: Anti-PSMAxCD3 RiboMabs convey target specific and concentration dependent induction of T-cell proliferation

### T-Cell Proliferation Assay with CD3-targeting bispecific antibodies

The assay was performed on a 48-well tissue culture plate. Cell suspensions were prepared in assay medium: 5.0 x 10⁵ cells/mL either of the PSMA-positive LNCaP clone FGC target cells and 5.0 x 10⁶ cell/mL of PBMCs (effector cells). 1.2 x 10⁷ effector cells per mL were stained with 1 µM of a CFSE dye (Thermo Fisher) for 5 min at room temperature to monitor cell proliferation. 100 µL of the target cells (5.0 x 10⁴) followed by 100 µL of the effector cells (5.0 x 10⁵) were added to the corresponding wells to achieve a 10:1 E:T ratio. The same was prepared with PSMA-negative PA1-SC12 cells, serving as a control for target-independent induction of T-cell proliferation. The first sample dilution of the PSMAxCD3 targeting RiboMabs was prepared in assay medium and 10x Mock to adjust samples matrices and 10-fold serial dilutions were prepared in assay medium (4-point; end concentration 1,000; 10; 0.1; 0.001 ng/mL). 50 µL of the dilutions were added in triplicate to the test wells, respectively. A purified anti-PSMAxCD3 antibody as well as the anti-CD3 antibody OKT3 (BioXCell) were used as positive controls, an anti-CLDN6xCD3 (Control bsAb) was used as a negative control for LNCaP cells and a positive control for PA1-SC12, and mock supernatant from the RiboMab production was used as an additional negative control. The matrices of the control samples were adjusted to match those of the test items as described above. The mixes of target cells, effector cells and test item were incubated at 37 °C and 5 % CO₂ for 72 h. At the end of the incubation period, the contents of each well on the assay plate (250 µL) were transferred to a 96-well round-bottom plate and centrifuged for 5 min at 460 x g to pellet the cells. After discarding the resulting supernatant, the cells were stained with 50 µL of a master mix containing 0.5 µL CD5-PE-Cy7 (BD Biosciences) and 0.125 µL eFluor 506 (eBiosciences) in 49.375 µL FACS buffer (DPBS, 5 % heat inactivated FBS and 5 mM EDTA). The plate was then incubated for 30 min at 4°C, followed by two wash steps with FACS buffer and addition of 50 µL of the stabilizing fixative solution (BD Biosciences). The cells were then acquired on a FACSCelesta flow cytometer and the data collected using FACS Diva software (both from BD Biosciences). Data analysis was performed with FlowJo v10 software (TreeStar Inc./BD Biosciences). CD5 positive cells were marked as T cells; surface CD3 expression had been confirmed prior to cryopreservation of the PBMC samples. The reduction in CFSE fluorescence intensity after every cell division indicated successful T-cell expansion.

### Results of T-Cell Proliferation Assay

After 72 h of incubation with target cells and effector cells, the T cells, identified by CD5 staining, showed enhanced proliferation in the presence of the different RiboMabs. This effect was concentration-dependent. Neither the anti-CLDN6xCD3 control (Control bsAb) nor the mock supernatant induced any proliferation in the presence of PSMA-positive cells. The OKT3 control illustrated the possible T-cell proliferation after 72 h independent of target expression. Incubated with PSMA-negative target cells, only the anti-CLDN6xCD3 antibody induced T-cell proliferation, as expected. None of the three RiboMabs induced T-cell proliferation without access to PSMA, even at concentrations of 1 µg per mL.

### Example 7: Anti-PSMAxCD3 RiboMab reference proteins are able to inhibit the glutamate carboxypeptidase function of PSMA which could enhance anti-tumoral effect

### RiboMab Reference Protein Production

Reference proteins of corresponding RiboMabs were produced via cloning into the pcDNA3.4TOPO vector and transient transfection of HEK Expi293F cells according to the Expifectamine Transfection Kit (Thermo Fisher) method at a 250 mL scale in a 1 L Erlenmeyer flask. Monomeric antibody was purified via FPLC with Protein A affinity chromatography using a HiTrap Protein A HP (GE Healthcare) and size exclusion chromatography using a Superdex 200 pg HiLoad 26/600 column (GE Haelthcare) and analyzed via SDS-PAGE and HPLC for at least 95% purity.

### PSMA Glutamate Carboxypeptidase Inhibition Assay

The enzymatic activity of PSMA as a glutamate carboxypeptidase one of the substrates NAAG via the release of glutamate during the reaction. This glutamate can be detected using ortho-phtalaldehyde (OPA) and β-Mercaptoethanol at alkaline pH to yield a fluorescent reaction product. All reagents of the assay are diluted in assay buffer (50 mM HEPES, 100 mM NaCl, pH 7.5). Serial dilutions of purified antibodies, reference proteins to the tested RiboMabs, were generated in assay buffer (8 points, 5x dilution: 200, 40, 8, 1.6, 0.32, 0.064, 0.0128, 0.00256). Firstly, 33,3 µL of a 0.6 µM hPSMA solution and 33,3 µL of the antibody solution are mixed. Then 33,3 µL of a 0.036 µM NAAG solution are added as substrate quickly to all wells to reduce variation in incubation time. As negative control hPSMA is heat inactivated for 5 min at 95 °C. Furthermore, mixes without substrate, enzyme or antibody were generated. The enzyme, substrate and antibody mixes are incubated for 1 h at 37 °C. To stop the enzymatic reaction and start detection, 100 µL of a 15 mM ortho-phtalaldehyde solution were added to each well and incubated for 10 min at room temperature (or 25 °C) in the dark. The resulting fluorescence values were detected using a Clariostar plate reader (BMG Labtech) at 355 nm excitation and 460 nm emission.

### Results of PSMA Glutamate Carboxypeptidase Inhibition Assay

PSMA has two enzymatic functions, namely as a folate hydrolase and a glutamate carboxypeptidase, which can both have an effect on tumor growth. The PSMA enzyme inhibition assay results show that all tested anti-PSMAxCD3 antibodies inhibit NAAG substrate conversion concentration-dependent to a certain degree. Fluorescence values in combination with control samples without antibody suggest complete inhibition at higher concentrations for all three tested antibodies (Table 5).

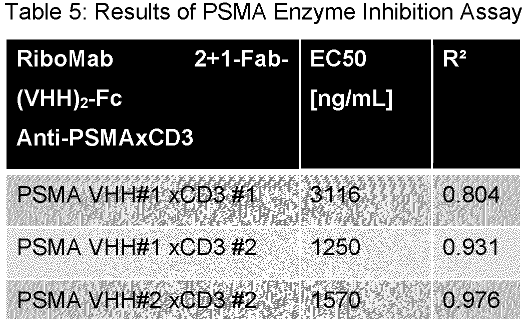

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. A bispecific binding molecule comprising a CD3-binding moiety and a first prostate-specific membrane antigen (PSMA)-binding moiety.

2. The bispecific binding molecule according to claim 1, wherein the CD3-binding moiety comprises:
(i) a CD3 heavy chain variable (CD3-VH) domain and/or a CD3 light chain variable (CD3-VL) domain; or
(ii) a Fab, wherein the Fab comprises:
(a) a heavy chain comprising a CD3 heavy chain variable (CD3-VH) domain and a CD3 heavy chain constant (CD3-CH1) domain; and
(b) a light chain comprising a CD3 light chain variable (CD3-VL) domain and a CD3 light chain constant (CD3-CL) domain.

3. The bispecific binding molecule according to claim 1 or claim 2, wherein the first PSMA-binding moiety comprises a PSMA-binding VHH domain;
optionally wherein the bispecific binding molecule further comprises a second PSMA-binding moiety;
further optionally wherein each PSMA-binding moiety comprises a PSMA-binding VHH domain.

4. The bispecific binding molecule according to any of claims 1-3, wherein the bispecific binding molecule further comprises an Fc region comprising a first subunit and a second subunit;
optionally wherein the first subunit of the Fc region and the second subunit of the Fc region each comprise a CH2 domain and/or a CH3 domain.

5. The bispecific binding molecule according to claim 4, wherein
(i) the first PSMA-binding moiety is linked to the CD3-binding moiety; optionally wherein
(a) the C-terminus of the first PSMA-binding moiety is linked to the N-terminus of the CD3-VH domain; or
(b) the C-terminus of the first PSMA-binding moiety is linked to the N-terminus of the CD3-VL domain; and/or
(ii) the CD3-binding moiety is linked to the Fc region, optionally wherein
(a) the C-terminus of the CD3-CH1 domain is linked to the first subunit of the Fc region; or
(b) the C-terminus of the CD3-CL domain is linked to the first subunit of the Fc region; and/or
(iii) the second PSMA-binding moiety is linked to the Fc region, optionally wherein the C-terminus of the second PSMA-binding moiety is linked to the second subunit of the Fc region.

6. The bispecific binding molecule according to any of claims 1-5, wherein:
(i) the CD3-binding moiety comprises a Fab, wherein the Fab comprises:
(a) a heavy chain comprising the CD3-VH domain and a heavy chain constant (CD3-CH1) domain; and
(b) a light chain comprising the CD3-VL domain and a light chain constant (CD3-CL) domain;
(ii) the bispecific binding molecule further comprises a second PSMA-binding moiety, wherein the first PSMA-binding moiety and the second PSMA-binding moiety are the same, wherein the first PSMA-binding moiety and the second PSMA-binding moiety each comprise a PSMA-binding VHH domain;
(iii) the bispecific binding molecule further comprises an Fc region comprising a first subunit and a second subunit; wherein the first subunit of the Fc region and the second subunit of the Fc region each comprise a CH2 domain and a CH3 domain; optionally wherein the CH3 domain of one subunit of the Fc region comprises one or more mutations selected from the list consisting of: Y349C, T366S, L368A, and Y407V; and the CH3 domain of the other subunit of the Fc region comprises one or more mutations selected from the list consisting of: S354C and T366W;
(iv) the C-terminus of the PSMA-binding VHH domain of the first PSMA-binding moiety is linked to the N-terminus of the CD3-VH domain;
(v) the C-terminus of the CD3-CH1 domain is linked to the N-terminus of the CH2 domain of the first subunit of the Fc region, preferably to the N-terminus of a hinge region of the CH2 domain of the first subunit of the Fc region; and
(vi) the C-terminus of the PSMA-binding VHH domain of the second PSMA-binding moiety is linked to the N-terminus of the CH2 domain of the second subunit of the Fc region, preferably to the N-terminus of a hinge region of the CH2 domain of the second subunit of the Fc region.

7. The bispecific binding molecule according to any of claims 1-6, wherein the CD3-binding moiety comprises heavy chain complementarity determining regions (HCDRs) 1-3, and/or light chain complementarity determining regions (LCDRs) 1-3, wherein
(i) (according to Kabat numbering)
HCDR1 comprises the amino acid sequence according to SEQ ID NO: 1,
HCDR2 comprises the amino acid sequence according to SEQ ID NO: 2, and
HCDR3 comprises the amino acid sequence according to SEQ ID NO: 3,
and/or
LCDR1 comprises the amino acid sequence according to SEQ ID NO: 4,
LCDR2 comprises the amino acid sequence according to SEQ ID NO: 5, and
LCDR3 comprises the amino acid sequence according to SEQ ID NO: 6;
or
(ii) (according to IMGT numbering)
HCDR1 comprises the amino acid sequence according to SEQ ID NO: 7,
HCDR2 comprises the amino acid sequence according to SEQ ID NO: 8,
HCDR3 comprises the amino acid sequence according to SEQ ID NO: 9, and
and/or
LCDR1 comprises the amino acid sequence according to SEQ ID NO: 10,
LCDR2 comprises the amino acid sequence according to SEQ ID NO: 11, and
LCDR3 comprises the amino acid sequence according to SEQ ID NO: 12;
optionally wherein one or more of the CDRs comprises one, two or three amino acid mutations relative to the recited sequences.

8. The bispecific binding molecule according to any of claims 1-7, wherein
(i) the CD3-binding moiety comprises a CD3-VH domain sequence having at least 70%, at least 75%, at least 80%, at least 85%, at least 90%, at least 95%, at least 97%, at least 99%, or 100% identity to any one of SEQ ID NO: 13, SEQ ID NO: 14, or SEQ ID NO: 15;
(ii) the CD3-binding moiety comprises a CD3-VL domain sequence having at least 70%, at least 75%, at least 80%, at least 85%, at least 90%, at least 95%, at least 97%, at least 99%, or 100% identity to any one of SEQ ID NO: 17, SEQ ID NO: 18, or SEQ ID NO: 19;
(iii) the CD3-binding moiety comprises a heavy chain comprising a sequence having at least 70%, at least 75%, at least 80%, at least 85%, at least 90%, at least 95%, at least 97%, at least 99%, or 100% identity to any one of SEQ ID NO: 21, SEQ ID NO: 22, or SEQ ID NO: 23; and/or
(iv) the CD3-binding moiety comprises a light chain comprising a sequence having at least 70%, at least 75%, at least 80%, at least 85%, at least 90%, at least 95%, at least 97%, at least 99%, or 100% identity to any one of SEQ ID NO: 25, SEQ ID NO: 26, or SEQ ID NO: 27.

9. The bispecific binding molecule according to any of claims 1-8, wherein the, or each, PSMA-binding moiety independently comprises heavy chain complementarity determining regions (HCDRs) 1-3, wherein
(a) (according to Kabat numbering)
(i)
HCDR1 comprises the amino acid sequence according to SEQ ID NO: 29,
HCDR2 comprises the amino acid sequence according to SEQ ID NO: 30, and
HCDR3 comprises the amino acid sequence according to SEQ ID NO: 31;
optionally wherein one or more of the CDRs comprises one, two or three amino acid mutations relative to the recited sequences; or
(ii)
HCDR1 comprises the amino acid sequence according to SEQ ID NO: 36,
HCDR2 comprises the amino acid sequence according to SEQ ID NO: 37, and
HCDR3 comprises the amino acid sequence according to SEQ ID NO: 38;
optionally wherein one or more of the CDRs comprises one, two or three amino acid mutations relative to the recited sequences;
or
(b) (according to IMGT numbering)
(iii)
HCDR1 comprises the amino acid sequence according to SEQ ID NO: 32,
HCDR2 comprises the amino acid sequence according to SEQ ID NO: 33, and
HCDR3 comprises the amino acid sequence according to SEQ ID NO: 34;
optionally wherein one or more of the CDRs comprises one, two or three amino acid mutations relative to the recited sequences; or
(iv)
HCDR1 comprises the amino acid sequence according to SEQ ID NO: 39,
HCDR2 comprises the amino acid sequence according to SEQ ID NO: 40, and
HCDR3 comprises the amino acid sequence according to SEQ ID NO: 41;
optionally wherein one or more of the CDRs comprises one, two or three amino acid mutations relative to the recited sequences;
optionally wherein
(v) the, or each, PSMA-binding moiety independently comprises a sequence having at least 70%, at least 75%, at least 80%, at least 85%, at least 90%, at least 95%, at least 97%, at least 99%, or 100% identity to SEQ ID NO: 35; or
(vi) the, or each, PSMA-binding moiety independently comprises a sequence having at least 70%, at least 75%, at least 80%, at least 85%, at least 90%, at least 95%, at least 97%, at least 99%, or 100% identity to SEQ ID NO: 42.

10. One or more polynucleotide(s) comprising a nucleic acid sequence encoding the bispecific binding molecule according to any of claims 1-9.

11. The one or more polynucleotide(s) according to claim 10, wherein the nucleic acid sequence is selected from the list consisting of: a DNA sequence, a cDNA sequence, an RNA sequence, and an mRNA sequence; optionally wherein the nucleic acid sequence is an RNA sequence; optionally wherein the RNA sequence is an mRNA sequence; optionally wherein the RNA is modRNA (N1-methyl-pseudouridine); optionally wherein
(i) the RNA sequence comprises a 5'-cap, further optionally wherein
(a) the 5'-cap is CC413; or
(b) wherein the 5'-cap is m₂^{7,3'-O}Gppp(m₁^{2'-O})ApG, which comprises the following structure: 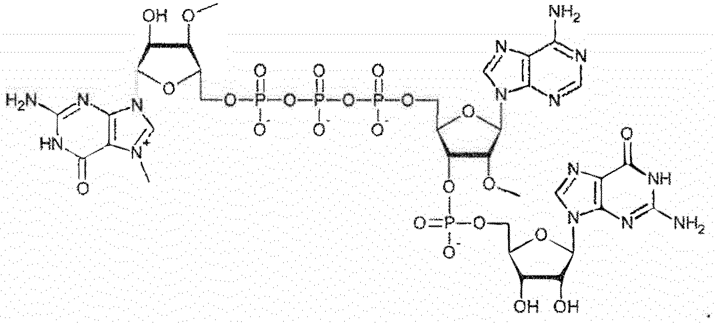
(ii) the RNA sequence comprises a poly adenine sequence, optionally wherein the poly adenine sequence comprises the nucleic acid sequence according to SEQ ID NO: 43 or a variant having at least 80% sequence identity thereto;
(iii) the RNA sequence comprises a 5'UTR; optionally wherein the 5'UTR comprises the nucleic acid sequence according to SEQ ID NO: 44 or a variant having at least 80% sequence identity thereto.
(iv) the RNA sequence comprises a 3'UTR; optionally wherein the 3'UTR comprises the nucleic acid sequence according to SEQ ID NO: 45 or a variant having at least 80% sequence identity thereto.

12. The one or more polynucleotide(s) according to claim 10 or claim 11 wherein a plurality of polynucleotides comprises:
(i) a first polynucleotide comprising a nucleic acid sequence encoding the following polypeptide chain from N to C terminus:
PSMA-binding moiety, preferably a PSMA-binding VHH domain, - (optional linker) - CD3-VH domain - CD3-CH1 domain - CH2 domain - CH3 domain;
(ii) a second polynucleotide comprising a nucleic acid sequence encoding the following polypeptide chain from N to C terminus:
PSMA-binding moiety, preferably a PSMA-binding VHH domain, - (optional linker) - CH2 domain - CH3 domain; and/or
(iii) a third polynucleotide comprising a nucleic acid sequence encoding the following polypeptide chain from N to C terminus:
CD3-VL domain - CD3-CL domain;
optionally wherein
(iv) the first polynucleotide comprises a nucleic acid sequence comprising in 5'-3' orientation:
5'-cap - 5'UTR - PSMA-binding moiety, preferably a PSMA-binding VHH domain, - (optional linker) - CD3-VH domain -CD3-CH1 domain - CH2 domain - CH3 domain - 3'UTR - Poly(A)
(v) the second polynucleotide comprises a nucleic acid sequence comprising in 5'-3' orientation:
5'-cap - 5'UTR - PSMA-binding moiety, preferably a PSMA-binding VHH domain, - (optional linker) - CH2 domain - CH3 domain - 3'UTR - Poly(A); and/or
(vi) the third polynucleotide comprises a nucleic acid sequence comprising in 5'-3' orientation:
5'-cap - 5'UTR - CD3-VL domain - CD3-CL domain - 3'UTR - Poly(A);
further optionally wherein:
(i) the first polynucleotide comprises:
(a) a nucleic acid sequence according to SEQ ID NO: 46 or a variant having at least 80% sequence identity to SEQ ID NO: 46;
(b) a nucleic acid sequence according to SEQ ID NO: 47 or a variant having at least 80% sequence identity to SEQ ID NO: 47;
(c) a nucleic acid sequence according to SEQ ID NO: 48 or a variant having at least 80% sequence identity to SEQ ID NO: 48;
(e) a nucleic acid sequence according to SEQ ID NO: 49 or a variant having at least 80% sequence identity to SEQ ID NO: 49;
(f) a nucleic acid sequence according to SEQ ID NO: 50 or a variant having at least 80% sequence identity to SEQ ID NO: 50; or
(g) a nucleic acid sequence according to SEQ ID NO: 51 or a variant having at least 80% sequence identity to SEQ ID NO: 51;
(ii) the second polynucleotide comprises
(a) a nucleic acid sequence according to SEQ ID NO: 52 or a variant having at least 80% sequence identity to SEQ ID NO: 52; or
(b) a nucleic acid sequence according to SEQ ID NO: 53 or a variant having at least 80% sequence identity to SEQ ID NO: 53;
and/or
(iii) the third polynucleotide comprises
(a) a nucleic acid sequence according to SEQ ID NO: 54 or a variant having at least 80% sequence identity to SEQ ID NO: 54;
(b) a nucleic acid sequence according to SEQ ID NO: 55 or a variant having at least 80% sequence identity to SEQ ID NO: 55; or
(c) a nucleic acid sequence according to SEQ ID NO: 56 or a variant having at least 80% sequence identity to SEQ ID NO: 56.

13. A vector comprising one or more polynucleotide(s) according to any of claims 10-12.

14. A composition comprising:
(i)
(a) the bispecific binding molecule according to any of claims 1-9; or
(b) the one or more polynucleotide(s) according to any of claims 10-12; or
(c) the vector according to claim 13;
optionally wherein the composition is a pharmaceutical composition comprising:
(ii) a pharmaceutically acceptable carrier, diluent or excipient,
optionally wherein the pharmaceutical composition further comprises
(iii) a vesicle, a nanoparticle, a lipid nanoparticle (LNP), a liposome or a polymeric mixture wherein the bispecific binding molecule, the one or more polynucleotide(s), or the composition is encapsulated within the vesicle, nanoparticle, LNP, liposome or polymeric mixture.

15. The bispecific binding molecule according to any of claims 1-9; or the one or more polynucleotide(s) according to any of claims 10-12; or the composition according to claim 13, or the composition according to claim 14, for use in the treatment or prevention of a disease; optionally wherein the disease is cancer; further optionally wherein the cancer is prostate cancer.
